# EUROPEAN PATENT APPLICATION

(11) **EP 2 301 928 A1**
(43) Date of publication of application: **30.03.2011**
(21) Application number: 10184712.7
(22) Date of filing: 27.07.2006
(51) Int. Cl.: C07D 403/04, A61K 31/506

(54) **Imidazolyl-pyrimidine compounds for use in the treatment of proliferative disorders**

(30) Priority: 30.07.2005 GB 0515743; 06.10.2005 GB 0520281; 22.12.2005 GB 0526015; 28.04.2006 GB 0608371
(62) Divisional of application: 06765122.4
(71) Applicant: AstraZeneca AB, 151 85 Södertälje (SE)
(72) Inventor: Andrews, David Michael, Macclesfield, Cheshire SK10 4TG (GB); Finlay, Maurice Raymond Verschoyle, Macclesfield, Cheshire SK10 4TG (GB); Green, Clive, Macclesfield, Cheshire SK10 4TG (GB); Jones, Clifford David, Macclesfield, Cheshire SK10 4TG (GB)
(74) Representative: Padget, Lucy Clare

(57) **Abstract**

Compounds of formula (**I**): 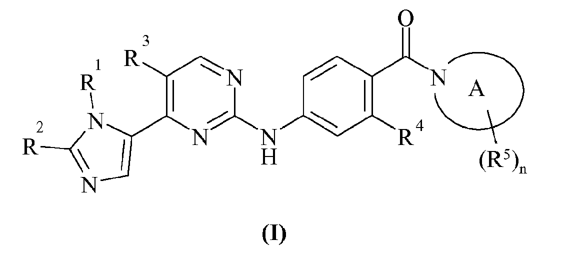 which possess cell-cycle inhibitory activity are described.

## Description

The invention relates to pyrimidine derivatives, or pharmaceutically acceptable salts or *in vivo* hydrolysable esters thereof, which possess cell-cycle inhibitory activity and are accordingly useful for their anti-cell-proliferation (such as anti-cancer) activity and are therefore useful in methods of treatment of the human or animal body. The invention also relates to processes for the manufacture of said pyrimidine derivatives, to pharmaceutical compositions containing them and to their use in the manufacture of medicaments of use in the production of an anti-cell-proliferation effect in a warm-blooded animal such as man.

The cell cycle is fundamental to the survival, regulation and proliferation of cells and is highly regulated to ensure that each step progresses in a timely and orderly manner. The progression of cells through the cell cycle arises from the sequential activation and de-activation of several members of the cyclin-dependent kinase (CDK) family. The activation of CDKs is dependent on their interaction with a family of intracellular proteins called cyclins. Cyclins bind to CDKs and this association is essential for CDK activity within the cell. Different cyclins are expressed and degraded at different points in the cell cycle to ensure that activation and inactivation of CDKs occurs in the correct order for progression through the cell cycle.

Moreover, CDKs appear to be downstream of a number of oncogene signalling pathways. Deregulation of CDK activity by upregulation of cyclins and/or deletion of endogenous inhibitors appears to be an important axis between mitogenic signalling pathways and proliferation of tumour cells.

Accordingly it has been recognised that an inhibitor of cell cycle kinases, particularly inhibitors of CDK1, CDK2, CDK4 and CDK6 (which operate at the G2/M, G1/S-S-G2/M and G1-S phases respectively) should be of value as an active inhibitor of cell proliferation, such as growth of mammalian cancer cells.

Tumour cells are also thought to be highly dependent on the continual transcriptional activity of RNA polymerase II to maintain appropriate levels of anti-apoptotic proteins and ensure tumour cell survival. CDK1, CDK7, CDK8 and CDK9 in particular are known to regulate the activity of RNA polymerase II through phosphorylation of the C-terminal domain of the protein. Thus, the inhibition of RNA polymerase II activity through inhibitors of these CDKs may contribute to a pro-apoptotic effect in tumour cells.

The inhibition of cell cycle kinases is expected to be of value in the treatment of disease states associated with aberrant cell cycles and cell proliferation such as cancers (solid tumours and leukemias), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

WO 02/20512, WO 03/076435, WO 03/076436, WO 03/076434, WO 03/076433 and WO 04/101549 describe certain 2-anilino-4-imidazolylpyrimidine derivatives that inhibit the effect of cell cycle kinases. The present invention is based on the discovery that a novel group of 2-(4-heterocyclocarbonylanilino)-4-(imidazolyl)pyrimidines inhibit the effects of cell cycle kinases, particularly CDK2, and thus possess anti-cell-proliferation properties. The compounds of the present invention are not specifically disclosed in any of the above applications and we expect that these compounds possess beneficial properties in terms of one or more of their pharmacological activity (particularly as compounds which inhibit CDK2) and / or pharmacokinetic, efficacious, metabolic and toxicological profiles that make them particularly suitable *for in vivo* administration to a warm blooded animal, such as man. In particular these compounds generally have very high levels of cell and enzyme potency, high aqueous solubility and favorable protein binding characteristics.

Accordingly, the present invention provides a compound of formula (I): 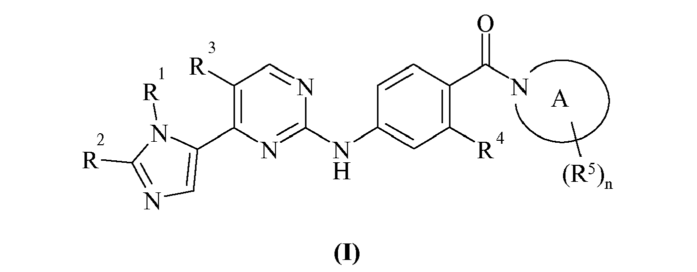 wherein:
**R**¹ is ethyl, propyl, isopropyl, butyl, iso-butyl, sec-butyl, t-butyl, cyclopropyl, cyclopropylmethyl, 1-cyclopropylethyl, cyclobutylmethyl, cyclopentyl or cyclobutyl; wherein R¹ may be optionally substituted on carbon by one or more R⁶;
**R**² is methyl, ethyl, isopropyl, fluoromethyl, difluoromethyl, trifluoromethyl, methoxymethyl, cyclopropylmethyl or cyclopropyl;
**R**³ is hydrogen or halo;
**R**⁴ is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
**Ring A** is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
**R**⁵ is a substituent on carbon and is selected from halo, cyano, hydroxy, trifluoromethoxy, amino, carboxy, carbamoyl, sulphamoyl, C₁_₆alkyl, C₂_₆alkenyl, C₂-₆akynyl, C₁_₆alkanoyl, *N-*(C₁-₆alkyl)carbamoyl, *N,N-*(C₁-₆alkyl)₂carbamoyl, C₁-₆alkylS(O)ₐ wherein a is 0 to 2, C₁-₆alkylsulphonyloxy, C₁-₆alkoxycarbonyl, carbocyclyl, heterocyclyl, *N-*(C₁-₆alkyl)sulphamoyl or *N,N-*(C₁-₆alkyl)₂sulphamoyl; wherein R⁵ independently may be optionally substituted on carbon by one or more R⁸; wherein if said heterocyclyl contains an -NH- moiety that nitrogen may be optionally substituted by R¹⁵; or R⁵ is -NHR⁹, -NR¹⁰R¹¹ or -O-R¹².
**n** is 0-2; wherein the values of ^{R}5 maybe the same or different;
**R**⁶ is selected from halo, methoxy and hydroxy;
**R** ⁷, **R**⁹, **R**¹⁰, **R**¹¹, **R** ¹² and **R** ¹⁵ are independently selected from C₁-₄alkyl, C₁-₄akanoyl, C₁-₄alkylsulphonyl, C₂-₄alkenylsulphonyl, C₂-₄akynylsulphonyl, C₁₋₄alkoxycarbonyl, carbamoyl, *N-*(C₁₋₄alkyl)carbamoyl, *N,N* (C₁₋₄alkyl)carbamoyl, carbocyclyl or heterocyclyl; wherein R⁷, **R**⁹, R¹⁰, R¹¹, R¹² and R¹⁵ may be independently optionally substituted on carbon by one or more groups selected from R¹³; and wherein if said heterocyclyl contains an -NH- moiety that nitrogen may be optionally substituted by R¹⁴;
**R**⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N*-methyl-*N*-ethylamino, acetylamino, phenylamino, *N*-methylcarbamoyl, *N*-ethylcarbamoyl, *N,N* dimethylcarbamoyl, *N,N* diethylcarbamoyl, *N-*methyl-*N-*ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N-*dimethylsulphamoyl, *N,N-*diethylsulphamoyl or *N-*methyl-*N*-ethylsulphamoyl;
**R**¹³ is selected from halo, cyano, hydroxy, amino, trifluoromethyl, trifluoromethoxy, dimethylamino, carbocyclyl, heterocyclyl, C₁-₃alkyl and C₁-₃alkoxy; and
**R**¹⁴ is selected from C₁-₃alkyl, C₁-₃alkanoyl, C₁₋₃alkylsulphonyl, C₁₋₃alkoxycarbonyl, carbamoyl, *N-*(C₁₋₃alkyl)carbamoyl and *N,N-*(C₁₋₃alkyl)carbamoyl;
or a pharmaceutically acceptable salt or an *in vivo* hydrolysable ester thereof.

According to a further feature of the present invention there is provided a compound of formula (I) wherein:
**R**¹ is ethyl, propyl, isopropyl, butyl, iso-butyl, sec-butyl, t-butyl, cyclopropyl, cyclopropylmethyl, 1-cyclopropylethyl or cyclobutyl; wherein R¹ may be optionally substituted on carbon by one or more R⁶;
**R**² is methyl, ethyl, isopropyl, fluoromethyl, difluoromethyl, trifluoromethyl, methoxymethyl, cyclopropylmethyl or cyclopropyl;
**R**³ is hydrogen or halo;
**R**⁴ is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
**Ring A** is a nitrogen linked 4-7 membered saturated ring which optionally contains an additional nitrogen, oxygen or sulphur atom; wherein if Ring A contains an additional nitrogen atom that nitrogen may be optionally substituted by R⁷;
**R**⁵ is selected from halo, cyano, hydroxy, trifluoromethoxy, amino, carboxy, carbamoyl, sulphamoyl, C₁₋₆alkyl, C₂₋₆alkenyl, C₂-₆alkynyl, C₁₋₆alkanoyl, *N-*(C₁₋₆alkyl)carbamoyl, *N,N* (C₁₋₆alkyl)₂Carbamoyl, C₁-₆alkylS(O)ₐ wherein a is 0 to 2, C₁₋₆alkoxycarbonyl, *N-*(C₁₋₆alkyl)sulphamoyl or *N,N-*(C₁₋₆alkyl)₂sulphamoyl; wherein R⁵ independently may be optionally substituted on carbon by one or more R⁸; or R⁵ is -NHR⁹, -NR¹⁰R¹¹ or -O-R¹²_{.}
**n** is 0-2; wherein the values of R⁵ maybe the same or different;
**R**⁶ is selected from halo, methoxy and hydroxy;
**R**⁷, **R**⁹, **R**¹⁰, **R**¹¹ and R¹² are independently selected from C₁-₄alkyl, C₁-₄alkanoyl, C₁₋₄alkylsulphonyl, C₂₋₄alkenylsulphonyl, C₂-₄akynylsulphonyl, C₁₋₄alkoxycarbonyl, carbamoyl, *N-*(C₁₋₄alkyl)carbamoyl, *N,N-* (C₁₋₄alkyl)carbamoyl, carbocyclyl or heterocyclyl; wherein R⁷, R⁹, R¹⁰, R¹¹ and R¹² may be independently optionally substituted on carbon by a group selected from R¹³; and wherein if said heterocyclyl contains an -NH- moiety that nitrogen may be optionally substituted by R¹⁴_{;}
**R**⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N-*methyl-*N-*ethylamino, acetylamino, *N-*methylcarbamoyl, *N-*ethylcarbamoyl, *N,N-*dimethylcarbamoyl, *N,N-*diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N-*methylsulphamoyl, *N-*ethylsulphamoyl, *N,N-*dimethylsulphamoyl, *N,N-*diethylsulphamoyl or *N-*methyl-*N-*ethylsulphamoyl;
**R**¹³ is selected from halo, cyano, hydroxy, amino, trifluoromethyl, trifluoromethoxy, C₁-₃alkyl and C₁-₃alkoxy; and
**R**¹⁴ is selected from C₁-₃alkyl, C₁-₃alkanoyl, C₁₋₃alkylsulphonyl, C₁₋₃alkoxycarbonyl, carbamoyl, *N*-(C₁-3alkyl)carbamoyl and *N,N-*(C₁₋₃alkyl)carbamoyl;
or a pharmaceutically acceptable salt or an *in vivo* hydrolysable ester thereof.

According to a further feature of the present invention there is provided a compound of formula (**I**) wherein:
**R**¹ is ethyl, propyl, isopropyl, butyl, iso-butyl, sec-butyl, t-butyl, cyclopropyl, cyclopropylmethyl, 1-cyclopropylethyl or cyclobutyl; wherein R¹ may be optionally substituted on carbon by one or more R⁶;
**R**² is methyl, ethyl, isopropyl, fluoromethyl, difluoromethyl, trifluoromethyl, methoxymethyl, cyclopropylmethyl or cyclopropyl;
**R**³ is hydrogen or halo;
**R**⁴ is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
**Ring A** is a nitrogen linked 4-7 membered saturated ring which optionally contains an additional nitrogen, oxygen or sulphur atom; wherein if Ring A contains an additional nitrogen atom that nitrogen may be optionally substituted by R⁷;
**R**⁵ is a substituent on carbon and is selected from halo, cyano, hydroxy, trifluoromethoxy, amino, carboxy, carbamoyl, sulphamoyl, C₁₋₆alkyl, C₂-₆alkenyl, C₂-₆akynyl, C₁₋₆alkanoyl, *N*-(C₁₋₆alkyl)carbamoyl, *N,N* (C₁₋₆alkyl)₂Carbamoyl, C₁-₆akylS(O)ₐ wherein a is 0 to 2, C₁₋₆alkoxycarbonyl, carbocyclyl, heterocyclyl, *N-*(C₁₋₆alkyl)sulphamoyl or *N,N-*(C₁₋₆alkyl)₂Sulphamoyl; wherein R⁵ independently may be optionally substituted on carbon by one or more R⁸; wherein if said heterocyclyl contains an -NH- moiety that nitrogen may be optionally substituted by R¹⁵; or R⁵ is -NHR⁹, -NR¹⁰R¹¹ or -O-R¹²_{;}
n is 0-2; wherein the values of ^{R}5 maybe the same or different;
**R**⁶ is selected from halo, methoxy and hydroxy;
**R**⁷, **R**⁹, **R**¹⁰, **R**¹¹, **R** ¹² and R ¹⁵ are independently selected from C₁-₄alkyl, C₁-₄alkanoyl, C₁₋₄alkylsulphonyl, C₂₋₄alkenylsulphonyl, C₂-₄akynylsulphonyl, C₁₋₄alkoxycarbonyl, carbamoyl, *N*-(C₁₋₄alkyl)carbamoyl, *N,N-*(C₁₋₄alkyl)carbamoyl, carbocyclyl or heterocyclyl; wherein R⁷, R⁹, R^{10,} R¹¹, R¹² and R¹⁵ may be independently optionally substituted on carbon by a group selected from R¹³; and wherein if said heterocyclyl contains an -NH- moiety that nitrogen may be optionally substituted by R¹⁴;
**R**⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N* dimethylcarbamoyl, *N,N* diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N-*dimethylsulphamoyl, *N,N-*diethylsulphamoyl or *N*-methyl-*N*-ethylsulphamoyl;
**R**¹³ is selected from halo, cyano, hydroxy, amino, trifluoromethyl, trifluoromethoxy, dimethylamino, carbocyclyl, heterocyclyl, C₁-₃alkyl and C₁₋₃alkoxy; and
**R**¹⁴ is selected from C₁-₃alkyl, C₁-₃alkanoyl, C₁₋₃alkylsulphonyl, C₁₋₃alkoxycarbonyl, carbamoyl, *N-*(C₁₋₃alkyl)carbamoyl and *N,N-*(C₁₋₃alkyl)carbamoyl;
or a pharmaceutically acceptable salt or an *in vivo* hydrolysable ester thereof.

According to a further feature of the present invention there is provided a compound of formula **(I)** wherein:
**R¹** is ethyl, propyl, isopropyl, butyl, iso-butyl, sec-butyl, t-butyl, cyclopropyl, cyclopropylmethyl, 1-cyclopropylethyl or cyclobutyl; wherein R¹ may be optionally substituted on carbon by one or more R⁶;
**R²** is methyl, ethyl, isopropyl, fluoromethyl, difluoromethyl, trifluoromethyl, methoxymethyl, cyclopropylmethyl or cyclopropyl;
**R³** is hydrogen or halo;
**R⁴** is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
**R**⁵ is a substituent on carbon and is selected from halo, cyano, hydroxy, trifluoromethoxy, amino, carboxy, carbamoyl, sulphamoyl, C₁₋₆alkyl, C₂₋₆alkenyl, C₂-₆alkynyl, C₁₋₆alkanoyl, *N-*(C₁₋₆alkyl)carbamoyl, *NN*-(C₁-₆akl)₂carbamoyl, C₁-₆alkylS(O)a wherein a is 0 to 2, C₁₋₆alkoxycarbonyl, carbocyclyl, heterocyclyl, *N-*(C₁₋₆alkyl)sulphamoyl or *N,N-*(C₁₋₆alkyl)₂Sulphamoyl; wherein R⁵ independently may be optionally substituted on carbon by one or more R⁸; wherein if said heterocyclyl contains an -NH- moiety that nitrogen may be optionally substituted by R¹⁵; or R⁵ is -NHR⁹, -NR¹⁰R¹¹ or -O-R¹²_{;}
**n** is 0-2; wherein the values of ^{R}5 maybe the same or different;
**R⁶** is selected from halo, methoxy and hydroxy;
**R ⁷_{,} R⁹, R¹⁰, R¹¹, R ¹²** and **R¹⁵** are independently selected from C₁-₄alkyl, C₁-₄alkanoyl, C₁₋₄alkylsulphonyl, C₂₋₄alkenylsulphonyl, C₂-₄alkynylsulphonyl, C₁₋₄alkoxycarbonyl, carbamoyl, *N-*(C₁₋₄alkyl)carbamoyl, *N,N-*(C₁₋₄alkyl)carbamoyl, carbocyclyl or heterocyclyl; wherein **R⁷, R⁹, R¹⁰, R¹¹, R¹²,** and **R¹⁵** may be independently optionally substituted on carbon by a group selected from R¹³; and wherein if said heterocyclyl contains an -NH- moiety that nitrogen may be optionally substituted by **R¹⁴**;
**R**⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N*-methyl-*N*-ethylamino, acetylamino, *N*-methylcarbamoyl, *N-*ethylcarbamoyl, *N,N* dimethylcarbamoyl, *N,N-*diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N-*dimethylsulphamoyl, *N,N-*diethylsulphamoyl or *N*-methyl-*N*-ethylsulphamoyl;
**R¹³** is selected from halo, cyano, hydroxy, amino, trifluoromethyl, trifluoromethoxy, dimethylamino, carbocyclyl, heterocyclyl, C₁-₃alkyl and C₁-₃akoxy; and
**R¹⁴** is selected from C₁-₃alkyl, C₁-₃alkanoyl, C₁₋₃alkylsulphonyl, C₁₋₃alkoxycarbonyl, carbamoyl, *N-*(C₁₋₃alkyl)carbamoyl and *N,N-*(C₁₋₃alkyl)carbamoyl;
or a pharmaceutically acceptable salt or an *in vivo* hydrolysable ester thereof.

According to a further feature of the present invention there is provided a compound of formula (**I**) wherein:
**R¹** is ethyl, propyl, isopropyl, butyl, iso-butyl, sec-butyl, t-butyl, cyclopropyl, cyclopropylmethyl, 1-cyclopropylethyl, cyclobutylmethyl, cyclopentyl or cyclobutyl; wherein R¹ may be optionally substituted on carbon by one or more R⁶;
**R²** is methyl, ethyl, isopropyl, fluoromethyl, difluoromethyl, trifluoromethyl, methoxymethyl, cyclopropylmethyl or cyclopropyl;
**R³** is hydrogen or halo;
**R⁴** is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
**Ring A** is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
R⁵ is a substituent on carbon and is selected from halo, cyano, hydroxy, trifluoromethoxy, amino, carboxy, carbamoyl, sulphamoyl, C₁₋₆alkyl, C₂-₆alkenyl, C₂-₆alkynyl, C₁₋₆alkanoyl, *N-*(C₁₋₆alkyl)carbamoyl, *N,N-*(C₁₋₆alkyl)₂Carbamoyl, C₁-₆alkylS(O)ₐ wherein a is 0 to 2, C₁₋₆alkylsulphonyloxy, C₁₋₆alkoxycarbonyl, carbocyclyl, heterocyclyl, *N-*(C₁₋₆alkyl)sulphamoyl or *N,N-*(C₁₋₆alkyl)₂Sulphamoyl; wherein R⁵ independently may be optionally substituted on carbon by one or more R⁸; wherein if said heterocyclyl contains an -NH- moiety that nitrogen may be optionally substituted by R¹⁵; or R⁵ is -NHR⁹, -NR¹⁰R¹¹ or -O-R¹²;
**n** is 0-2; wherein the values of R⁵ maybe the same or different;
**R⁶** is selected from halo, methoxy and hydroxy;
**R⁷,R⁹, R¹⁰, R¹¹, R ¹²** and **R¹⁵** are independently selected from C₁-₄alkyl, C₁-₄akanoyl, C₁-₄alkylsulphonyl, C₂₋₄alkenylsulphonyl, C₂-₄akynylsulphonyl, C₁₋4alkoxycarbonyl, carbamoyl, *N-*(C₁₋₄alkyl)carbamoyl, *N,N-*(C₁₋₄alkyl)carbamoyl, carbocyclyl or heterocyclyl; wherein R⁷, R⁹, R¹⁰, R¹¹, R¹², and R¹⁵ may be independently optionally substituted on carbon by a group selected from R¹³; and wherein if said heterocyclyl contains an -NH- moiety that nitrogen may be optionally substituted by R¹⁴;
R⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N-*methyl-*N-*ethylamino, acetylamino, *N-*methylcarbamoyl, *N-*ethylcarbamoyl, *N,N-*dimethylcarbamoyl, *N,N-*diethylcarbamoyl, *N-*methyl-*N-*ethylcarbamoyl, methylthio, ethylthio, methylsulphinyl, ethylsulphinyl, mesyl, ethylsulphonyl, methoxycarbonyl, ethoxycarbonyl, *N-*methylsulphamoyl, *N-*ethylsulphamoyl, *N,N-*dimethylsulphamoyl, *N,N-*diethylsulphamoyl or *N-*methyl-*N-*ethylsulphamoyl;
**R**¹³ is selected from halo, cyano, hydroxy, amino, trifluoromethyl, trifluoromethoxy, dimethylamino, carbocyclyl, heterocyclyl, C₁-₃alkyl and C₁-₃alkoxy; and
**R**¹⁴ is selected from C₁-₃akyl, C₁-₃akanoyl, C₁₋₃alkylsulphonyl, C₁₋₃alkoxycarbonyl, carbamoyl, *N-*(C₁₋₃alkyl)carbamoyl and *N,N-*(C₁₋₃alkyl)carbamoyl;
or a pharmaceutically acceptable salt or an *in vivo* hydrolysable ester thereof.

In this specification the term "alkyl" includes both straight and branched chain alkyl groups but references to individual alkyl groups such as "propyl" are specific for the straight chain version only. For example, "C₁₋₆alkyl" and "C₁-₄alkyl" include methyl, ethyl, propyl, isopropyl and t-butyl. "C₁-₃alkyl" includes methyl, ethyl, propyl and isopropyl. However, references to individual alkyl groups such as 'propyl' are specific for the straight chained version only and references to individual branched chain alkyl groups such as 'isopropyl' are specific for the branched chain version only. A similar convention applies to other radicals. The term "halo" refers to fluoro, chloro, bromo and iodo.

Where optional substituents are chosen from "one or more" groups it is to be understood that this definition includes all substituents being chosen from one of the specified groups or the substituents being chosen from two or more of the specified groups.

A "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂- group can optionally be replaced by a -C(O)-, a ring nitrogen atom may optionally bear a C₁₋₆alkyl group and form a quaternary compound or a ring nitrogen and/or sulphur atom may be optionally oxidised to form the *N-*oxide and or the S-oxides. Examples and suitable values of the term "heterocyclyl" are morpholino, piperidyl, pyridyl, pyranyl, pyrrolyl, isothiazolyl, indolyl, quinolyl, thienyl, 1,3-benzodioxolyl, thiadiazolyl, piperazinyl, thiazolidinyl, pyrrolidinyl, thiomorpholino, pyrrolinyl, homopiperazinyl, 3,5-dioxapiperidinyl, tetrahydropyranyl, imidazolyl, pyrimidyl, pyrazinyl, pyridazinyl, isoxazolyl, *N-*methylpyrrolyl, 4-pyridone, 1-isoquinolone, 2-pyrrolidone, 4-thiazolidone, pyridine-N oxide and quinoline-N oxide. In one aspect of the invention a "heterocyclyl" is a saturated, partially saturated or unsaturated, mono or bicyclic ring containing 5 or 6 atoms of which at least one atom is chosen from nitrogen, sulphur or oxygen, it may, unless otherwise specified, be carbon or nitrogen linked, a -CH₂-group can optionally be replaced by a -C(O)-and a ring sulphur atom may be optionally oxidised to form the S-oxides.

A "carbocyclyl" is a saturated, partially saturated or unsaturated, mono or bicyclic carbon ring that contains 3-12 atoms; wherein a -CH₂- group can optionally be replaced by a -C(O)-. Particularly "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl.

Ring A is a "nitrogen linked 4-7 membered saturated ring which optionally contains an additional nitrogen, oxygen or sulphur atom". A "nitrogen linked 4-7 membered saturated ring which optionally contains an additional nitrogen, oxygen or sulphur atom" is a saturated monocyclic ring containing 4-7 atoms linked to the phenyl moiety of formula (I) via a nitrogen atom contained in the ring, the ring optionally contains an additional heteroatom selected from nitrogen, sulphur or oxygen, wherein a -CH₂- group can optionally be replaced by a -C(O)-, and the optional sulphur atom may be optionally oxidised to form the S-oxides. A "nitrogen linked 5 or 6 membered saturated ring which optionally contains an additional nitrogen, oxygen or sulphur atom" is defined as for a "nitrogen linked 4-7 membered saturated ring which optionally contains an additional nitrogen, oxygen or sulphur atom" but wherein the ring has only 5 or 6 atoms.

Two atoms of Ring A, when Ring A is a nitrogen linked 5-7 membered saturated ring, may optionally be connected by a one or two atom bridge. A bridge is an atom or two atoms connecting two different parts of a molecule. The "one or two atom bridge" may be made up of one or two carbon atoms, or one heteroatom or one heteroatom and one carbon atom. The heteroatom is selected from oxygen, nitrogen or sulphur. Particularly the bridge is one oxygen atom. Alternatively the bridge is one carbon atom. Examples of a "nitrogen linked 5-7 membered saturated ring which optionally contains an additional nitrogen, oxygen or sulphur atom .... connected by a one or two atom bridge" include 8-oxa-3-azabicyclo[3.2.1]octan-3-yl, 2,5-diazabicyclo[2.2.1]heptan-2-yl and 3-azabicyclo[3.2.1]octan-3-yl.

Examples of "C₁₋₆alkoxycarbonyl" and "C₁₋₄alkoxycarbonyl" include methoxycarbonyl, ethoxycarbonyl, n- and t-butoxycarbonyl. Examples of "C₁_₃alkoxycarbonyl" include methoxycarbonyl and ethoxycarbonyl. Examples of "C₁-₃alkoxy" include methoxy, ethoxy and propoxy. Examples of "C₁-₆alkyIS(O)ₐ wherein a is 0 to 2" include methylthio, ethylthio, methylsulphinyl, ethylsulphinyl, mesyl and ethylsulphonyl. Examples of "C₁-₆alkanoyl", "C₁₋₄alkanoyl" and "C₁-₃alkanoyl" include propionyl and acetyl. Examples of "C₂-₆alkenyl" are vinyl, allyl and 1-propenyl. Examples of "C₂-₆alkynyl" are ethynyl, 1-propynyl and 2-propynyl. Examples of "N-(Cₗ-₆alkyl)sulphamoyl" are *N-*(methyl)sulphamoyl and *N*-(ethyl)sulphamoyl. Examples of "*N,N*-(C₁-₆alkyl)₂sulphamoyl" are *N,N-*(dimethyl)sulphamoyl and *N-*(methyl)-*N* (ethyl)sulphamoyl. Examples of "N-(C₁-₆alkyl)carbamoyl", "*N* (C₁₋₄alkyl)carbamoyl" and *"N* (C₁₋₃alkyl)carbamoyl" are methylaminocarbonyl and ethylaminocarbonyl. Examples of "*N,N-*(C₁₋₆alkyl)₂carbamoyl", "*N,N*-(C₁₋₄alkyl)₂carbamoyl" and "*N,N-*(C₁₋₃alkyl)₂carbamoyl" are dimethylaminocarbonyl andmethylethylaminocarbonyl. Examples of "C₁-₄alkylsulphonyl" and include methylsulphonyl, isopropylsulphonyl and t-butylsulphonyl. Examples of "C₁-₃alkylsulphonyl" and include methylsulphonyl and isopropylsulphonyl. Examples of "C₁-₄alkenylsulphonyl" include ethenylsulphonyl and allylsulphonyl. Examples of "C₁₋₄alknylsulphonyl" include ethynylsulphonyl and propynylsulphonyl. Examples of "C₁₋₆alkylsulphonyloxy" are mesyloxy and isopropylsulphonyloxy.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

*An in vivo* hydrolysable ester of a compound of the formula (I) containing carboxy or hydroxy group is, for example, a pharmaceutically acceptable ester which is hydrolysed in the human or animal body to produce the parent acid or alcohol. Suitable pharmaceutically acceptable esters for carboxy include C₁₋₆alkoxymethyl esters for example methoxymethyl, C₁₋₆alkanoyloxymethyl esters for example pivaloyloxymethyl, phthalidyl esters, C₃₋₈cycloalkoxycarbonyloxyCₗ_₆alkyl esters for example 1-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyloxyethyl and may be formed at any carboxy group in the compounds of this invention.

*An in vivo* hydrolysable ester of a compound of the formula (**I**) containing a hydroxy group includes inorganic esters such as phosphate esters and α-acyloxyalkyl ethers and related compounds which as a result of *the in vivo* hydrolysis of the ester breakdown to give the parent hydroxy group. Examples of α-acyloxyalkyl ethers include acetoxymethoxy and 2,2-dimethylpropionyloxy-methoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and *N*-(dialkylaminoethyl)-N-alkylcarbamoyl (to give carbamates), dialkylaminoacetyl and carboxyacetyl. Examples of substituents on benzoyl include morpholino and piperazino linked from a ring nitrogen atom via a methylene group to the 3- or 4- position of the benzoyl ring.

Some compounds of the formula (**I**) may have chiral centres and/or geometric isomeric centres (E- and Z- isomers), and it is to be understood that the invention encompasses all such optical, diastereoisomers and geometric isomers that possess CDK inhibitory activity.

The invention relates to any and all tautomeric forms of the compounds of the formula (I) that possess CDK inhibitory activity.

It is also to be understood that certain compounds of the formula (**I**) can exist in solvated as well as unsolvated forms such as, for example, hydrated forms. It is to be understood that the invention encompasses all such solvated forms which possess CDK inhibitory activity.

Particular values of variable groups are as follows. Such values may be used where appropriate with any of the definitions, claims or embodiments defined hereinbefore or hereinafter.
R¹ is ethyl, isopropyl, cyclopropylmethyl, 1-cyclopropylethyl or cyclobutyl.
R¹ is ethyl, isopropyl, cyclopropylmethyl, 1-cyclopropylethyl, cyclobutylmethyl, cyclopentyl or cyclobutyl.
R¹ is ethyl.
R¹ is isopropyl.
R¹ is cyclopropylmethyl.
R¹ is 1-cyclopropylethyl.
R¹ is cyclobutyl.
R¹ is cyclopentyl.
R¹ is cyclobutyl.
R² is methyl, ethyl, isopropyl, difluoromethyl, trifluoromethyl, methoxymethyl or cyclopropyl.
R² is methyl or methoxymethyl.
R² is methyl.
R² is ethyl.
R² is isopropyl.
R² is difluoromethyl.
R² is trifluoromethyl.
R² is methoxymethyl.
R² is cyclopropyl.
R³ is hydrogen or fluoro.
R³ is hydrogen, fluoro or chloro.
R³ is hydrogen.
R³ is fluoro.
R³ is chloro.
R⁴ is hydrogen, halo, cyano, mesyl, methyl or methoxy.
R⁴ is hydrogen, fluoro, chloro, cyano, mesyl, methyl or methoxy.
R⁴ is hydrogen.
R⁴ is halo.
R4 is fluoro.
R⁴ is chloro.
R⁴ is cyano.
R⁴ is mesyl.
R⁴ is methyl.
R⁴ is methoxy.
Ring A is a nitrogen linked 5 or 6 membered saturated ring which optionally contains an additional nitrogen, oxygen or sulphur atom; wherein if Ring A contains an additional nitrogen atom that nitrogen may be optionally substituted by R⁷; wherein R⁷ is C₁₋₄alkyl.
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷; wherein
R⁷ is selected from C₁-₄alkyl, carbocyclyl or heterocyclyl; wherein R⁷ may be optionally substituted on carbon by one or more groups selected from R¹³;
R¹³ is selected from halo, hydroxy, C₁-₃alkyl, C₁-₃alkoxy, dimethylamino or heterocyclyl.
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷; wherein
R⁷ is selected from C₁-₄alkyl or carbocyclyl; wherein R⁷ may be optionally substituted on carbon by a group selected from R^{13;}
R¹³ is selected from hydroxy, C₁₋₃alkoxy, dimethylamino or heterocyclyl.
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷; wherein
R⁷ is selected from C₁-₄alkyl or carbocyclyl; wherein R⁷ may be optionally substituted on carbon by a group selected from R¹³;
R¹³ is selected from C₁₋₃alkoxy, dimethylamino or heterocyclyl.
Ring A is a nitrogen linked 4-7 membered saturated ring which optionally contains an additional nitrogen, oxygen or sulphur atom; wherein if Ring A contains an additional nitrogen atom that nitrogen may be optionally substituted by R⁷; wherein
R⁷ is selected from C₁₋₄alkyl or carbocyclyl; wherein R⁷ may be optionally substituted on carbon by a group selected from R¹³;
R¹³ is selected from C₁₋₃alkoxy, dimethylamino or heterocyclyl.
Ring A is morpholino, piperazin-1-yl or pyrrolidin-1-yl; wherein said piperazin-1-yl may be optionally substituted on nitrogen by R⁷; wherein R⁷ is C₁-₄alkyl.
Ring A is morpholino, 1, 1-dioxothiomorpholino, piperidin-1-yl, 1,4-diazepan-l-yl, azetidin-1-yl, piperazin-1-yl, 1,4-oxazepan-4-yl, 8-oxa-3-azabicyclo[3.2.1]oct-3-yl, pyrrolidin-1-yl or 2,5-diazabicyclo[2.2. l]hept-5-yl; wherein Ring A may be optionally substituted on nitrogen by R⁷; wherein
R⁷ is selected from methyl, ethyl, isopropyl, cyclopropyl, cyclobutyl, phenyl or pyridyl; wherein R⁷ may be optionally substituted on carbon by one or more groups selected from R¹³;
R¹³ is selected from fluoro, chloro, hydroxy, methyl, methoxy, dimethylamino or pyrrolidin-1-yl.
Ring A is morpholino, l,l-dioxothiomorpholino, piperidin-1-yl, 1,4-diazepan-1-yl, azetidin-1-yl, piperazin-1-yl, 1,4-oxazepan-4-yl, 8-oxa-3-azabicyclo[3.2.1]oct-3-yl, pyrrolidin-1-yl or 2,5-diazabicyclo[2.2.1]hept-5-yl; wherein said 1,4-diazepan-1-yl, piperazin-1-yl or 2,5-diazabicyclo[2.2.1 ]hept-5-yl may be optionally substituted on nitrogen by R⁷; wherein
R⁷ is selected from methyl, ethyl, isopropyl, cyclopropyl or cyclobutyl; wherein R⁷ may be optionally substituted on carbon by a group selected from R¹³;
R¹³ is selected from hydroxy, methoxy, dimethylamino or pyrrolidin-1-yl.
Ring A is morpholino, 1,1-dioxothiomorpholino, piperidin-1-yl, 1,4-diazepan-l-yl, azetidin-1-yl, 1,1-dioxothimorpholino, piperazin-1-yl, 1,4-oxazepan-4-yl, 8-oxa-3-azabicyclo[3.2.1]oct-3-yl or pyrrolidin-1-yl; wherein said 1,4-diazepan-1-yl or piperazin-1-yl may be optionally substituted on nitrogen by R⁷; wherein
R⁷ is selected from methyl, ethyl, isopropyl or cyclopropyl; wherein R⁷ may be optionally substituted on carbon by a group selected from R¹³;
R¹³ is selected from methoxy, dimethylamino or pyrrolidin-1-yl.
Ring A is morpholino, piperidin-1-yl, 1,4-diazepan-1-yl, azetidin-1-yl, 1,l-dioxothimorpholino, piperazin- 1-yl or pyrrolidin-1-yl; wherein said 1,4-diazepan-1-yl or piperazin-1-yl may be optionally substituted on nitrogen by R⁷; wherein
R⁷ is selected from methyl, ethyl or cyclopropyl; wherein R⁷ may be optionally substituted on carbon by a group selected from R¹³;
R¹³ is selected from methoxy, dimethylamino or pyrrolidin-1-yl.
Ring A is morpholino, piperazin-1-yl or pyrrolidin-1-yl; wherein said piperazin-1-yl may be optionally substituted on nitrogen by R⁷; wherein R⁷ is methyl.
Ring A is morpholino, 4-methylpiperazin-1-yl or pyrrolidin-1-yl.
Ring A is morpholino, 1,1-dioxothimorpholino, piperidin-1-yl, piperazin-1-yl, azetidin-1-yl, 4-methyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-cyclopropylhomopiperazin-1-yl, 4-cyclobutylhomopiperazin-1-yl, 4-(2-hydroxyethyl)homopiperazin-1-yl, 4-isopropylhomopiperazin-1-yl, 1,4-oxazepan-4-yl, 8-oxa-3-azabicyclo[3.2.1]oct-3-yl, pyrrolidin-1-yl, 2,5-diazabicyclo[2.2.1]hept-5-yl or 2-ethyl-2,5-diazabicyclo[2.2.1 ]hept-5-yl.
Ring A is morpholino, 1,l-dioxothimorpholino, piperidin-1-yl, piperazin-1-yl, azetidin-1-yl, 4-methyl-1,4-diazepan-1-yl, 4-ethyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-(4-fluorophenyl)piperazin- 1-yl, 4-(2-fluorophenyl)piperazin-1-yl, 4-(2,4-difluorophenyl)piperazin-1-yl, 4-(3,4-difluorophenyl)piperazin-1-yl, 4-(2-chlorophenyl)piperazin-1-yl, 4-(4-chlorophenyl)piperazin-1-yl, 4-(4-phenyl)piperazin-1-yl, 4-(2-methoxyphenyl)piperazin-1-yl, 4-(3-methoxyphenyl)piperazin-1-yl, 4-(4-methoxyphenyl)piperazin-1-yl, 4-(3-methylphenyl)piperazin-1-yl, 4-(2-methylphenyl)piperazin-1-yl, 4-(4-methylphenyl)piperazin-1-yl, 4-(2,3-dimethylphenyl)piperazin-1-yl, 4-(2,6-dimethylphenyl)piperazin-1-yl, 4-(4-hydroxyphenyl)piperazin-1-yl, 4-(2-hydroxyphenyl)piperazin-1-yl, 4-(5-chloropyrid-2-yl)piperazin-1-yl, 4-cyclopropylhomopiperazin-1-yl, 4-cyclobutylhomopiperazin-1-yl, 4-(2-hydroxyethyl)homopiperazin-1-yl, 4-(2-methoxyethyl)homopiperazin-1-yl, 4-isopropylhomopiperazin-1-yl, 1,4-oxazepan-4-yl, 8-oxa-3-azabicyclo[3.2.1]oct-3-yl, pyrrolidin-1-yl, 2,5-diazabicyclo[2.2.1]hept-5-yl, 2-methyl-2,5-diazabicyclo[2.2.1 ]hept-5-yl, 2-(2-methoxyethyl)-2,5-diazabicyclo[2.2.1 ]hept-5-yl, 2-ethyl-2,5-diazabicyclo[2.2.1 ]hept-5-yl or 2-isopropyl-2,5-diazabicyclo[2.2.1]hept-5-yl.
Ring A is morpholino, 1,l-dioxothimorpholino, piperidin-1-yl, piperazin-1-yl, azetidin-1-yl, 4-methyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-isopropylhomopiperazin-1-yl, 1,4-oxazepan-4-yl, 8-oxa-3-azabicyclo[3.2.1]oct-3-yl or pyrrolidin-1-yl.
Ring A is morpholino, 1,1-dioxothimorpholino, piperidin-1-yl, piperazin-1-yl, azetidin-1-yl, 4-methyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl or pyrrolidin-1-yl.
Ring A is morpholino.
Ring A is 4-methylpiperazin-1-yl.
Ring A is pyrrolidin-1-yl.
R⁵ is -NR¹⁰R¹¹; wherein R¹⁰ and R¹¹ are independently selected from C₁-₄alkyl.
R⁵ is a substituent on carbon and is selected from hydroxy, amino, C₁₋₆alkyl, C₁₋₆alkylsulphonyloxy, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵; wherein
R⁶ is selected from halo, methoxy and hydroxy;
R⁹, R^{l0}, R¹¹ and R¹⁵ are independently selected from C₁-₄alkyl or carbocyclyl; wherein R⁹, R¹⁰ R¹¹ and R¹⁵ may be independently optionally substituted on carbon by one or more groups selected from R¹³_{;}
R⁸ is selected from hydroxy, amino and phenylamino; and
R¹³ is selected from carbocyclyl and C₁-₃alkoxy.
R⁵ is a substituent on carbon and is selected from hydroxy, amino, C₁₋₆alkyl, C₁₋₆alkylsulphonyloxy, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵; wherein
R⁶ is selected from halo, methoxy and hydroxy;
R⁹, R¹⁰,R¹¹and R¹⁵ are independently selected from C₁₋₄alkyl or carbocyclyl;
R⁸ is selected from hydroxy and amino.
R⁵ is a substituent on carbon and is selected from hydroxy, amino, C₁-₆alkyl, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵; wherein
R⁶ is selected from halo, methoxy and hydroxy;
R⁹, R¹⁰, R¹¹ and R¹⁵ are independently selected from C₁₋₄alkyl;
R⁸ is selected from hydroxy and amino.
R⁵ is -NR¹⁰R¹¹; wherein R¹⁰ and R¹¹ are methyl.
R⁵ is a substituent on carbon and is selected from hydroxy, amino, methyl, mesyl, mesyloxy, morpholino, piperidin-1-yl, pyrid-2-yl, homopiperazin-1-yl, piperazin-1-yl or pyrrolidin-1-yl; wherein R⁵ independently may be optionally substituted on carbon by one or more R⁸; wherein R⁵ may be optionally substituted on nitrogen by R¹⁵; or R⁵ is -NHR⁹ or-NR¹⁰R¹¹; wherein
R⁶ is selected from halo, methoxy and hydroxy;
R⁹, R¹⁰, R¹¹ and R¹⁵ are independently selected from methyl, ethyl, propyl, isopropyl, isobutyl, cyclopropyl or cyclobutyl; wherein R⁹, R¹⁰, R¹¹ and R¹⁵ may be independently optionally substituted on carbon by one or more groups selected from R¹³;
R⁸ is selected from hydroxy, amino and phenylamino; and
R¹³ is selected from cyclopropyl and methoxy.
R⁵ is a substituent on carbon and is selected from hydroxy, amino, methyl, mesyl, mesyloxy, morpholino, piperidin-1-yl, piperazin-1-yl or pyrrolidin-1-yl; wherein R⁵ independently may be optionally substituted on carbon by one or more R⁸; wherein said piperazin-1-yl may be optionally substituted on nitrogen by R¹⁵; or R⁵ is -NHR⁹ or-NR¹⁰R¹¹; wherein
R⁶ is selected from halo, methoxy and hydroxy;
R⁹, R¹⁰, R¹¹ and R¹⁵ are independently selected from methyl or cyclopropyl;
R⁸ is selected from hydroxy and amino.
R⁵ is a substituent on carbon and is selected from hydroxy, amino, methyl, mesyl, piperazin-1-yl or pyrrolidin-1-yl; wherein R⁵ independently may be optionally substituted on carbon by one or more R⁸; wherein said piperazin-1-yl may be optionally substituted on nitrogen by R¹⁵; or R⁵ is -NHR⁹ or- NR¹⁰R¹¹ wherein
R⁶ is selected from halo, methoxy and hydroxy;
R⁹, R¹⁰, R¹¹ and R¹⁵ are independently selected from methyl;
R⁸ is selected from hydroxy and amino.
R⁵ is a substituent on carbon and is selected from hydroxy, amino, methyl, mesyl, mesyloxy, morpholino, piperidin-1-yl, dimethylamino, diethylamino, isopropyl, pyrid-2-yl, hydroxymethyl, methylamino, aminomethyl, 4-methylpiperazin-1-yl, cyclopropylamino, pyrrolidin-1-yl, homopiperazin-1-yl, cyclobutylamino, phenylaminomethyl, *N-*methyl-*N-*(cyclopropylmethyl)amino, *N-*methyl-*N-*cyclopropylamino, *N-*methyl-*N-*isobutylamino, *N-*methyl-*N-*(2-methoxyethyl)amino, *N-*ethyl-*N-*propylamino or *N-*methyl-*N-*cyclobutylamino.
R⁵ is a substituent on carbon and is selected from hydroxy, amino, methyl, mesyl, mesyloxy, morpholino, piperidin-1-yl, dimethylamino, hydroxymethyl, methylamino, aminomethyl, 4-methylpiperazin-1-yl, cyclopropylamino or pyrrolidin-1-yl.
R⁵ is a substituent on carbon and is selected from hydroxy, amino, methyl, mesyl, dimethylamino, hydroxymethyl, methylamino, aminomethyl, 4-methylpiperazin-1-yl or pyrrolidin-1-yl.
n is 0 or 1.
n is 0.
n is 1.
n is 2; wherein the values of R⁵ maybe the same or different.

Therefore in a further aspect of the invention there is provided a compound of formula (**I**) (as depicted above) wherein:
R¹ is ethyl, isopropyl, cyclopropylmethyl, 1-cyclopropylethyl or cyclobutyl;
R² is methyl or methoxymethyl;
R³ is hydrogen or fluoro;
R⁴ is hydrogen, halo, cyano, mesyl, methyl or methoxy;
Ring A is a nitrogen linked 5 or 6 membered saturated ring which optionally contains an additional nitrogen, oxygen or sulphur atom; wherein if Ring A contains an additional nitrogen atom that nitrogen may be optionally substituted by R⁷; wherein R⁷ is C₁₋₄alkyl;
R⁵ is -NR¹⁰R¹¹; wherein R¹⁰and R¹¹ are independently selected from C₁₋₄alkyl; and
n is 0 or 1;
or a pharmaceutically acceptable salt or an *in vivo* hydrolysable ester thereof.

Therefore in a further aspect of the invention there is provided a compound of formula (I) (as depicted above) wherein:
R¹ is ethyl, isopropyl, cyclopropylmethyl, 1-cyclopropylethyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, halo, cyano, mesyl, methyl or methoxy;
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
R⁵ is a substituent on carbon and is selected from hydroxy, amino, C₁-₆alkyl, C₁-₆alkylS(O)ₐ wherein a is 2 or heterocyclyl; wherein R⁵ independently may be optionally substituted on carbon by one or more R⁸; or R⁵ is -NHR⁹ or - NR¹⁰R¹¹; wherein if said heterocyclyl contains an -NH- moiety that nitrogen may be optionally substituted by R¹⁵;
n is 0 or 1;
R⁷ is selected from C₁-₄alkyl or carbocyclyl; wherein R⁷ may be optionally substituted on carbon by a group selected from R¹³;
R⁸ is selected from hydroxy and amino;
R⁹, R¹⁰, R¹¹ and R¹⁵ are independently selected from C₁₋₄alkyl; and
R¹³ is selected from C₁₋₃alkoxy, dimethylamino or heterocyclyl;
or a pharmaceutically acceptable salt or an *in vivo* hydrolysable ester thereof.

Therefore in a further aspect of the invention there is provided a compound of formula (I) (as depicted above) wherein:
R¹ is ethyl, isopropyl, cyclopropylmethyl, 1-cyclopropylethyl or cyclobutyl;
R² is methyl, ethyl, isopropyl, difluoromethyl, trifluoromethyl, methoxymethyl or cyclopropyl;
R³ is hydrogen or fluoro;
R⁴ is hydrogen, halo, cyano, mesyl, methyl or methoxy;
Ring A is a nitrogen linked 4-7 membered saturated ring which optionally contains an additional nitrogen, oxygen or sulphur atom; wherein if Ring A contains an additional nitrogen atom that nitrogen may be optionally substituted by R⁷;
R⁵ is a substituent on carbon and is selected from hydroxy, amino, C₁₋₆alkyl, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵;
n is 0 or 1;
R⁷ is selected from C₁-₄alkyl or carbocyclyl; wherein R⁷ may be optionally substituted on carbon by a group selected from R¹³;
R⁸ is selected from hydroxy and amino;
R⁹, R¹⁰, R¹¹ and R¹⁵ are independently selected from C₁₋₄alkyl; and
R¹³ is selected from C₁₋₃alkoxy, dimethylamino or heterocyclyl;
or a pharmaceutically acceptable salt or an *in vivo* hydrolysable ester thereof.

Therefore in a further aspect of the invention there is provided a compound of formula (**I**) (as depicted above) wherein:
R¹ is ethyl, isopropyl, cyclopropylmethyl, 1-cyclopropylethyl, cyclobutylmethyl, cyclopentyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, halo, cyano, mesyl, methyl or methoxy;
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
R⁵ is a substituent on carbon and is selected from hydroxy, amino, C₁₋₆alkyl, C₁₋₆alkylsulphonyloxy, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵_{;}
n is 0 or 1;
R⁶ is selected from halo, methoxy and hydroxy;
R⁷ is selected from C₁-₄alkyl or carbocyclyl; wherein R⁷ may be optionally substituted on carbon by a group selected from R¹³;
R⁸ is selected from hydroxy and amino;
R⁹, R¹⁰, R¹¹ and R¹⁵ are independently selected from C₁-₄alkyl or carbocyclyl;
R¹³ is selected from hydroxy, C₁₋₃alkoxy, dimethylamino or heterocyclyl;
or a pharmaceutically acceptable salt or an *in vivo* hydrolysable ester thereof.

Therefore in a further aspect of the invention there is provided a compound of formula (**I**) (as depicted above) wherein:
R¹ is ethyl, isopropyl, cyclopropylmethyl, 1-cyclopropylethyl, cyclobutylmethyl, cyclopentyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, halo, cyano, mesyl, methyl or methoxy;
Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
R⁵ is a substituent on carbon and is selected from hydroxy, amino, C₁₋₆alkyl, C₁₋₆alkylsulphonyloxy, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R¹¹; wherein if said heterocyclyl contains an -NH- moiety that nitrogen may be optionally substituted by R¹⁵_{;}
n is 0 or 1;
R⁶ is selected from halo, methoxy and hydroxy;
R⁷ is selected from C₁-₄alkyl, carbocyclyl or heterocyclyl; wherein R⁷ may be optionally substituted on carbon by one or more groups selected from R¹³;
R⁸ is selected from hydroxy, amino and phenylamino;
R⁹, R¹⁰, R¹¹ and R¹⁵ are independently selected from C₁-₄alkyl or carbocyclyl; wherein R⁹, R¹⁰ R¹¹ and R¹⁵ may be independently optionally substituted on carbon by one or more groups selected from R¹³_{;}
R¹³ is selected from halo, carbocyclyl, hydroxy, C₁₋₃alkyl, C₁-₃akoxy, dimethylamino or heterocyclyl;
or a pharmaceutically acceptable salt or an *in vivo* hydrolysable ester thereof.

Therefore in a further aspect of the invention there is provided a compound of formula (**I**) (as depicted above) wherein
R¹ is ethyl, isopropyl, cyclopropylmethyl, 1-cyclopropylethyl or cyclobutyl;
R² is methyl or methoxymethyl;
R³ is hydrogen or fluoro;
R⁴ is hydrogen, fluoro, chloro, cyano, mesyl, methyl or methoxy;
Ring A is morpholino, 4-methylpiperazin-1-yl or pyrrolidin-1-yl;
R⁵ is -NR¹⁰ R¹¹; wherein R¹⁰ and R¹¹are methyl; and
n is 0 or 1;
or a pharmaceutically acceptable salt or an *in vivo* hydrolysable ester thereof.

Therefore in a further aspect of the invention there is provided a compound of formula (**I**) (as depicted above) wherein:
R¹ is ethyl, isopropyl, cyclopropylmethyl, 1-cyclopropylethyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, fluoro, chloro, cyano, mesyl, methyl or methoxy;
Ring A is morpholino, 1,1-dioxothimorpholino, piperidin-1-yl, piperazin-1-yl, azetidin-1-yl, 4-methyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-isopropylhomopiperazin-1-yl, 1,4-oxazepan-4-yl, 8-oxa-3-azabicyclo[3.2.1]oct-3-yl or pyrrolidin-1-yl;
R⁵ is a substituent on carbon and is selected from hydroxy, amino, methyl, mesyl, dimethylamino, hydroxymethyl, methylamino, aminomethyl, 4-methylpiperazin-1-yl or pyrrolidin-1-yl;
n is 0 or 1;
or a pharmaceutically acceptable salt or an *in vivo* hydrolysable ester thereof.

Therefore in a further aspect of the invention there is provided a compound of formula **(I)** (as depicted above) wherein:
R¹ is ethyl, isopropyl, cyclopropylmethyl, 1-cyclopropylethyl or cyclobutyl;
R² is methyl, ethyl, isopropyl, difluoromethyl, trifluoromethyl, methoxymethyl or cyclopropyl;
R³ is hydrogen or fluoro;
R⁴ is hydrogen, fluoro, chloro, cyano, mesyl, methyl or methoxy;
Ring A is morpholino, 1,1-dioxothimorpholino, piperidin-1-yl, piperazin-1-yl, azetidin-1-yl, 4-methyl- 1,4-diazepan-1-yl, 4-(2-dimethylaminoethyl)piperazin-1-yl, 4-(2-methoxyethyl)piperazin-1-y1, 4-(2-pyrrolidin-1-ylethyl)piperazin-1-y1, 4-cyclopropylpiperazin-1-y1, 4-methylpiperazin-1-y1 or pyrrolidin-1-y1;
R⁵ is a substituent on carbon and is selected from hydroxy, amino, methyl, mesyl, dimethylamino, hydroxymethyl, methylamino, aminomethyl, 4-methylpiperazin-1-y1 or pyrrolidin-1-y1;
nis0orl;
or a pharmaceutically acceptable salt or an *in vivo* hydrolysable ester thereof.

Therefore in a further aspect of the invention there is provided a compound of formula **(I)** (as depicted above) wherein:
R¹ is ethyl, isopropyl, cyclopropylmethyl, 1-cyclopropylethyl, cyclobutylmethyl, cyclopentyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, fluoro, chloro, cyano, mesyl, methyl or methoxy;
Ring A is morpholino, 1,1dioxothimorpholino, piperidin-1-y1, piperazin-1-y1, azetidin-1-y1, 4-methyl-1,4-diazepan-1-y1, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-cyclopropylhomopiperazin-1-yl, 4-cyclobutylhomopiperazin-1-yl, 4-(2-hydroxyethyl)homopiperazin-1-yl, 4-isopropylhomopiperazin-1-yl, 1,4-oxazepan-4-yl, 8-oxa-3-azabicyclo[3.2.1]oct-3-yl, pyrrolidin-1-yl, 2,5-diazabicyclo[2.2.1]hept-5-yl or 2-ethyl-2,5-diazabicyclo[2.2.1]hept-5-yl.
R⁵ is a substituent on carbon and is selected from hydroxy, amino, methyl, mesyl, mesyloxy, morpholino, piperidin-1-yl, dimethylamino, hydroxymethyl, methylamino, aminomethyl, 4-methylpiperazin-1-yl, cyclopropylamino or pyrrolidin-1-yl;
nis0orl;
or a pharmaceutically acceptable salt or an *in vivo* hydrolysable ester thereof.

Therefore in a further aspect of the invention there is provided a compound of formula **(I)** (as depicted above) wherein:
R¹ is ethyl, isopropyl, cyclopropylmethyl, 1-cyclopropylethyl, cyclobutylmethyl, cyclopentyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, fluoro, chloro, cyano, mesyl, methyl or methoxy;
Ring A is morpholino, 1,1-dioxothimorpholino, piperidin-1-yl, piperazin-1-yl, azetidin-1-yl, 4-methyl-1,4-diazepan-1-yl, 4-ethyl-1,4-diazepan-1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-(4-fluorophenyl)piperazin-1-yl, 4-(2-fluorophenyl)piperazin-1-yl, 4-(2,4-difluorophenyl)piperazin-1-yl, 4-(3,4-difluorophenyl)piperazin-1-yl, 4-(2-chlorophenyl)piperazin-1-yl, 4-(4-chlorophenyl)piperazin-1-yl, 4-(4-phenyl)piperazin-1-yl, 4-(2-methoxyphenyl)piperazin-1-yl, 4-(3-methoxyphenyl)piperazin-1-yl, 4-(4-methoxyphenyl)piperazin-1-yl, 4-(3-methylphenyl)piperazin-1-yl, 4-(2-methylphenyl)piperazin-1-yl, 4-(4-methylphenyl)piperazin-1-yl, 4-(2,3-dimethylphenyl)piperazin-1-yl, 4-(2,6-dimethylphenyl)piperazin-1-yl, 4-(4-hydroxyphenyl)piperazin-1-yl, 4-(2-hydroxyphenyl)piperazin-1-yl, 4-(5-chloropyrid-2-yl)piperazin-1-yl, 4-cyclopropylhomopiperazin-1-yl, 4-cyclobutylhomopiperazin-1-yl, 4-(2-hydroxyethyl)homopiperazin-1-yl, 4-(2-methoxyethyl)homopiperazin-1-yl, 4-isopropylhomopiperazin-1-yl, 1,4-oxazepan-4-yl, 8-oxa-3-azabicyclo[3.2.1]oct-3-yl, pyrrolidin-1-yl, 2,5-diazabicyclo[2.2.1]hept-5-yl, 2-methyl-2,5-diazabicyclo[2.2.1 ]hept-5-yl, 2-(2-methoxyethyl)-2,5-diazabicyclo[2.2.1]hept-5-yl, 2-ethyl-2,5-diazabicyclo[2.2.1]hept-5-yl or 2-isopropyl-2,5-diazabicyclo[2.2. 1hept-5-yl;
R⁵ is a substituent on carbon and is selected from hydroxy, amino, methyl, mesyl, mesyloxy, morpholino, piperidin-1-yl, dimethylamino, diethylamino, isopropyl, pyrid-2-yl, hydroxymethyl, methylamino, aminomethyl, 4-methylpiperazin-1-yl, cyclopropylamino, pyrrolidin-1-yl, homopiperazin-1-yl, cyclobutylamino, phenylaminomethyl, N-methyl-N-(cyclopropylmethyl)amino, N-methyl-N-cyclopropylamino, *N-*methyl-*N*-isobutylamino, *N-*methyl-*N*-(2-methoxyethyl)amino, *N*-ethyl-*N*-propylamino or N-methyl-N-cyclobutylamino;
n is 0 or 1;
or a pharmaceutically acceptable salt or an *in vivo* hydrolysable ester thereof.

In another aspect of the invention, preferred compounds of the invention are any one of the Examples or a pharmaceutically acceptable salt or an *in vivo* hydrolysable ester thereof.

In another aspect of the invention, preferred compounds of the invention are selected from:
4-(1-Isopropyl-2-methyl-1H-imidazol-5-yl)-N- {4-[(4-methyl-1,4-diazepan-1 - yl)carbonyl]phenyl}pyrimidin-2-amine;
N-(4-{[(3S)-3-(Dimethylamino)pyrrolidin-l-yl]carbonyl}phenyl)-5-fluoro-4-(l-isopropyl-2-methyl- H-imidazol-5-yl)pyrimidin-2-amine;
5-Fluoro-4-(1-isopropyl-2-methyl-1H-imidazol-5-yl)-N- {4-[(4-methyl-1,4-diazepan-1 - yl)carbonyl]phenyl}pyrimidin-2-amine;
5-Chloro-N-(4-{[(3S)-3-(dimethylamino)pyrrolidin-l-yl]carbonyl}phenyl)-4-(l-isopropyl-2-methyl-1H-imidazol-5-yl)pyrimidin-2-amine;
5-Chloro-4-(1-isopropyl-2-methyl-1H-imidazol-5-yl)-N- {4-[(4-methyl-1,4-diazepan-1 - yl)carbonyl]phenyl}pyrimidin-2-amine;
N-{4-[(4-Isopropyl-1,4-diazepan-1-yl)carbonyl]phenyl}-4-(1-isopropyl-2-methyl-1H-imidazol-5-yl)pyrimidin-2-amine;
N-(4- {[(3S)-3-(Dimethylamino)pyrrolidin-1-yl]carbonyl}phenyl)-4-(1-isopropyl-2-methyl-1H-imidazol-5-yl)pyrimidin-2-amine;
N-(4-{[(3S)-3-(Dimethylamino)pyrrolidin-1-yl]carbonyl}-3-fluorophenyl)-5-fluoro-4-(1-isopropyl-2-methyl-1H-imidazol-5-yl)pyrimidin-2-amine;
[4-[[5-Fluoro-4-(2-methyl-3-propan-2-yl-3H-imidazol-4-yl)pyrimidin-2-yl]amino]phenyl]-(4-propan-2-yl-1,4-diazepan-1-yl)methanone;
[4-[[5-Fluoro-4-(2-methyl-3-propan-2-yl-3H-imidazol-4-yl)pyrimidin-2-yl]amino]phenyl]-[(3 S)-3 -(methylamino)pyrrolidin- I -yl]methanone;
or a pharmaceutically acceptable salt or an *in vivo* hydrolysable ester thereof.

Preferred aspects of the invention are those which relate to the compound of formula (I) or a pharmaceutically acceptable salt thereof.

Another aspect of the present invention provides a process for preparing a compound of formula (I) or a pharmaceutically acceptable salt or an *in vivo* hydrolysable ester thereof which process (wherein variable groups are, unless otherwise specified, as defined in formula (I)) comprises of:
*Process a)* reaction of a pyrimidine of formula (II): 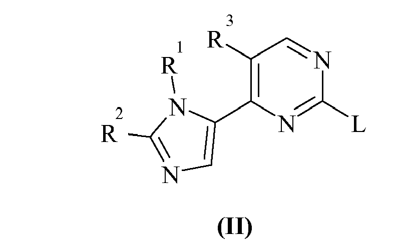 wherein L is a displaceable group; with an aniline of formula (III): 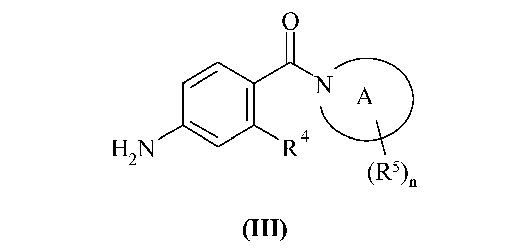 or
*Process b)* reacting a compound of formula (IV): 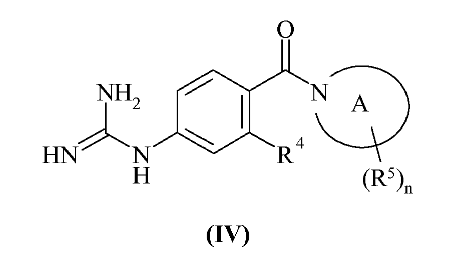 with a compound of formula (V): 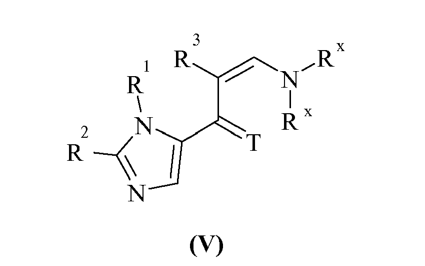 wherein T is O or S; R^{X} may be the same or different and is selected from C₁-₆alkyl; or
*Process* c) reacting an acid of formula **(VI):** 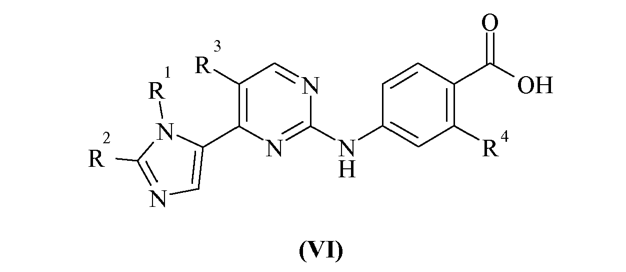 or an activated acid derivative thereof; with an amine of formula **(VII):** 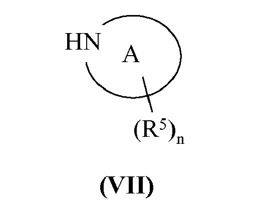 or
*Process d)* for compounds of formula (I); reacting a pyrimidine of formula **(VIII)** 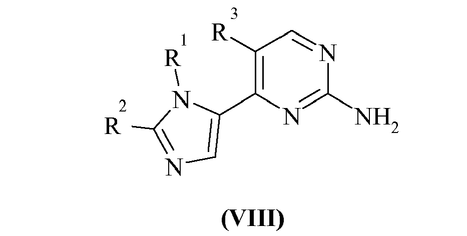 with a compound of formula **(IX):** 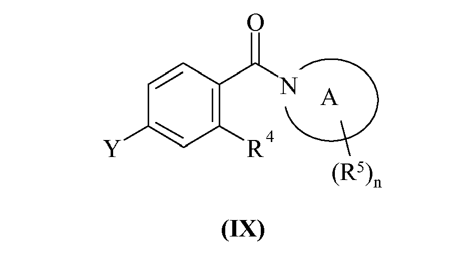 where Y is a displaceable group;
   and thereafter if necessary:
   i) converting a compound of the formula **(I)** into another compound of the formula **(I);**
   ii) removing any protecting groups;
   iii) forming a pharmaceutically acceptable salt *or in vivo* hydrolysable ester.
L is a displaceable group, suitable values for L are for example, a halogeno or sulphonyloxy group, for example a chloro, bromo, methanesulphonyloxy or toluene-4-sulphonyloxy group.
Y is a displaceable group, suitable values for Y are for example, a halogeno or sulphonyloxy group, for example a bromo, iodo or trifluoromethanesulphonyloxy group. Preferably Y is iodo.

### Specific reaction conditions for the above reactions are as follows.

*Process a)* Pyrimidines of formula **(II)** and anilines of formula **(III)** may be reacted together:
i) in the presence of a suitable solvent for example a ketone such as acetone or an alcohol such as ethanol or butanol or an aromatic hydrocarbon such as toluene or N methyl pyrrolidine, optionally in the presence of a suitable acid for example an inorganic acid such as hydrochloric acid or sulphuric acid, or an organic acid such as acetic acid or formic acid (or a suitable Lewis acid) and at a temperature in the range of 0°C to reflux, preferably reflux; or
ii) under standard Buchwald conditions (for example see J. Am. Chem. Soc., 118, 7215; J. Am. Chem. Soc., 119, 8451; J. Org. Chem., 62, 1568 and 6066) for example in the presence of palladium acetate, in a suitable solvent for example an aromatic solvent such as toluene, benzene or xylene, with a suitable base for example an inorganic base such as caesium carbonate or an organic base such as potassium-t-butoxide, in the presence of a suitable ligand such as 2,2'-bis(diphenylphosphino)-1,1'-binaphthyl and at a temperature in the range of 25 to 80°C.

### Pyrimidines of the formula (II) where L is chloro may be prepared according to Scheme 1:

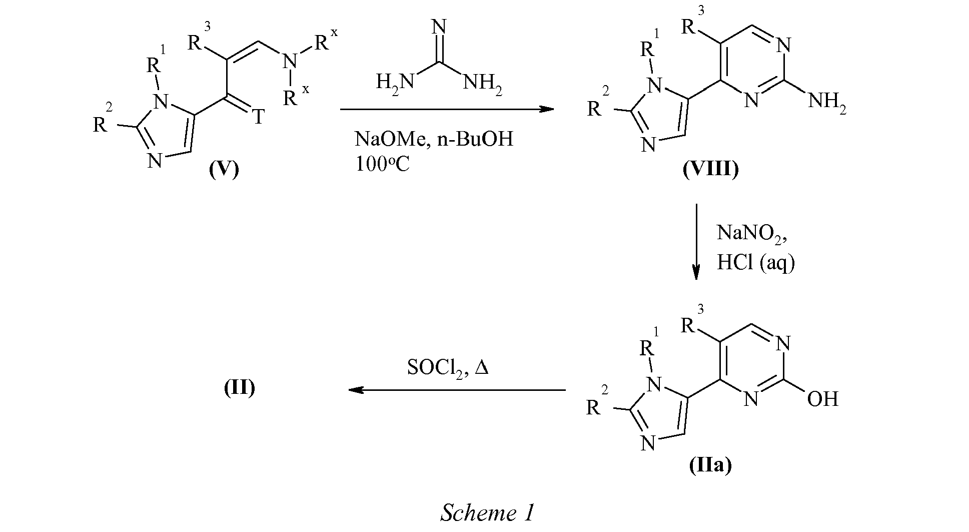

Anilines of formula **(III)** are commercially available compounds, or they are known in the literature, or they are prepared by standard processes known in the art. *Process b)* Compounds of formula **(IV)** and compounds of formula **(V)** are reacted together in a suitable solvent such as *N* methylpyrrolidinone or butanol at a temperature in the range of 100-200°C, preferably in the range of 150-170°C. The reaction is preferably conducted in the presence of a suitable base such as, for example, sodium hydride, sodium methoxide or potassium carbonate.

### Compounds of formula (V) wherein R³ is hydrogen may be prepared according to Scheme 2:

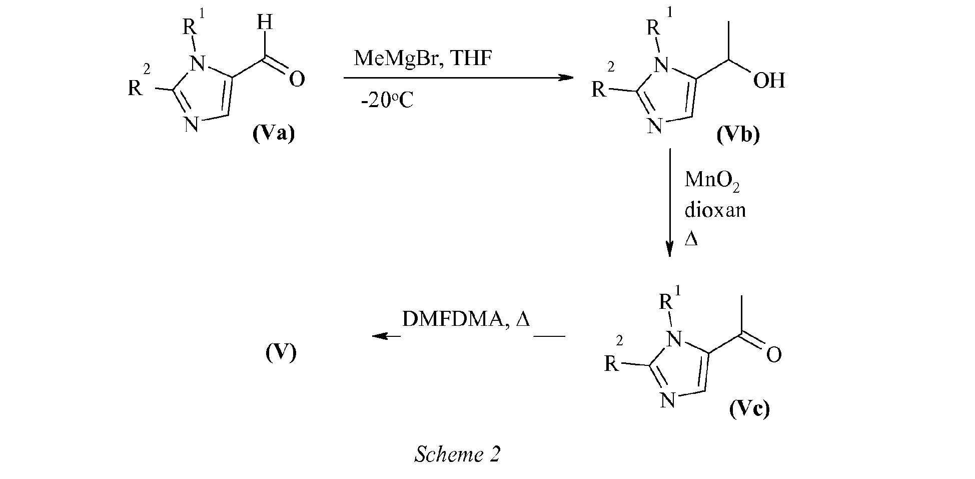

Subsequent halogenation will be required for compounds of formula **(V)** wherein R³ is halo.

Compounds of formula **(IV)** and **(Va)** are commercially available compounds, or they are known in the literature, or they are prepared by standard processes known in the art. *Process c)* Acids and amines may be coupled together in the presence of a suitable coupling reagent. Standard peptide coupling reagents known in the art can be employed as suitable coupling reagents, or for example carbonyldiimidazole and dicyclohexyl-carbodiimide, optionally in the presence of a catalyst such as dimethylaminopyridine or 4-pyrrolidinopyridine, optionally in the presence of a base for Example triethylamine, pyridine, or 2,6-di-alkyl-pyridines such as 2,6-lutidine or 2,6-di-tert-butylpyridine. Suitable solvents include dimethylacetamide, dichloromethane, benzene, tetrahydrofuran and dimethylformamide. The coupling reaction may conveniently be performed at a temperature in the range of -40 to 40°C.

Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40°C.

Compounds of formula **(VI)** may be prepared by adapting *Process a), b)* or *c).*

Amines of formula **(VII)** are commercially available compounds, or they are known in the literature, or they are prepared by standard processes known in the art.

*Process d)* Compounds of formula **(VIII)** and amines of formula **(IX)** may be reacted together under standard Buchwald conditions as described in *Process a.*

### The synthesis of compounds of formula (VIII) is described in Scheme 1.

Compounds of formula **(IX)** are commercially available compounds, or they are known in the literature, or they are prepared by standard processes known in the art.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a t-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

As stated hereinbefore the compounds defined in the present invention possesses anti-cell-proliferation activity such as anti-cancer activity which is believed to arise from the CDK inhibitory activity of the compound. These properties may be assessed, for example, using the procedure set out below:

### Assay

The following abbreviations have been used :

HEPES is N[2-Hydroxyethyl]piperazine-N-[2-ethanesulfonic acid]

DTT is Dithiothreitol

PMSF is Phenylmethylsulphonyl fluoride

The compounds were tested in an *in vitro* kinase assay in 96 well format using Scintillation Proximity Assay (SPA - obtained from Amersham) for measuring incorporation of Υ-33-P]-Adenosine Triphosphate into a test substrate (GST-Retinoblastoma protein; GST-Rb). In each well was placed the compound to be tested (diluted in DMSO and water to correct concentrations) and in control wells either roscovitine as an inhibitor control or DMSO as a positive control.

Approximately 0.2µlof CDK2/Cyclin E partially-purified enzyme (amount dependent on enzyme activity) diluted in 25µl incubation buffer was added to each well then 20µl of GST-Rb/ATP/ATP33 mixture (containing 0.5µg GST-Rb and O.2µM ATP and 0.14µCi [Υ-33-P]-Adenosine Triphosphate in incubation buffer), and the resulting mixture shaken gently, then incubated at room temperature for 60 minutes.

To each well was then added 150 µL stop solution containing (0.8mg/well of Protein A-PVT SPA bead (Amersham)), 20pM/well of Anti-Glutathione Transferase, Rabbit IgG (obtained from Molecular Probes), 61mM EDTA and 50mM HEPES pH 7.5 containing 0.05% sodium azide.

The plates were sealed with Topseal-S plate sealers, left for two hours then spun at 2500rpm, 1124xg., for 5 minutes. The plates were read on a Topcount for 30 seconds per well.

The incubation buffer used to dilute the enzyme and substrate mixes contained 50mM HEPES pH7.5, 10mM MnCI₂, 1mM DTT, 100µMSodium vanadate, 100µMNaF, 10mM Sodium Glycerophosphate, BSA (lmg/ml final).

### Test substrate

In this assay only part of the retinoblastoma protein (Science 1987 Mar13;235(4794):1394-1399; Lee W.H., Bookstein R., Hong F., Young L.J., Shew J.Y., Lee E.Y.) was used, fused to a GST tag. PCR of retinoblastoma gene encoding amino acids 379-928 (obtained from retinoblastoma plasmid ATCC pLRbRNL) was performed, and the sequence cloned into pGEx 2T fusion vector (Smith D.B. and Johnson, K.S. Gene 67, 31 (1988); which contained a tac promoter for inducible expression, internal lac I^{q} gene for use in any E.Coli host, and a coding region for thrombin cleavage - obtained from Pharmacia Biotech) which was used to amplify amino acids 792-928. This sequence was again cloned into pGEx 2T.

The retinoblastoma 792-928 sequence so obtained was expressed in E.Coli (BL211 (DE3) pLysS cells) using standard inducible expression techniques, and purified as follows.

E.coli paste was resuspended in 10ml/g of NETN buffer (50mM Tris pH 7.5, 120mM NaCI, 1mM EDTA, 0.5%v/v NP-40, 1mM PMSF, 1ug/ml leupeptin 1ug/ml aprotinin and 1 *ug*/*ml* pepstatin) and sonicated for 2 x 45 seconds per 100m1 homogenate. After centrifugation, the supernatant was loaded onto a 10ml glutathione Sepharose column (Pharmacia Biotech, Herts, UK), and washed with NETN buffer. After washing with kinase buffer (50mM HEPES pH 7.5, 10mM MgC12, 1mM DTT, 1mM PMSF, 1ug/ml leupeptin, 1 ug/ml aprotinin and 1 ug/ml pepstatin) the protein was eluted with 50mM reduced glutathione in kinase buffer. Fractions containing GST-Rb(792-927) were pooled and dialysed overnight against kinase buffer. The final product was analysed by Sodium Dodeca Sulfate (SDS) PAGE (Polyacrylamide gel) using 8-16% Tris-Glycine gels (Novex, San Diego, USA).

### CDK2 and Cyclin E

The open reading frames of CDK2 and Cyclin E were isolated by reverse transcriptase-PCR using HeLa cell and activated T cell mRNA as a template and cloned into the insect expression vector pVL1393 (obtained from Invitrogen 1995 catalogue number: V 1392-20). CDK2 and cyclin E were then dually expressed [using a standard virus Baculogold co-infection technique] in the insect SF21 cell system (Spodoptera Frugiperda cells derived from ovarian tissue of the Fall Army Worm - commercially available).

### Example production of Cyclin E/CDK2

The following Example provides details of the production of Cyclin E/CDK2 in SF21 cells (in TC100 + 10% FBS(TCS) + 0.2% Pluronic) having dual infection MOI 3 for each virus of Cyclin E & CDK2.

SF21 cells grown in a roller bottle culture to 2.33 x 10⁶ cells/ml were used to inoculate 10 x 500 ml roller bottles at 0.2 x 10E6 cells/ml. The roller bottles were incubated on a roller rig at 28°C.

After 3 days (72 hrs.) the cells were counted, and the average from 2 bottles found to be 1.86 x 10E6 cells/ml. (99% viable). The cultures were then infected with the dual viruses at an MOI 3 for each virus.

The viruses were mixed together before addition to the cultures, and the cultures returned to the roller rig 28°C.

After 2 days (48 hrs.) post infection the 5 Litres of culture was harvested. The total cell count at harvest was 1.58 x 10E6 cells/ml.(99% viable). The cells were spun out at 2500rpm, 30 mins., 4°C in Heraeus Omnifuge 2.0 RS in 250 ml. lots. The supernatant was discarded.

### Partial co-purification of CDK2 and Cyclin E

Sf21 cells were resuspended in lysis buffer (50mM Tris pH 8.2, 10mM MgC1₂, 1mM DTT, 10mM glycerophosphate, 0.1mMsodium orthovanadate, 0.1mM NaF, 1mM PMSF, 1ug/mlleupeptin and 1ug/ml aprotinin) and homogenised for 2 minutes in a 10ml) Dounce homgeniser. After centrifugation, the supernatant was loaded onto a Poros HQ/M 1.4/100 anion exchange column (PE Biosystems, Hertford, UK). CDK2 and Cyclin E were coeluted at the beginning of a 0-1M NaCI gradient (run in lysis buffer minus protease inhibitors) over 20 column volumes. Co-elution was checked by western blot using both anti-CDK2 and anti-Cyclin E antibodies (Santa Cruz Biotechnology, California, US).

By analogy, assays designed to assess inhibition of CDK1 and CDK4 may be constructed. CDK2 (EMBL Accession No. X62071) may be used together with Cyclin A or Cyclin E (see EMBL Accession No. M73812), and further details for such assays are contained in PCT International Publication No. WO99/21845, the relevant Biochemical & Biological Evaluation sections of which are hereby incorporated by reference.

Although the pharmacological properties of the compounds of the formula (I) vary with structural change, in general activity possessed by compounds of the formula (I) may be demonstrated at IC₅₀ concentrations or doses in the range 250µM to 1nM.

When tested in the above in-vitro assay the CDK2 inhibitory activity of Example 14 was measured asIC₅₀= 3nM.

### In vivo activity

*The in vivo* activity of the compounds of the present invention may be assessed by standard techniques, for example by measuring inhibition of cell growth and assessing cytotoxicity.

Inhibition of cell growth may be measured by staining cells with Sulforhodamine B (SRB), a fluorescent dye that stains proteins and therefore gives an estimation of amount of protein (i.e. cells) in a well (see Boyd, M.R.(1989) Status of the NCI preclinical antitumour drug discovery screen. Prin. Prac Oncol 10:1-12). Thus, the following details are provided of measuring inhibition of cell growth:

Cells may be plated in appropriate medium in a volume of 100ml in 96 well plates; media maybe Dulbecco's Modified Eagle media for MCF-7, SK-UT-1B and SK-UT-1. The cells may be allowed to attach overnight, then inhibitor compounds added at various concentrations in a maximum concentration of 1 % DMSO (v/v). A control plate may be assayed to give a value for cells before dosing. Cells may be incubated at 37°C, (5% CO₂ for three days.

At the end of three days TCA may be added to the plates to a final concentration of 16% (v/v). Plates may be incubated at 4°C for 1 hour, the supernatant removed and the plates washed in tap water. After drying, 100m1 SRB dye (0.4% SRB in 1 % acetic acid) may be added for 30 minutes at 37°C. Excess SRB may be removed and the plates washed in 1 % acetic acid. The SRB bound to protein may be solubilised in 10mM Tris pH7.5 and shaken for 30 minutes at room temperature. The ODs may be read at 540nm, and the concentration of inhibitor causing 50% inhibition of growth determined from a semi-log plot of inhibitor concentration versus absorbance. The concentration of compound that reduced the optical density to below that obtained when the cells were plated at the start of the experiment should give the value for toxicity.

Typical IC₅₀ values for compounds of the invention when tested in the SRB assay should be in the range 1mM to 1nM.

According to a further aspect of the invention there is provided a pharmaceutical composition which comprises a pyrimidine derivative of the formula (I), or a pharmaceutically acceptable salt *or in vivo* hydrolysable ester thereof, as defined hereinbefore in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula (I) will normally be administered to a warm-blooded animal at a unit dose within the range 5-5000 mg per square meter body area of the animal, i.e. approximately 0.1-100 mg/kg, and this normally provides a therapeutically-effective dose. A unit dose form such as a tablet or capsule will usually contain, for example 1-250 mg of active ingredient. Preferably a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

According to a further aspect of the present invention there is provided a compound of the formula **(I)**, or a pharmaceutically acceptable salt *or in vivo* hydrolysable ester thereof, as defined hereinbefore for use in a method of treatment of the human or animal body by therapy.

We have found that the compounds defined in the present invention, or a pharmaceutically acceptable salt *or in vivo* hydrolysable ester thereof, are effective cell cycle inhibitors (anti-cell proliferation agents), which property is believed to arise from their CDK inhibitory properties. Accordingly the compounds of the present invention are expected to be useful in the treatment of diseases or medical conditions mediated alone or in part by CDK enzymes, i.e. the compounds may be used to produce a CDK inhibitory effect in a warm-blooded animal in need of such treatment. Thus the compounds of the present invention provide a method for treating the proliferation of malignant cells characterised by inhibition of CDK enzymes, i.e. the compounds may be used to produce an anti-proliferative and potentially apoptotic effect mediated alone or in part by the inhibition of CDKs. Particularly, an inhibitory effect is produced by preventing entry into or progression through the S phase by inhibition of CDK2, CDK4 and/or CDK6, especially CDK2 and entry into or progression through M phase by inhibition of CDK1. Apoptotic effects may also be envisaged through down-regulation of RNA polymerase II activity by inhibition of CDK1, CDK7, CDK8 and in particular, CDK9. Such a compound of the invention is expected to possess a wide range of anti-cancer properties as CDKs have been implicated in many common human cancers such as leukaemia and breast, lung, colon, rectal, stomach, prostate, bladder, pancreas and ovarian cancer. Thus it is expected that a compound of the invention will possess anti-cancer activity against these cancers. It is in addition expected that a compound of the present invention will possess activity against a range of leukaemias, lymphoid malignancies and solid tumours such as carcinomas and sarcomas in tissues such as the liver, kidney, prostate and pancreas. In particular such compounds of the invention are expected to slow advantageously the growth of primary and recurrent solid tumours of, for example, the colon, breast, prostate, lungs and skin. More particularly such compounds of the invention, or a pharmaceutically acceptable salt *or in vivo* hydrolysable ester thereof, are expected to inhibit the growth of those primary and recurrent solid tumours which are associated with CDKs, especially those tumours which are significantly dependent on CDKs for their growth and spread, including for example, certain tumours of the colon, breast, prostate, lung, vulva and skin.

It is further expected that a compound of the present invention will possess activity against other cell-proliferation diseases in a wide range of other disease states including leukaemias, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

Thus according to this aspect of the invention there is provided a compound of the formula **(I)**, or a pharmaceutically acceptable salt *or in vivo* hydrolysable ester thereof, as defined hereinbefore for use as a medicament.

In a further aspect of the invention there is provided the use of a compound of the formula **(I)**, or a pharmaceutically acceptable salt *or in vivo* hydrolysable ester thereof, as defined hereinbefore in the manufacture of a medicament for use in the production of a cell cycle inhibitory effect.

In one aspect of the invention, where a cell cycle inhibitory effect is referred to this refers to inhibition of CDK1 . In a further aspect of the invention, this refers to inhibition of CDK2. In a further aspect of the invention, this refers to inhibition of CDK4. In a further aspect of the invention, this refers to inhibition of CDKS. In a further aspect of the invention, this refers to inhibition of CDK6. In a further aspect of the invention, this refers to inhibition of CDK7. In a further aspect of the invention, this refers to inhibition of CDK8. In a further aspect of the invention, this refers to inhibition of CDK9.

In a further aspect of the invention there is provided the use of a compound of the formula **(I),** or a pharmaceutically acceptable salt *or in vivo* hydrolysable ester thereof, as defined hereinbefore in the manufacture of a medicament for use in the production of an anti-cell-proliferation effect.

In a further aspect of the invention there is provided the use of a compound of the formula (I), or a pharmaceutically acceptable salt *or in vivo* hydrolysable ester thereof, as defined hereinbefore in the manufacture of a medicament for use in the production of a CDK2 inhibitory effect.

In a further aspect of the invention there is provided the use of a compound of the formula **(I),** or a pharmaceutically acceptable salt *or in vivo* hydrolysable ester thereof, as defined hereinbefore in the manufacture of a medicament for use in the treatment of cancer.

In a further aspect of the invention there is provided the use of a compound of the formula **(I)**, or a pharmaceutically acceptable salt *or in vivo* hydrolysable ester thereof, as defined hereinbefore in the manufacture of a medicament for use in the treatment of leukaemia or lymphoid malignancies or cancer of the breast, lung, colon, rectum, stomach, liver, kidney, prostate, bladder, pancreas, vulva, skin or ovary.

According to a further feature of the invention, there is provided the use of a compound of the formula **(I),** or a pharmaceutically acceptable salt *or in vivo* hydrolysable ester thereof, as defined herein before in the manufacture of a medicament for use in the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

In a further aspect of the invention there is provided a method of producing a cell cycle inhibitory effect, in a warm-blooded animal in need of such treatment, which comprises administering to said animal an effective amount of a compound of formula (I) or a pharmaceutically acceptable salt *or in vivo* hydrolysable ester thereof, as defined herein before.

In a further aspect of the invention there is provided a method of producing an anti-cell-proliferation effect, in a warm-blooded animal in need of such treatment, which comprises administering to said animal an effective amount of a compound of formula (I) or a pharmaceutically acceptable salt *or in vivo* hydrolysable ester thereof, as defined herein before.

In a further aspect of the invention there is provided a method of producing a CDK2 inhibitory effect, in a warm-blooded animal in need of such treatment, which comprises administering to said animal an effective amount of a compound of formula (I) or a pharmaceutically acceptable salt *or in vivo* hydrolysable ester thereof, as defined herein before.

In a further aspect of the invention there is provided a method of treating cancer, in a warm-blooded animal in need of such treatment, which comprises administering to said animal an effective amount of a compound of formula (I) or a pharmaceutically acceptable salt *or in* vivo hydrolysable ester thereof, as defined herein before.

In a further aspect of the invention there is provided a method of treating leukaemia or lymphoid malignancies or cancer of the breast, lung, colon, rectum, stomach, liver, kidney, prostate, bladder, pancreas, vulva, skin or ovary, in a warm-blooded animal in need of such treatment, which comprises administering to said animal an effective amount of a compound of formula **(I)** or a pharmaceutically acceptable salt *or in vivo* hydrolysable ester thereof, as defined herein before.

In a further aspect of the invention there is provided a method of treating cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation, in a warm-blooded animal in need of such treatment, which comprises administering to said animal an effective amount of a compound of formula **(I)** or a pharmaceutically acceptable salt *or in vivo* hydrolysable ester thereof, as defined herein before.

In a further aspect of the invention there is provided a pharmaceutical composition which comprises a compound of the formula **(I)**, or a pharmaceutically acceptable salt *or in* vivo hydrolysable ester thereof, as defined herein before and a pharmaceutically-acceptable diluent or carrier.

In a further aspect of the invention there is provided a pharmaceutical composition which comprises a compound of the formula **(I),** or a pharmaceutically acceptable salt *or in vivo* hydrolysable ester thereof, as defined herein before and a pharmaceutically-acceptable diluent or carrier for use as a medicament.

In a further aspect of the invention there is provided a pharmaceutical composition which comprises a compound of the formula **(I),** or a pharmaceutically acceptable salt *or in* vivo hydrolysable ester thereof, as defined herein before and a pharmaceutically-acceptable diluent or carrier for use in the production of a cell cycle inhibitory effect.

In a further aspect of the invention there is provided a pharmaceutical composition which comprises a compound of the formula **(I),** or a pharmaceutically acceptable salt *or in* vivo hydrolysable ester thereof, as defined herein before and a pharmaceutically-acceptable diluent or carrier for use in the production of an anti-cell-proliferation effect.

In a further aspect of the invention there is provided a pharmaceutical composition which comprises a compound of the formula **(I)**, or a pharmaceutically acceptable salt *or in* vivo hydrolysable ester thereof, as defined herein before and a pharmaceutically-acceptable diluent or carrier for use in the production of a CDK2 inhibitory effect.

In a further aspect of the invention there is provided a pharmaceutical composition which comprises a compound of the formula **(I)**, or a pharmaceutically acceptable salt *or in* vivo hydrolysable ester thereof, as defined herein before and a pharmaceutically-acceptable diluent or carrier for use in the treatment of cancer.

In a further aspect of the invention there is provided a pharmaceutical composition which comprises a compound of the formula **(I)**, or a pharmaceutically acceptable salt *or in* vivo hydrolysable ester thereof, as defined herein before and a pharmaceutically-acceptable diluent or carrier for use in the treatment of leukaemia or lymphoid malignancies or cancer of the breast, lung, colon, rectum, stomach, liver, kidney, prostate, bladder, pancreas, vulva, skin or ovary.

In a further aspect of the invention there is provided a pharmaceutical composition which comprises a compound of the formula **(I)**, or a pharmaceutically acceptable salt *or in vivo* hydrolysable ester thereof, as defined herein before and a pharmaceutically-acceptable diluent or carrier for use in the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

In a further aspect of the invention there is provided the use of a compound of the formula (I), or a pharmaceutically acceptable salt *or in vivo* hydrolysable ester thereof, as defined hereinbefore, in the production of a cell cycle inhibitory effect.

In a further aspect of the invention there is provided the use of a compound of the formula (I), or a pharmaceutically acceptable salt *or in vivo* hydrolysable ester thereof, as defined hereinbefore, in the production of an anti-cell-proliferation effect.

In a further aspect of the invention there is provided the use of a compound of the formula (I), or a pharmaceutically acceptable salt *or in vivo* hydrolysable ester thereof, as defined hereinbefore, in the production of a CDK2 inhibitory effect.

In a further aspect of the invention there is provided the use of a compound of the formula (I), or a pharmaceutically acceptable salt *or in vivo* hydrolysable ester thereof, as defined hereinbefore, in the treatment of cancer.

In a further aspect of the invention there is provided the use of a compound of the formula (I), or a pharmaceutically acceptable salt *or in vivo* hydrolysable ester thereof, as defined hereinbefore in the treatment of leukaemia or lymphoid malignancies or cancer of the breast, lung, colon, rectum, stomach, liver, kidney, prostate, bladder, pancreas, vulva, skin or ovary.

According to a further feature of the invention, there is provided the use of a compound of the formula **(I),** or a pharmaceutically acceptable salt *or in vivo* hydrolysable ester thereof, as defined herein before in the treatment of cancer, fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

Preventing cells from entering DNA synthesis by inhibition of essential S-phase initiating activities such as CDK2 initiation may also be useful in protecting normal cells of the body from toxicity of cycle-specific pharmaceutical agents. Inhibition of CDK2 or 4 will prevent progression into the cell cycle in normal cells which could limit the toxicity of cycle-specific pharmaceutical agents which act in S-phase, G2 or mitosis. Such protection may result in the prevention of hair loss normally associated with these agents.

Therefore in a further aspect of the invention there is provided a compound of formula **(I)** as defined above or a pharmaceutically acceptable salt *or in vivo* hydrolysable ester thereof for use as a cell protective agent.

Therefore in a further aspect of the invention there is provided a compound of formula **(I)** as defined above or a pharmaceutically acceptable salt *or in vivo* hydrolysable ester thereof for use in preventing hair loss arising from the treatment of malignant conditions with pharmaceutical agents.

Examples of pharmaceutical agents for treating malignant conditions that are known to cause hair loss include alkylating agents such as ifosfamide and cyclophosphamide; antimetabolites such as methotrexate, 5-fluorouracil, gemcitabine and cytarabine; vinca alkaloids and analogues such as vincristine, vinbalstine, vindesine, vinorelbine; taxanes such as paclitaxel and docetaxel; topoisomerase I inhibitors such as irintotecan and topotecan; cytotoxic antibiotics such as doxorubicin, daunorubicin, mitoxantrone, actinomycin-D and mitomycin; and others such as etoposide and tretinoin.

In another aspect of the invention, the compound of formula **(I),** or a pharmaceutically acceptable salt *or in vivo* hydrolysable ester thereof, may be administered in association with a one or more of the above pharmaceutical agents. In this instance the compound of formula (I) may be administered by systemic or non systemic means. Particularly the compound of formula **(I)** my may administered by non-systemic means, for example topical administration.

Therefore in an additional feature of the invention, there is provided a method of preventing hair loss during treatment for one or more malignant conditions with pharmaceutical agents, in a warm-blooded animal, such as man, which comprises administering to said animal an effective amount of a compound of formula **(I)**, or a pharmaceutically acceptable salt *or in vivo* hydrolysable ester thereof.

In an additional feature of the invention, there is provided a method of preventing hair loss during treatment for one or more malignant conditions with pharmaceutical agents, in a warm-blooded animal, such as man, which comprises administering to said animal an effective amount of a compound of formula **(I),** or a pharmaceutically acceptable salt *or in vivo* hydrolysable ester thereof in simultaneous, sequential or separate administration with an effective amount of said pharmaceutical agent.

According to a further aspect of the invention there is provided a pharmaceutical composition for use in preventing hair loss arising from the treatment of malignant conditions with pharmaceutical agents which comprises a compound of formula **(I),** or a pharmaceutically acceptable salt *or in vivo* hydrolysable ester thereof, and said pharmaceutical agent, in association with a pharmaceutically acceptable diluent or carrier.

According to a further aspect of the present invention there is provided a kit comprising a compound of formula **(I)**, or a pharmaceutically acceptable salt *or in vivo* hydrolysable ester thereof, and a pharmaceutical agent for treating malignant conditions that is known to cause hair loss.

According to a further aspect of the present invention there is provided a kit comprising:
a) a compound of formula **(I),** or a pharmaceutically acceptable salt *or in vivo* hydrolysable ester thereof, in a first unit dosage form;
b) a pharmaceutical agent for treating malignant conditions that is known to cause hair loss; in a second unit dosage form; and
c) container means for containing said first and second dosage forms.

According to another feature of the invention there is provided the use of a compound of the formula (**I),** or a pharmaceutically acceptable salt *or in vivo* hydrolysable ester thereof, in the manufacture of a medicament for the prevention of hair loss during treatment of malignant conditions with pharmaceutical agents.

According to a further aspect of the present invention there is provided a combination treatment for the prevention of hair loss comprising the administration of an effective amount of a compound of the formula (I), or a pharmaceutically acceptable salt *or in vivo* hydrolysable ester thereof, optionally together with a pharmaceutically acceptable diluent or carrier, with the simultaneous, sequential or separate administration of an effective amount of a pharmaceutical agent for treatment of malignant conditions to a warm-blooded animal, such as man.

As stated above the size of the dose required for the therapeutic or prophylactic treatment of a particular cell-proliferation disease will necessarily be varied depending on the host treated, the route of administration and the severity of the illness being treated. A unit dose in the range, for example, 1-100 mg/kg, preferably 1-50 mg/kg is envisaged.

The CDK inhibitory activity defined hereinbefore may be applied as a sole therapy or may involve, in addition to a compound of the invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the cell cycle inhibitory treatment defined hereinbefore may be: surgery, radiotherapy or chemotherapy. Such chemotherapy may cover three main categories of therapeutic agent:
(i) other cell cycle inhibitory agents that work by the same or different mechanisms from those defined hereinbefore;
(ii) cytostatic agents such as antioestrogens (for example tamoxifen,toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone 5α-dihydroreductase (for example finasteride), anti-invasion agents (for example metalloproteinase inhibitors like marimastat and inhibitors of urokinase plasminogen activator receptor function) and inhibitors of growth factor function, (such growth factors include for example platelet derived growth factor and hepatocyte growth factor such inhibitors include growth factor antibodies, growth factor receptor antibodies, tyrosine kinase inhibitors and serine/threonine kinase inhibitors); and
(iii) antiproliferative/antineoplastic drugs and combinations thereof, as used in medical oncology, such as antimetabolites (for example antifolates like methotrexate, fluoropyrimidines like 5-fluorouracil, purine and adenosine analogues, cytosine arabinoside); antitumour antibiotics (for example anthracyclines like doxorubicin, daunomycin, epirubicin and idarubicin, mitomycin-C, dactinomycin, mithramycin); platinum derivatives (for example cisplatin, carboplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincristine and taxoids like taxol, taxotere); topoisomerase inhibitors (for example epipodophyllotoxins like etoposide and teniposide, amsacrine, topotecan). According to this aspect of the invention there is provided a pharmaceutical product comprising a compound of the formula (I) as defined hereinbefore and an additional anti-tumour substance as defined hereinbefore for the conjoint treatment of cancer.

In addition to their use in therapeutic medicine, the compounds of formula (I) and their pharmaceutically acceptable salts are also useful as pharmacological tools in the development and standardisation of in vitro *and in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

### Examples

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulphate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example; and
(xvi) the following abbreviations have been used:
   - THF: tetrahydrofuran;
   - DMF: *N,N*-dimethylformamide;
   - EtOAc: ethyl acetate;
   - MeOH: methanol;
   - ether: diethyl ether;
   - EtOH: ethanol;
   - DCM: dichloromethane;
   - DMSO: dimethylsulphoxide;
   - Pd₂(dba)₃: bis(dibenzylideneacetone) palladium;
   - BINAP: 2,2'-bis(diphenylphosphino)- 1, I'-binaphthyl;
   - TEA: triethylamine;
   - EDTA: ethylenediaminetetraacetic acid;
   - HBTU: *O*-benzotriazol-1-yl-*N*,*N,N',N'*-tetramethyluronium hexafluorophosphate;
   - DIPEA: *N,N-*diisopropylethylamine;
   - DMFDMA: *N,N* dimethylformamide dimethyl acetal;
   - HPLC: high performance liquid chromatography;
   - MPLC: medium pressure liquid chromatography;
   - RPHPLC: reverse phase high performance liquid chromatography; and
   - Xantphos: 9,9-dimethyl-4,5-bis(diphenylphosphino)xanthene;
(xvii) where an SCX-2 column is referred to, this means an "ion exchange" extraction cartridge for adsorption of basic compounds, i.e. a polypropylene tube containing a benzenesulphonic acid based strong cation exchange sorbent, used according to the manufacturers instructions obtained from International Sorbent Technologies Limited, Dyffryn Business Park, Hengeod, Mid Glamorgan, UK, CF82 7RJ.

### Example 1

### 5-Fluoro-4-(1-isopropyl-2-methyl-1H-imidazol-5-yl)-N-[4-(morpholin-4-ylcarbonyl)phenyl]pyrimidin-2-amine

5-Fluoro-4-(3-isopropyl-2-methyl-3H imidazol-4-yl)-pyrimidin-2-ylamine (Method 2, 0.20g, 0.85 mmol), palladium acetate (8 mg, 0.034 mmol), Xantphos (30 mg, 0.051 mmol), caesium carbonate (0.42 g, 1.3 mmol) and (4-iodo-phenyl)-morpholin-4-yl-methanone (Method 16a in WO 05/044814; 290 mg, 0.90 mmol) were added to dioxane (7 ml) under an inert atmosphere and heated at reflux for 6 hours. Purification on silica using 0-10 % MeOH in DCM as eluent gave the title compound as a yellow solid. Further purification was achieved using RPHPLC to give a colourless foam (292 mg, 81%). NMR (400.132 MHz): 9.78 (s, 1H), 8.59 (d, 1H), 7.73 (d, 2H), 7.38 - 7.36 (m, 3H), 5.43 (septet, 1H), 3.64 - 3.56 (m, 4H), 3.54 - 3.46 (m, 4H), 2.53 (s, 3H), 1.45 (d, 6H); m/z 425.

### Examples 2-115

The following compounds were prepared by the procedure of Example 1 using the appropriate starting materials.

| **Ex** | **Compound** | **NMR** | **m/z** | **SM** |
|---|---|---|---|---|
| **2** | 5-Fluoro-4-(1-isopropyl-2-methyl-1H imidazol-5-yl)-N {3-methyl-4-[(4-methyl piperazin-1-yl) carbonyl]phenyl} pyrimidin-2-amine | (400.132 MHz) 9.58 (s, 1H), 8.56 (s, 1H), 7.58 (d, 1H), 7.45 (s, 1H), 7.37 (d, 1H), 7.07 (d, 1H), 5.41 (septet, 1H), 3.66 -3.60 (m, 2H), 3.19 -3.13 (m, 2H), 2.52 (s, 3H), 2.38 - 2.32 (m, 2H), 2.24 - 2.17 (m, 8H), 1.43 (d, 6H) | 452 | Method 4 and Method 2 |
| **3** | 5-Fluoro-4-(1- isopropyl-2-methyl-1H imidazol-5-yl)-N[3-methyl-4-(morpholin-4-ylcarbonyl)phenyl] pyrimidin-2-amine | (400.132 MHz) 9.59 (s, 1H), 8.57 (d, 1H), 7.58 (d, 1H), 7.46 (s, 1H), 7.37 (d, 1H), 7.11 (d, 1H), 5.40 (septet, 1H), 3.69 - 3.57 (m, 4H), 3.54 - 3.47 (m, 2H), 3.21 - 3.14 (m, 2H), 2.52 (s, 3H), 2.20 (s, 3H), 1.43 (d, 6H) | 439 | Method 9 and Method 2 |
| **4** | 5-Fluoro-N-{3-fluoro-4-[(4-methylpiperazin- 1-yl)carbonyl]phenyl}- 4-(1-isopropyl-2- methyl-1H-imidazol-5-yl)pyrimidin-2-amine | (400.132 MHz) 9.96 (s, 1H), 8.63 (d, 1H), 7.73 (d, 1H), 7.47 (d, 1H), 7.39 (d, 1H), 7.30 (t, 1H), 5.41 (septet, 1H), 3.67 - 3.57 (m, 2H), 3.30 - 3.20 (m, 2H), 2.54 (s, 3H), 2.38 - 2.32 (m, 2H), 2.30 - 2.23 (m, 2H), 2.20 (s, 3H), 1.46 (d, 6H) | 456 | Method 5 and Method 2 |
| **5** | *N* [3-Chloro-4-(morpholin-4-ylcarbonyl)phenyl]-5-fluoro-4-(1-isopropyl- 2-methyl-1H-imidazol-5-yl)pyrimidin-2-amine | (400.132 MHz) 9.87 (s, 1H), 8.62 (d, 1H), 7.88 (s, 1H), 7.67 (d, 1H), 7.38 (d, 1H), 7.30 (d, 1H), 5.36 (septet, 1H), 3.67 - 3.62 (m, 4H), 3.57 - 3.52 (m, 2H), 3.19 - 3.15 (m, 2H), 2.53 (s, 3H), 1.46 (d, 6H) | 459 | Method 8 and Method 2 |
| **6** | N (4-{[3-(Dimethyl amino)pyrrolidin-1-yl]carbonyl}phenyl)-5-fluoro-4-(1-isopropyl-2-methyl-1*H*-imidazol-5-yl)pyrimidin-2-amine | 9.77 (s, 1H), 8.57 (d, 1H), 7.71 (d, 2H), 7.47 (d, 2H), 5.50-5.35 (m, 1H), 3.74-3.41 (br m, 3H), 2.77- 2.57 (m, 1H), 2.52 (s, 3H), 2.28- 1.91 (m, 8H), 1.79-1.71 (m, 1H), 1.44 (d, 6H) | 453 | Method 10 and Method 2 |
| **7** | 4-(1-Isopropyl-2- methyl-1H-imidazol-5-yl)-N-{4-[(4-methyl piperazin-1-yl) carbonyl]phenyl} pyrimidin-2-amine | (400.132 MHz) 9.68 (s,1H), 8.43 (d, 1H), 7.76 (d, 2H), 7.44 (s, 1H), 7.35 (d, 2H), 7.10 (d, 1H), 5.69 (quintet, 1H), 3.57 - 3.43 (m, 4H), 3.32 (s, 3H), 2.36 - 2.28 (m, 4H), 2.20 (s, 3H), 1.46 (d, 6H) | 420 | Example 59 of WO 03 004472 and Method 14 |
| **8** | 4-(1-Isopropyl-2-methyl-1H-imidazol-5-yl)-N-{3-methyl-4-[(4-methylpiperazin-1-yl) carbonyl]phenyl} pyrimidin-2-amine | (400.132 MHz) 9.48 (s, 1H), 8.41 (d, 1H), 7.63 (d, 1H), 7.48 (s, 1H), 7.43 (s, 1H), 7.08 (d, 1H), 7.06 (d, 1H), 5.66 (septet, 1H), 3.69 - 3.59 (m, 2H), 3.22 - 3.12 (m, 2H), 2.51 (s, 3H), 2.40 - 2.29 (m, 2H), 2.26 - 2.19 (m, 8H), 1.44 (d, 6H) | 434 | Method 4 and Method 14 |
| **9** | 4-(1-Isopropyl-2- methyl-1*H*-imidazol-5-yl)-N-[3-methyl-4-(morpholin-4-ylcarbonyl)phenyl] pyrimidin-2-amine | (400.132 MHz) 9.49 (s, 1H), 8.41 (d, 1H), 7.65 (d, 1H), 7.49 (s, 1H), 7.43 (s, 1H), 7.11 (d, 1H), 7.07 (d, 1H), 5.65 (septet, 1H), 3.72 - 3.58 (m, 4H), 3.55 - 3.46 (m, 2H), 3.28 - 3.12 (m, 2H), 2.50 (s, 3H), 2.21 (s, 3H), 1.45 (d, 6H) | 421 | Method 9 and Method 14 |
| **10** | N-{3-Fluoro-4-[(4-methylpiperazin-1-yl)carbonyl]phenyl}-4-(1-isopropyl-2-methyl- 1*H*-imidazol-5-yl)pyrimidin-2-amine | (400.132 MHz) 9.86 (s,1H), 8.47 (d, 1H), 7.80 (d, 1H), 7.50 (d, 1H), 7.45 (s, 1H), 7.30 (t, 1H), 7.14 (d, 1H), 5.66 (septet, 1H), 3.67 - 3.58 (m, 2H), 3.30 - 3.22 (m, 2H), 2.51 (s, 3H), 2.37 - 2.31 (m, 2H), 2.30 - 2.23 (m, 2H), 2.19 (s, 3H), 1.47 (d, 6H) | 438 | Method 5 and Method 14 |
| **11** | N-[3-Fluoro-4-(morpholin-4-ylcarbonyl)phenyl]-4-(1-isopropyl-2-methyl-1*H*-imidazol-5-yl)pyrimidin-2-amine | (400.132 MHz) 9.88 (s, 1H), 8.47 (d, 1H), 7.81 (d, 1H), 7.52 (d, 1H), 7.45 (s, 1H), 7.34 (t, 1H), 7.15 (d, 1H), 5.66 (septet, 1H), 3.68 - 3.60 (m, 4H), 3.59 - 3.52 (m, 2H), 3.32 - 3.26 (m, 2H), 2.51 (s, 3H), 1.48 (d, 6H) | 425 | Method 6 and Method 14 |
| | *N*-{3-Chloro-4-[(4-methylpiperazin-1- yl)carbonyl]phenyl}-4-(1-isopropyl-2-methyl-1H imidazol-5-yl)pyrimidin-2-amine | (400.132 MHz) 9.76 (s, 1H), 8.46 (d, 1H), 7.92 (s, 1H), 7.70 (d, 1H), 7.45 (s, 1H), 7.27 (d, 1H), 7.13 (d, 1H), 5.61 (septet, 1H), 3.67 - 3.59 (m, 2H), 3.20 - 3.13 (m, 2H), 2.50 (s, 3H), 2.39 - 2.33 (m, 2H), 2.30 - 2.23 (m, 2H), 2.19 (s, 3H), 1.47 (d, 6H) | 454 | Method 7 and Method 14 |
| **13** | *N*-[3-Chloro-4-(morpholin-4- ylcarbonyl)phenyl]-4-(1-isopropyl-2-methyl-1H imidazol-5-yl)pyrimidin-2-amine | (400.132 MHz) 9.77 (s, 1H), 8.47 (d, 1H), 7.93 (s, 1H), 7.72 (d, 1H), 7.45 (s, 1H), 7.30 (d, 1H), 7.13 (d, 1H), 5.61 (septet, 1H), 3.68 - 3.61 (m, 4H), 3.58 - 3.54 (m, 2H), 3.20 - 3.16 (m, 2H), 2.51 (s, 3H), 1.47 (d, 6H) | 441 | Method 8 and Method 14 |
| **14** | 4-(1-Isopropyl-2- methyl-1H imidazol-5- yl)-N-[4-(morpholin-4- ylcarbonyl)phenyl] pyrimidin-2-amine | (400.132 MHz) 9.70 (s, 1H), 8.43 (d, 1H), 7.78 (d, 2H), 7.44 (s, 1H), 7.38 (d, 2H), 7.10 (d, 1H), 5.69 (quintet, 1H), 3.63 - 3.60 (m, 4H), 3.55 - 3.48 (m, 4H), 2.50 (s, 3H), 1.46 (d, 6H) | 407 | Method 16a in WO 05 / 044814 and Method 14 |
| **15** | *N-(4-{[3-* (Dimethylamino) pyrrolidin-1- yl]carbonyl}phenyl)-4-(1-isopropyl-2-methyl-1H imidazol-5-yl)pyrimidin-2-amine | (400.13 MHz) 9.74 (s, 1H), 8.44 (d, 1H), 7.76 (d, 2H), 7.50 (app t, 2H), 7.45 (s, 1H), 7.11 (d, 1H), 5.77- 5.64 (m, 1H), 7.73-3.39 (m, 7H overlap with water), 2.75-2.58 (m, 1H), 2.23-1.95 (m, 7H), 1.80-1.63 (m, 1H), 1.47 (d, 6H) | 434 | Method 10 and Method 14 |
| **16** | 4-(1-Isopropyl-2- methyl-1H-imidazol-5-yl)-N-{3-methoxy-4-[(4-methylpiperazin-1- yl)carbonyl]phenyl} pyrimidin-2-amine | (400.132 MHz) 9.52 (s, 1H), 8.43 (d, 1H), 7.51 (d, 1H), 7.45 (s, 1H), 7.35 (s, 1H), 7.11 - 7.07 (m, 2H), 5.65 (septet, 1H), 3.78 (s, 3H), 3.68 - 3.52 (m, 2H), 3.21 - 3.12 (m, 2H), 2.50 (s, 3H), 2.38 - 2.24 (m, 4H), 2.19(s,3H), 1.46(d,6H) | 450 | Method 14 and Method 19 |
| **17** | 4-(1-Isopropyl-2- methyl-1H-imidazol-5-yl)-N-[3-methoxy-4-(morpholin-4- ylcarbonyl)phenyl] pyrimidin-2-amine | (400.132 MHz, CDC1₃): 8.38 (d, 1H), 7.40 - 7.37 (m, 2H), 7.30 - 7.21 (m, 3H), 6.94 (d, 1H), 5.56 (septet, 1H), 3.85 (s, 3H), 3.83 - 3.71 (m, 4H), 3.67 - 3.56 (m, 2H), 3.38 - 3.26 (m, 2H), 2.59 (s, 3H), 1.53 (d, 6H) | 437 | Method 14 and Method 22 |
| **18** | 5-Fluoro-4-(1- isopropyl-2-methyl-1H-imidazol-5-yl)-N-[3-methoxy-4-(morpholin-4- ylcarbonyl)phenyl] pyrimidin-2-amine | (400.132 MHz, CDC1₃) 8.31 (d, 1H), 7.59 (d, 1H), 7.32 (s, 1H), 7.25 - 7.19 (m, 3H), 5.49 (septet, 1H), 3.84 (s, 3H), 3.81 - 3.72 (m, 4H), 3.67 - 3.55 (m, 2H), 3.34 - 3.29 (m, 2H), 2.61 (s, 3H), 1.53 (d, 6H) | 455 | Method 2 and Method 20 |
| **19** | 4-[1-Isopropyl-2-(methoxymethyl)-I*H*- imidazol-5-yl]-N-[4-(morpholin-4- ylcarbonyl)phenyl] pyrimidin-2-amine | (400.132 MHz, CDC1₃) 8.43 (d, 1H), 7.68 (d, 2H), 7.48 (s, 1H), 7.43 - 7.41 (m, 3H), 6.98 (d, 1H), 5.50 (septet, 1H), 4.66 (s, 2H), 3.79 - 3.59 (m, 8H), 3.41 (s, 3H), 1.57 (d, 6H) | 437 | Method 16a in WO 05 / 044814 and Method 3 |
| **20** | 4-[1-Isopropyl-2-(methoxymethyl)-IH- imidazol-5-yl]-N-[3- methyl-4-(morpholin- 4-ylcarbonyl)phenyl] pyrimidin-2-amine | (400.132 MHz, CDC1₃) 8.41 (d, 1H), 7.56 (d, 1H), 7.42 (s, 1H), 7.41 (s, 1H), 7.32 (s, 1H), 7.14 (d, 1H), 6.95 (d, 1H), 5.46 (septet, 1H), 4.65 (s, 2H), 3.86 - 3.80 (m, 2H), 3.80 - 3.75 (m, 2H), 3.61 - 3.56 (m, 2H), 3.41 (s, 3H), 3.34 - 3.27 (m, 2H), 2.33 (s, 3H), 1.56 (d, 6H) | 451 | Method 9 and Method 3 |
| **21** | N [3-Fluoro-4-(morpholin-4- ylcarbonyl)phenyl]-4-[1-isopropyl-2-(methoxymethyl)-IH- imidazol-5-yl] pyrimidin-2-amine | (400.132 MHz, CDC1₃) 8.44 (d, 1H), 7.81 (d, 1H), 7.44 - 7.43 (m, 2H), 7.38 (t, 1H), 7.22 (d, 1H), 7.02 (d, 1H), 5.49 (septet, 1H), 4.67 (s, 2H), 3.85 - 3.75 (m, 4H), 3.69 - 3.63 (m, 2H), 3.43 - 3.39 (m, 5H), 1.59 (d, 6H) | 455 | Method 6 and Method 3 |
| **22** | 4-[1-Isopropyl-2-(methoxymethyl)-IH- imidazol-5-yl]-N-{4-[(4-methylpiperazin-1- yl)carbonyl]phenyl} pyrimidin-2-amine | (400.132 MHz, CDC1₃) 8.42 (d, 1H), 7.66 (d, 2H), 7.62 (s, 1H), 7.43 - 7.40 (m, 3H), 6.97 (d, 1H), 5.51 (septet, 1H), 4.66 (s, 2H), 3.94 - 3.48 (m, 4H), 3.41 (s, 3H), 2.51 - 2.37 (m, 4H), 2.33 (s, 3H), 1.57 (d, 6H) | 450 | Example 59 of WO 03 / 004472 and Method 3 |
| **23** | 4-[1-Isopropyl-2-(methoxymethyl)-IH- imidazol-5-yl]-*N*-{3- methyl-4-[(4-methyl piperazin-1-yl) carbonyl]phenyl} pyrimidin-2-amine | (400.132 MHz, CDC1₃) 8.41 (d, 1H), 7.55 (d, 1H), 7.42 (s, 1H), 7.39 - 7.37 (m, 2H), 7.14 (d, 1H), 6.95 (d, 1H), 5.47 (septet, 1H), 4.65 (s, 2H), 3.89 - 3.79 (m, 2H), 3.41 (s, 3H), 3.36 - 3.27 (m, 2H), 2.52 - 2.45 (m, 2H), 2.36 - 2.26 (m, 8H), 1.56 (d, 6H) | 464 | Method 4 and Method 3 |
| **24** | N-{3-Fluoro-4-[(4-methylpiperazin-1- yl)carbonyl]phenyl}-4-[1-isopropyl-2-(methoxymethyl)-IH- imidazol-5-yl]pyrimidin-2-amine | (400.132 MHz, CDC1₃) 8.44 (d, 1H), 7.78 (d, 1H), 7.66 (s, 1H), 7.44 (s, 1H), 7.35 (t, 1H), 7.21 (d, 1H), 7.01 (d, 1H), 5 .51 (septet, 1H), 4.66 (s, 2H), 3.87 - 3.78 (m, 2H), 3.48 - 3.35 (m, 5H), 2.52 - 2.44 (m, 2H), 2.41 - 2.35 (m, 2H), 2.33 (s, 3H), 1.58 (d, 6H) | 468 | Method 5 and Method 3 |
| **25** | 4-(1-Cyclobutyl-2- methyl-1H imidazol-5-yl)-N-[4-(morpholin-4- ylcarbonyl)phenyl] pyrimidin-2-amine | (400.132 MHz, CDC1₃) 8.39 (d, 1H), 7.71 (d, 2H), 7.58 (s, 1H), 7.44 (d, 2H), 7.32 (s, 1H), 6.93 (d, 1H), 5.42 (quintet, 1H), 3.81 - 3.54 (m, 8H), 2.60 (s, 3H), 2.50 - 2.43 (m, 4H), 1.85 - 1.66 (m, 2H) | 419 | Method 16a in WO 05 / 044814 and Method 51 in WO 03 / 076435 |
| **26** | 4-(1-Cyclobutyl-2- methyl-1H imidazol-5-yl)-N-[3-methyl-4-(morpholin-4- ylcarbonyl)phenyl] pyrimidin-2-amine | (400.132 MHz, CDCl₃) 8.38 (d, 1H), 7.62 (d, 1H), 7.42 (s, 1H), 7.32 - 7.31 (m, 2H), 7.16 (d, 1H), 6.90 (d, 1H), 5.37 (quintet, 1H), 3.86 - 3.74 (m, 4H), 3.62 - 3.54 (m, 2H), 3.34 - 3.27 (m, 2H), 2.59 (s, 3H), 2.49 - 2.42 (m, 4H), 2.34 (s, 3H), 1.84 - 1.69 (m, 2H) | 433 | Method 9 and Method 51 in WO 03 / 076435 |
| **27** | 4-(1-Cyclobutyl-2- methyl-IH-imidazol-5-yl)-N-{4-[(4-methylpiperazin-1- yl)carbonyl]phenyl} pyrimidin-2-amine | (400.132 MHz, CDC1₃) 8.39 (d, 1H), 7.69 (d, 2H), 7.47 (s, 1H), 7.43 (d, 2H), 7.32 (s, 1H), 6.92 (d, 1H), 5.43 (quintet, 1H), 3.89 - 3.47 (m, 8H), 2.60 (s, 3H), 2.50 - 2.43 (m, 4H), 2.33 (s, 3H), 1.85 - 1.66 (m, 2H) | 432 | Example 59 of WO 03 / 004472 and Method 51 in WO 03 / 076435 |
| **28** | 4-(1-Cyclobutyl-2- methyl-1H imidazol-5-yl)-N {3-methyl-4-[(4-methylpiperazin-1- yl)carbonyl]phenyl} pyrimidin-2-amine | (400.132 MHz, CDC1₃) 8.38 (d, 1H), 7.60 (d, 1H), 7.41 (s, 1H), 7.36 (s, 1H), 7.31 (s, 1H), 7.15 (d, 1H), 6.90 (d, 1H), 5.38 (quintet, 1H), 3.87 - 3.81 (m, 2H), 3.34 - 3.28 (m, 2H), 2.59 (s, 3H), 2.52 - 2.42 (m, 6H), 2.33 - 2.26 (m, 8H), 1.84 - 1.66 (m, 2H) | 446 | Method 4 and Method 51 in WO 03 / 076435 |
| **29** | 4-(1-Cyclobutyl-2- methyl-1H imidazol-5-yl)-N-{3-fluoro-4-[(4-methylpiperazin-1- yl)carbonyl]phenyl} pyrimidin-2-amine | (400.132 MHz, CDC1₃) 8.40 (d, 1H), 7.84 (d, 1H), 7.75 (s, 1H), 7.36 (t, 1H), 7.32 (s, 1H), 7.22 (d, 1H), 6.95 (d, 1H), 5.41 (quintet, 1H), 3.86 - 3.79 (m, 2H), 3.47 - 3.38 (m, 2H), 2.60 (s, 3H), 2.53 - 2.42 (m, 6H), 2.41 - 2.35 (m, 2H), 2.33 (s, 3H), 1.86 - 1.68 (m, 2H) | 450 | Method 5 and Method 51 in WO 03 / 076435 |
| **30** | 4-(1-Ethyl-2-methyl-1H imidazol-5-yl)-N [4-(morpholin-4- ylcarbonyl)phenyl] pyrimidin-2-amine | (400.132 MHz, CDC1₃) 8.36 (d, 1H), 7.65 (d, 2H), 7.55 (s, 1H), 7.43 (d, 2H), 7.37 (s, 1H), 6.99 (d, 1H), 4.51 (q, 2H), 3.79 - 3.58 (m, 8H), 2.48 (s, 3H), 1.30 (t, 3H) | 393 | Method 16a in WO 05 / 044814 and Method 30 in WO 02 / 020512 |
| **31** | 4-(1-Ethyl-2-methyl- 1*H*-imidazol-5-yl)-N- {4-[(4-methylpiperazin-1- yl)carbonyl]phenyl} pyrimidin-2-amine | (400.132 MHz, CDC1₃) 8.35 (d, 1H), 7.63 (d, 2H), 7.55 (s, 1H), 7.45 (s, 1H), 7.42 (d, 2H), 6.98 (d, 1H), 4.50 (q, 2H), 3.92 - 3.41 (m, 4H), 2.54 - 2.38 (m, 7H), 2.33 (s, 3H), 1.29 (t, 3H) | 406 | Example 59 of WO 03 / 004472 and Method 30 in WO 02 / 020512 |
| **32** | 4-(1-Ethyl-2-methyl-1H imidazol-5-yl)-N {3-methyl-4-[(4-methylpiperazin-1- yl)carbonyl]phenyl} pyrimidin-2-amine | (400.132 MHz, CDC1₃) 8.34 (d, 1H), 7.54 (s, 1H), 7.47 (d, 1H), 7.43 (s, 1H), 7.40 (s, 1H), 7.14 (d, 1H), 6.96 (d, 1H), 4.50 (q, 2H), 3.87 - 3.80 (m, 2H), 3.35 - 3.28 (m, 2H), 2.52 - 2.45 (m, 5H), 2.35 - 2.27 (m, 8H), 1.27 (t, 3H) | 420 | Method 4 and Method 30 in WO 02 / 020512 |
| **33** | 4-(1-Ethyl-2-methyl- 1*H* imidazol-5-yl)-N {3-fluoro-4-[(4-methylpiperazin-1- yl)carbonyl]phenyl} pyrimidin-2-amine | (400.132 MHz, CDC1₃) 8.37 (d, 1H), 7.73 (d, 1H), 7.56 (s, 1H), 7.43 (s, 1H), 7.36 (t, 1H), 7.20 (d, 1H), 7.02 (d, 1H), 4.52 (q, 2H), 3.85 - 3.80 (m, 2H), 3.45 - 3.38 (m, 2H), 2.52 - 2.46 (m, 5H), 2.40 - 2.35 (m, 2H), 2.33 (s, 3H), 1.33 (t, 3H) | 424 | Method 5 and Method 30 in WO 02 / 020512 |
| **34** | 4-(1-Ethyl-2-methyl-1H imidazol-5-yl)-N {3-methoxy-4-[(4-methylpiperazin-1- yl)carbonyl]phenyl} pyrimidin-2-amine | (400.132 MHz, CDC1₃) 8.36 (d, 1H), 7.54 (s, 1H), 7.39 (s, 1H), 7.36 (s, 1H), 7.21 (d, 1H), 7.14 (d, 1H), 6.96 (d, 1H), 4.50 (q, 2H), 3.93 - 3.73 (m, 5H), 3.40 - 3.27 (m, 2H), 2.57 - 2.37 (m, 5H), 2.32 - 2.18(m,5H), 1.29 (t, 3H) | 436 | Method 19 and Method 30 in WO 02 / 020512 |
| **35** | 4-[1-(Cyclopropyl methyl)-2-methyl-1H- imidazol-5-yl]-N-[4-(morpholin-4- ylcarbonyl)phenyl] pyrimidin-2-amine | (400.132 MHz, CDC1₃): 8.18 (d, 1H), 7.46 (d, 2H), 7.36 (s, 1H), 7.28 (s, 1H), 7.25 (d, 2H), 6.82 (d, 1H), 4.25 (d, 2H), 3.60 - 3.40 (m, 8H), 2.30 (s, 3H), 0.99 - 0.89 (m, 1H), 0.28 - 0.23 (m, 2H), -0.01 (q, 2H) | 419 | Method 54 in WO 03 / 076435 and Method 16a in WO 05 / 044814 |
| **36** | 4-[1-(Cyclopropyl methyl)-2-methyl-1H- imidazol-5-yl]-N-{4-[(4-methylpiperazin-1- yl)carbonyl]phenyl} pyrimidin-2-amine | (400.132 MHz, CDC1₃) 8.19 (d, 1H), 7.45 (d, 2H), 7.37 (s, 1H), 7.34 (s, 1H), 7.25 (d, 2H), 6.82 (d, 1H), 4.25 (d, 2H), 3.78 - 3.27 (m, 4H), 2.30 (s, 3H), 2.28 - 2.21 (m, 4H), 2.15 (s, 3H), 0.99 - 0.89 (m, IH), 0.28 - 0.23 (m, 2H), -0.01 (q, 2H) | 432 | Method 54 in WO 03 / 076435 and Example 59 of WO 03 / 004472 |
| **37** | 4-[1-(1-Cyclopropyl ethyl)-2-methyl-1H- imidazol-5-yl]-N-{4-[(4-methylpiperazin-1- yl)carbonyl]phenyl} pyrimidin-2-amine | (400.132 MHz, CDC1₃): 8.36 (d, 1H), 7.61 (d, 2H), 7.43 - 7.41 (m, 3H), 7.35 (s, 1H), 6.96 (d, 1H), 4.84 - 4.70 (m, 1H), 3.98 - 3.42 (m, 4H), 2.64 (s, 3H), 2.51 - 2.36 (m, 4H), 2.33 (s, 3H), 1.62 (d, 3H), 1.44 - 1.33 (m, 1H), 0.69 - 0.59 (m, 1H), 0.44 - 0.37 (m, 1H), 0.22 - 0.10 (m, 2H) | 446 | Example 59 of WO 03/004472 and Method 32 in WO 02/020512 |
| **38** | {4-[4-(3- Cyclopropylmethyl-2- ethyl-3*H*-imidazol-4- yl)-pyrimidin-2- ylamino]-phenyl}-(4-methyl-piperazin-1- yl)-methanone | (400.132 MHz, CDC1₃) 8.20 (d, 1H), 7.46 (d, 2H), 7.28 - 7.24 (m, 2H), 6.84 (d, 1H), 4.27 (d, 2H), 3.74 - 3.29 (m, 4H), 2.60 (q, 2H), 2.36 - 2.21 (m, 4H), 2.17 (s, 3H), 1.24 (t, 3H), 0.99 - 0.88 (m, 1H), 0.28 - 0.23 (m, 2H), 0.02 - -0.03 (m, 2H) | 446 | Method 56 in WO03/07643 5 and Example 59 of WO03/004472 |
| **39** | {4-[4-(3-Ethyl-2- isopropyl-3H- imidazol-4-yl)- pyrimidin-2-ylamino]- phenyl}-(4-methyl- piperazin-1-yl)- methanone | (400.132 MHz, CDC1₃) 8.35 (d, 1H), 7.63 (d, 2H), 7.59 (d, 1H), 7.42 (d, 2H), 7.36 (s, 1H), 6.99 (d, 1H), 4.54 (q, 2H), 3.90 - 3.46 (m, 4H), 3.09 (septet, 1H), 2.52 - 2.37 (m, 4H), 2.33 (s, 3H), 1.38 (d, 6H), 1.30 (t, 3H) | 434 | Method 27 and Example 59 of WO03/004472 |
| **40** | {4-[4-(2-Cyclopropyl- 3-ethyl-3H-imidazol- 4-yl)-pyrimidin-2- ylamino]-phenyl}-(4-methyl-piperazin-1- yl)-methanone | (400.132 MHz, CDC1₃) 8.34 (d, 1H), 7.64 (d, 2H), 7.50 (s, 1H), 7.43 (d, 2H), 7.35 (s, 1H), 6.97 (d, 1H), 4.67 (q, 2H), 3.89 - 3.51 (m, 4H), 2.54 - 2.38 (m, 4H), 2.33 (s, 3H), 1.92 - 1.85 (m, 1H), 1.36 (t, 3H), 1.13 - 1.09 (m, 2H), 1.07 - 1.01 (m, 2H) | 432 | Method 32 and Example 59 of WO03/004472 |
| **41** | {4-[4-(3-Ethyl-2- trifluoromethyl-3H- imidazol-4-yl)- pyrimidin-2-ylamino]- phenyl}-(4-methyl- piperazin-1-yl)- methanone | (400.132 MHz, CDC1₃) 8.49 (d, 1H), 7.63 (d, 2H), 7.62 (s, 1H), 7.44 (d, 2H), 7.31 (s, 1H), 7.05 (d, 1H), 4.70 (q, 2H), 3.89 - 3.49 (m, 4H), 2.55 - 2.38 (m, 4H), 2.33 (s, 3H), 1.35 (t, 3H) | 460 | Method 37 and Example 59 of WO03/004472 |
| **42** | {4-[4-(2- Difluoromethyl-3- ethyl-3H-imidazol-4- yl)-pyrimidin-2- ylamino]-phenyl}-(4-methyl-piperazin-1- yl)-methanone | (400.132 MHz, CDC1₃) 8.46 (d, 1H), 7.64 (d, 2H), 7.59 (s, 1H), 7.44 (d, 2H), 7.39 (s, 1H), 7.03 (d, 1H), 6.80 (t, 1H), 4.74 (q, 2H), 3.89 - 3.49 (m, 4H), 2.50 - 2.38 (m, 4H), 2.33 (s, 3H), 1.36 (t, 3H) | 442 | Method 42 and Example 59 of WO03/004472 |
| **43** | {4-[4-(2-Cyclopropyl- 3-isopropyl-3H- imidazol-4-yl)- pyrimidin-2-ylamino]- phenyl}-(4-methyl- piperazin-1-yl)- methanone | (300.072 MHz, CDC1₃) 8.35 (d, 1H), 7.66 (d, 2H), 7.48 (s, 1H), 7.40 (d, 2H), 7.32 (s, 1H), 6.92 (d, 1H), 5.72 (septet, 1H), 3.83 - 3.47 (m, 4H), 2.49 - 2.38 (m, 4H), 2.32 (s, 3H), 2.07 - 1.98 (m, 1H), 1.63 (d, 6H), 1.21 - 1.16 (m, 2H), 1.08 - 1.01 (m, 2H) | 446 | Method 46 and Example 59 of WO03/004472 |
| **44** | ((S)-3-Dimethylamino- pyrrolidin-l-yl)-{4-[4-(3-ethyl-2-isopropyl- 3H-imidazol-4-yl)- pyrimidin-2-ylamino]- phenyl}-methanone | (400.132 MHz, CDC1₃) 8.35 (d, 1H), 7.63 (d, 2H), 7.59 (s, 1H), 7.54 (d, 2H), 7.19 (s, 1H), 6.99 (d, 1H), 4.54 (q, 2H), 3.98 - 3.77 (m, 1H), 3.74 - 3.35 (m, 3H), 3.08 (septet, 1H), 2.82 - 2.63 (m, 1H), 2.35 - 2.02 (m, 7H), 1.89 - 1.76 (m, 1H), 1.38(d,6H), 1.29(t,3H) | 448 | Method 27 and Method 47 |
| **45** | ((R)-3- Dimethylamino- pyrrolidin-l-yl)-{4-[4-(3-ethyl-2-isopropyl- 3*H*-imidazol-4-yl)- pyrimidin-2-ylamino]- phenyl}-methanone | (400.132 MHz, CDC1₃) 8.35 (d, 1H), 7.63 (d, 2H), 7.58 (s, 1H), 7.54 (d, 2H), 7.28 (s, 1H), 6.99 (d, 1H), 4.54 (q, 2H), 3.98 - 3.77 (m, 1H), 3.72 - 3.34 (m, 3H), 3.08 (septet, 1H), 2.81 - 2.63 (m, 1H), 2.33 - 2.03 (m, 7H), 1.84 (quintet, 1H), 1.38 (d, 6H), 1.29 (t, 3H) | 448 | Method 27 and Method 48 |
| **46** | {4-[4-(2-Cyclopropyl- 3-ethyl-3H-imidazol- 4-yl)-pyrimidin-2- ylamino]-phenyl}-((S)- 3-dimethylamino- pyrrolidin-1-yl)- methanone | (400.132 MHz, CDC1₃) 8.34 (d, 1H), 7.63 (d, 2H), 7.55 (d, 2H), 7.49 (s, 1H), 7.26 (s, 1H), 6.96 (d, 1H), 4.67 (q, 2H), 3.98 - 3.34 (m, 4H), 2.83 - 2.61 (m, 1H), 2.33 - 2.03 (m, 7H), 1.93 - 1.79 (m, 2H), 1.36 (t, 3H), 1.12 - 1.08 (m, 2H), 1.07 - 1.00 (m, 2H) | 446 | Method 32 and Method 47 |
| **47** | {4-[4-(2-Cyclopropyl- 3-ethyl-3H-imidazol- 4-yl)-pyrimidin-2- ylamino]-phenyl}-((R)-3-dimethylamino- pyrrolidin-1-y)- methanone | (400.132 MHz, CDC1₃) 8.34 (d, 1H), 7.63 (d, 2H), 7.54 (d, 2H), 7.49 (s, 1H), 7.42 (s, 1H), 6.96 (d, 1H), 4.66 (q, 2H), 3.98 - 3.32 (m, 4H), 2.81 - 2.64 (m, 1H), 2.34 - 2.06 (m, 7H), 1.90 - 1.78 (m, 2H), 1.35 (t, 3H), 1.12 - 1.08 (m, 2H), 1.06 - 1.00 (m, 2H) | 446 | Method 32 and Method 48 |
| **48** | ((S)-3-Dimethylamino- pyrrolidin-l-yl)-{4-[4-(3-ethyl-2- trifluoromethyl-3H- imidazol-4-yl)- pyrimidin-2-ylamino]- phenyl}-methanone | (400.132 MHz, CDC1₃) 8.48 (d, 1H), 7.63 - 7.61 (m, 3H), 7.55 (d, 2H), 7.35 (s, 1H), 7.04 (d, 1H), 4.69 (q, 2H), 3.96 - 3.78 (m, 1H), 3.71 - 3.35 (m, 3H), 2.83 - 2.61 (m, 1H), 2.30 (s, 3H), 2.22 (s, 3H), 2.20 - 2.04 (m, 1H), 1.90 - 1.79 (m, 1H), 1.34 (t, 3H) | 474 | Method 37 and Method 47 |
| **49** | ((R)-3- Dimethylamino- pyrrolidin-l-yl)-{4-[4-(3-ethyl-2- trifluoromethyl-3H- imidazol-4-yl)- pyrimidin-2-ylamino]- phenyl}-methanone | (400.132 MHz, CDC1₃) 8.48 (d, 1H), 7.63 - 7.61 (m, 3H), 7.55 (d, 2H), 7.35 (s, 1H), 7.04 (d, 1H), 4.69 (q, 2H), 3.96 - 3.78 (m, 1H), 3.71 - 3.35 (m, 3H), 2.83 - 2.61 (m, 1H), 2.30 (s, 3H), 2.22 (s, 3H), 2.20 - 2.04 (m, 1H), 1.90 - 1.79 (m, 1H), 1.34 (t, 3H) | 474 | Method 37 and Method 48 |
| **50** | {4-[4-(2-Cyclopropyl- 3-isopropyl-3*H*- imidazol-4-yl)- pyrimidin-2-ylamino]- phenyl}-((S)-3- dimethylamino- pyrrolidin-1-yl)- methanone | (400.132 MHz, CDCl₃) 8.36 (d, 1H), 7.66 (d, 2H), 7.53 (d, 2H), 7.50 (s, 1H), 7.33 (s, 1H), 6.93 (d, 1H), 5.73 (septet, 1H), 3.99 - 3.33 (m, 4H), 2.82 - 2.62 (m, 1H), 2.31 (s, 3H), 2.23 (s, 3H), 2.18 - 1.99 (m, 2H), 1.84 (quintet, 1H), 1.63 (d, 6H), 1.12 - 1.08 (m, 2H), 1.06 - 1.00 (m, 2H) | 460 | Method 46 and Method 47 |
| **51** | {4-[4-(2-Cyclopropyl- 3-isopropyl-3H- imidazol-4-yl)- pyrimidin-2-ylamino]- phenyl}-((R)-3- dimethylamino- pyrrolidin-1-y)- methanone | (400.132 MHz, CDC1₃) 8.36 (d, 1H), 7.66 (d, 2H), 7.53 (d, 2H), 7.33 (s, 1H), 7.31 (s, 1H), 6.93 (d, 1H), 5.73 (septet, 1H), 3.96 - 3.35 (m, 4H), 2.83 - 2.62 (m, 1H), 2.31 (s, 3H), 2.22 (s, 3H), 2.19 - 1.99 (m, 2H), 1.92 - 1.79 (m, 1H), 1.64 (d, 6H), 1.20 - 1.16 (m, 2H), 1.07 - 1.02 (m, 2H) | 460 | Method 46 and Method 48 |
| **52** | {4-[4-(2- Difluoromethyl-3- ethyl-3H-imidazol-4- yl)-pyrimidin-2- ylamino]-phenyl}-((S)- 3-dimethylamino- pyrrolidin-1-yl)- methanone | (400.132 MHz, CDC1₃) 8.45 (d, 1H), 7.64 - 7.54 (m, 6H), 7.02 (d, 1H), 6.80 (t, 1H), 4.73 (q, 2H), 3.98 - 3.78 (m, 1H), 3.72 - 3.34 (m, 3H), 2.84 - 2.62 (m, 1H), 2.31 (s, 3H), 2.23 - 2.03 (m, 4H), 1.84 (quintet, 1H), 1.34 (t, 3H) | 456 | Method 42 and Method 47 |
| **53** | {4-[4-(2- Difluoromethyl-3- ethyl-3H-imidazol-4- yl)-pyrimidin-2- ylamino]-phenyl}-((R)-3-dimethylamino- pyrrolidin-1-yl)- methanone | (400.132 MHz, CDC1₃) 8.46 (d, 1H), 7.63 (d, 2H), 7.58 (s, 1H), 7.55 (d, 2H), 7.41 (s, 1H), 7.02 (d, 1H), 6.79 (t, 1H), 4.74 (q, 2H), 3.98 - 3.78 (m, 1H), 3.72 - 3.34 (m, 3H), 2.82 - 2.61 (m, 1H), 2.31 (s, 3H), 2.22 - 2.02 (m, 4H), 1.84 (quintet, 1H), 1.35 (t, 3H) | 456 | Method 42 and Method 48 |
| **54** | 1-(4-{[4-(1-Isopropyl- 2-methyl-1H-imidazol- 5-yl)pyrimidin-2- yl]amino}benzoyl)pyrr olidin-3-ol | 9.67 (s, 1H), 8.42 (d, 1H), 7.74 (d, 2H), 7.48 (d, 2H), 7.43 (s, 1H), 7.09 (d, 1H), 5.77-5.61 (m, 1H), 4.99-4.86 (br d, 1H), 4.35-4.18 (br d, 1H), 3.67-3.39 (m, 4H), 2.01- 1.71 (m, 2H), 1.46 (d, 6H) | 407 | Method 14 and Method 53 |
| **55** | 1-(4-{[5-Fluoro-4-(1- isopropyl-2-methyl-1H-imidazol-5-yl)pyrimidin-2- yl]amino}benzoyl)pyrr olidin-3-ol | 9.77 (s, 1H), 8.58 (d, 1H), 7.70 (d, 2H), 7.47 (d, 2H), 7.37 (d, 1H), 5.51-5.35 (m, 1H), 5.00-4.85 (br d, 1H), 4. 3 6-4.16 (br d, 1H), 3.71- 3.41 (m, 4H), 2.03-1.69 (m, 2H), 1.45 (d, 6H) | 424 | Method 2 and Method 53 |
| **56** | N-(4-{[(3S)-3-(Dimethylamino)pyrrol idin-1- yl]carbonyl}phenyl)-5- fluoro-4-(1-isopropyl- 2-methyl-1H-imidazol- 5-yl)pyrimidin-2-amine | (400.132 MHz, CDC1₃) 8.31 (d, 1H), 7.64 - 7.58 (m, 3H), 7.56 - 7.51 (m, 2H), 7.37 - 7.34 (m, 1H), 5.66 - 5.55 (m, 1H), 3.98 - 3.77 (m, 1H), 3.72 - 3.51 (m, 2H), 3.47 - 3.36 (m, 1H), 2.82 - 2.59 (m, 4H), 2.31 (s, 3H), 2.22 - 2.05 (m, 4H), 1.89 - 1.77 (m, 1H), 1.53 (d, 6H) | 452 | Method 2 and Method 47 |
| **57** | N-(4-{[(3R)-3-(Dimethylamino)pyrrol idin-1- yl]carbonyl}phenyl)-5- fluoro-4-(1-isopropyl- 2-methyl-1H-imidazol- 5-yl)pyrimidin-2-amine | (400.132 MHz, CDC1₃) 8.31 (d, 1H), 7.65 - 7.58 (m, 3H), 7.57 - 7.50 (m, 3H), 5.65 - 5.54 (m, 1H), 3.97 - 3.77 (m, 1H), 3.72 - 3.50 (m, 2H), 3.47 - 3.35 (m, 1H), 2.80 - 2.60 (m, 4H), 2.31 (s, 3H), 2.23 - 2.04 (m, 4H), 1.91 - 1.77 (m, 1H), 1.53 (d, 6H) | 452 | Method 2 and Method 48 |
| **58** | 4-(1-Cyclobutyl-2- methyl-1H-imidazol-5-yl)-N-(4-{[(3S)-3-(dimethylamino)pyrroli din-1- yl]carbonyl}phenyl)-5- fluoropyrimidin-2- amine | (400.132 MHz, CDC1₃) 8.29 (s, 1H), 7.64 (d, 2H), 7.55 (d, 2H), 7.50 (d, 1H), 7.25 (s, 1H), 5.31 (quintet, 1H), 3.98 - 3.76 (m, 1H), 3.72 - 3.33 (m, 3H), 2.83 - 2.56 (m, 4H), 2.51 - 2.38 (m, 4H), 2.34 - 2.03 (m, 7H), 1.90 - 1.67 (m, 3H) | 464 | Method 55 and Method 47 |
| **59** | 4-(1-Cyclobutyl-2- methyl-1H-imidazol-5-yl)-N-(4-{[(3R)-3-(dimethylamino)pyrroli din-1- yl]carbonyl}phenyl)-5- fluoropyrimidin-2- amine | (400.132 MHz, CDC1₃) 8.29 (d, 1H), 7.64 (d, 2H), 7.55 (d, 2H), 7.50 (d, 1H), 7.43 (s, 1H), 5.31 (quintet, 1H), 3.96 - 3.77 (m, 1H), 3.73 - 3.33 (m, 3H), 2.82 - 2.59 (m, 4H), 2.48 - 2.40 (m, 4H), 2.34 - 2.12 (m, 7H), 1.89 - 1.65 (m, 3H) | 464 | Method 55 and Method 48 |
| **60** | 5-Chloro-4-(1- isopropyl-2-methyl-1H-imidazol-5-yl)-N- {4-[(4-methylpiperazin-1- yl)carbonyl]phenyl}py rimidin-2-amine | 9.98 (s, 1H), 8.64 (s, 1H), 7.74 (d, 2H), 7.34 (d, 2H), 7.27 (s, 1H), 4.83 (septet, 1H), 3.54 - 3.44 (m, 4H), 2.50 (s, 3H), 2.34 - 2.28 (m, 4H), 2.20 (s, 3H), 1.37 (d, 6H) | 455 | Method 5 in WO05/07546 1 and Example 59 in WO03/00447 2 |
| **61** | 5-Chloro-N-(4- {[(3R)-3-(dimethylamino)pyrroli din-1- yl]carbonyl}phenyl)-4-(1-isopropyl-2-methyl-1H-imidazol-5-yl)pyrimidin-2-amine | (400.132 MHz, CDC1₃) 8.44 (s, 1H), 7.63 - 7.61 (m, 3H), 7.52 - 7.51 (m, 3H), 4.96 (septet, 1H), 3.97 - 3.76 (m, 1H), 3.70 - 3.38 (m, 3H), 2.88 - 2.64 (m, 1H), 2.59 (s, 3H), 2.31 - 2.00 (m, 7H), 1.85 (quintet, 1H), 1.47 (d, 6H) | 469 | Method 5 in WO05/07546 1 and Method 48 |
| **62** | 5-Chloro-N-(4-{[(3S)- 3-(dimethylamino)pyrroli din-1- yl]carbonyl}phenyl)-4-(1-isopropyl-2-methyl-1H-imidazol-5-yl)pyrimidin-2-amine | (400.132 MHz, CDC1₃) 8.45 (s, 1H), 7.63 (d, 2H), 7.56 - 7.51 (m, 4H), 4.97 (septet, 1H), 3.99 - 3.76 (m, 1H), 3.72 - 3.34 (m, 3H), 2.87 - 2.64 (m, 1H), 2.60 (s, 3H), 2.37 - 2.03 (m, 7H), 1.86 (quintet, 1H), 1.48 (d, 7H) | 469 | Method 5 in WO05/07546 1 and Method 47 |
| **63** | 5-Chloro-4-(1- isopropyl-2-methyl-1H-imidazol-5-yl)-N- {4-[(4-methyl-l,4- diazepan-1- yl)carbonyl]phenyl}py rimidin-2-amine | (400.132 MHz, CDC1₃) 8.45 (s, 1H), 7.61 (d, 2H), 7.52 (d, 2H), 7.39 (d, 2H), 4.96 (septet, 1H), 3.86 - 3.71 (m, 2H), 3.64 - 3.47 (m, 2H), 2.84 - 2.76 (m, 1H), 2.70 - 2.54 (m, 6H), 2.47 - 2.33 (m, 3H), 2.06 - 1.84 (m, 2H), 1.48 (d, 6H) | 469 | Method 5 in WO05/07546 1 and Method 56 |
| **64** | 4-(2-Cyclopropyl-1-isopropyl-1H- imidazol-5-yl)-N-{4-[(4-methyl-1,4-diazepan-1- yl)carbonyl]phenyl}py rimidin-2-amine | (400.132 MHz, CDC1₃) 8.36 (d, 1H), 7.65 (d, 2H), 7.53 (s, 1H), 7.40 (d, 2H), 7.33 (s, 1H), 6.93 (d, 1H), 5.72 (septet, 1H), 3.86 - 3.72 (m, 2H), 3.65 - 3.50 (m, 2H), 2.85 - 2.54 (m, 4H), 2.47 - 2.33 (m, 3H), 2.06 - 1.86 (m, 3H), 1.63 (d, 6H), 1.20-1.16 (m, 2H), 1.07 - 1.02 (m, 2H) | 460 | Method 46 and Method 56 |
| **65** | 4-(1-Cyclobutyl-2- methyl-1H-imidazol-5-yl)-N-(4-{[(3S)-3-(dimethylamino)pyrroli din-1- yl]carbonyl}phenyl)py rimidin-2-amine | (400.132 MHz, CDC1₃) 8.38 (d, 1H), 7.70 - 7.68 (m, 2H), 7.58 - 7.52 (m, 3H), 7.31 (s, 1H), 6.91 (d, 1H), 5.43 (quintet, 1H), 3.96 - 3.35 (m, 4H), 2.76 - 2.59 (m, 4H), 2.50 - 2.43 (m, 4H), 2.33 - 2.05 (m, 8H), 1.89 - 1.68 (m, 2H) | 447 | Method 51 in WO 03/ 076435 and Method 47 |
| **66** | 4-(1-Cyclobutyl-2- methyl-1H-imidazol-5-yl)-N-(4-{[(3R)-3-(dimethylamino)pyrroli din-1- yl]carbonyl}phenyl)py rimidin-2-amine | (400.132 MHz, CDC1₃) 8.38 (d, 1H), 7.70 - 7.68 (m, 2H), 7.56 - 7.52 (m, 3H), 7.31 (s, 1H), 6.91 (d, 1H), 5.43 (quintet, 1H), 3.94 - 3.38 (m, 4H), 2.76 - 2.59 (m, 4H), 2.50 - 2.43 (m, 4H), 2.34 - 2.06 (m, 8H), 1.89 - 1.66 (m, 2H) | 447 | Method 51 in WO 03 / 076435 and Method 48 |
| **67** | N-(4-{[(3R)-3-(Dimethylamino)pyrrol idin-1- yl]carbonyl}phenyl)-4-( 1-isopropyl-2-methyl-1H-imidazol-5-yl)pyrimidin-2-amine | 9.69 (s, 1H), 8.44 (d, 1H), 7.76 (d, 2H), 7.50 (br d, 2H), 7.44 (s, 1H), 7.11 (d, 2H), 5.77-5.64 (m, 1H), 3.79-3.43 (br t, 3H), 3.38-3.79 (m, 1H, under water), 2.81-2.59 (m, s), 2.28-1.95 (m, 7H), 1.81-1.64 (br m, 1H), 1.47 (m, 6H) | 434 | Method 14 and Method 48 |
| **68** | N-{4-[(4-Isopropyl- 1,4-diazepan-1- yl)carbonyl]phenyl}-4-(1-isopropyl-2-methyl-1H-imidazol-5-yl)pyrimidin-2-amine | 9.17 (s, 1H), 8.39 (d, 1H), 7.71 (d, 2H), 7.37 (s, 1H), 7.30 (d, 2H), 7.03 (d, 1H), 5.61 (septet, 1H), 3.55 - 3.48 (m, 4H), 2.94 - 2.84 (m, 1H), 2.68 - 2.66 (m, 2H), 2.63 (t, 2H), 2.49 (s, 3H), 1.74 - 1.68 (m, 2H), 1.47 (d, 6H), 0.98 (d, 6H) | 462 | Method 14 and Method 58 |
| **69** | N-(4-{[(3S)-3-(Dimethylamino)pyrrol idin-1- yl]carbonyl}phenyl)-4-( 1-isopropyl-2-methyl-1H-imidazol-5-yl)pyrimidin-2-amine | 9.69 (s, 1H), 8.44 (d, 1H), 7.76 (d, 2H), 7.50 (br d, 2H), 7.44 (s, 1H), 7.10 (d, 1H), 5.77-5.64 (m, 1H), 3.78-3.41 (br t, 3H), 3.40-3.17 (m, 1H, under water), 2. 79-2.5 8 (m, 1H), 2.50 (s, 3H, under DMSO), 2.27-1.96 (m, 7H), 1.81-1.63 (br m, 1H), 1.51-1.43 (m, 6H) | 434 | Method 14 and Method 47 |
| **70** | 4-(1-Isopropyl-2- methyl-1H-imidazol-5-yl)-N-{3-methyl-4-[(4-methyl-1,4-diazepan- 1- yl)carbonyl]phenyl}py rimidin-2-amine | 9.02 (s, 1H), 8.38 (d, 1H), 7.58 (dd, 1H), 7.47 (d, 1H), 7.36 (s, 1H), 7.06 (d, 1H), 7.00 (d, 1H), 5.57 (septet, 1H), 3.80 - 3.12 (m, 4H), 2.66 - 2.52 (m, 4H), 2.48 (s, 3H), 2.30 (s, 3H), 2.22 (s, 3H), 1.83 - 1.69 (m, 2H), 1.45 (d, 6H) | 448 | Method 14 and Method 59 |
| **71** | N-{3-Fluoro-4-[(4-methyl-1,4-diazepan- 1-yl)carbonyl]phenyl}- 4-(1-isopropyl-2- methyl-1H-imidazol-5-yl)pyrimidin-2-amine | 9.41 (s, 1H), 8.43 (d, 1H), 7.71 (dd, 1H), 7.50 (dd, 1H), 7.38 (s, 1H), 7.23 (t, 1H), 7.08 (d, 1H), 5.58 (septet, 1H), 3.78 - 3.30 (m, 4H), 2.66 - 2.52 (m, 4H), 2.50 (s, 3H), 2.30 (s, 3H), 1.86 - 1.71 (m, 2H), 1.48 (d, 6H) | 452 | Method 14 and Method 60 |
| **72** | N-(4-{[(3R)-3-(Dimethylamino)pyrrol idin-1-yl]carbonyl}-3- fluorophenyl)-4-(1- isopropyl-2-methyl-1H-imidazol-5-yl)pyrimidin-2-amine | 9.86 (d, 1H), 8.47 (d, 1H), 7.79 (d, 1H), 7.49 (d, 1H), 7.46 (s, 1H), 7.40-7.30 (m, 1H), 7.15(d, 1H), 5.73-5.60 (m, 1H), 3.76-3.58 (m, 1H), 3.49-3.35 (m, 2H, under water), 3.24-3.09 (m, 1H), 2.81- 2.65 (m, 1H), 2.51 (s, 3H, under DMSO), 2.16 (d, 6H), 2.05-1.97 (m, 1H), 1.82-1.66 (m, 1H), 1.48 (d, 6H) | 452 | Method 14 and Method 62 |
| **73** | 4-(1-Isopropyl-2- methyl-1H-imidazol-5-yl)-N-[3-methyl-4-(1,4-oxazepan-4- ylcarbonyl)phenyl]pyri midin-2-amine | 9.01 (s, 1H), 8.38 (d, 1H), 7.59 (dd, 1H), 7.49 (s, 1H), 7.35 (s, 1H), 7.09 (d, 1H), 7.00 (d, 1H), 5.56 (septet, 1H), 3.78 - 3.39 (m, 8H), 2.48 (s, 3H), 2.23 (s, 3H), 1.86 - 1.74 (m, 2H), 1.46 (d, 6H) | 435 | Method 14 and Method 63 |
| **74** | N-[3-Fluoro-4-(1,4-oxazepan-4- ylcarbonyl)phenyl]-4-(1-isopropyl-2-methyl-1H-imidazol-5-yl)pyrimidin-2-amine | 9.43 (s, 1H), 8.43 (d, 1H), 7.73 (dd, 1H), 7.52 (dd, 1H), 7.38 (s, 1H), 7.26 (t, 1H), 7.08 (d, 1H), 5.58 (septet, 1H), 3.84 - 3.42 (m, 8H), 2.50 (s, 3H), 1.89 - 1.75 (m, 2H), 1.49 (d, 6H) | 439 | Method 14 and Method 64 |
| **75** | N-(4-{[(3R)-3-(Dimethylamino)pyrrol idin-l-yl]carbonyl}-3- fluorophenyl)-5- fluoro-4-(1-isopropyl- 2-methyl-1H-imidazol- 5-yl)pyrimidin-2-amine | 9.97 (d, 1H), 8.65 (d, 1H), 7.73 (d, 1H), 7.46 (d, 1H), 7.42-7.40 (m, 2H), 5.49-5.35 (m, 1H), 3.76-3.57 (m, 1H), 3.48-3.33 (m, 2H, under water), 3.24-3.08 (m, 1H), 2.79- 2.62 (m, 1H), 2.51 (s, 3H, under DMSO), 2.54 (s, 3H), 2.22-1.95 (m, 7H), 1.82-1.64 (m, 1H), 1.46 (d, 6H) | 470 | Method 2 and Method 62 |
| **76** | N-(4-{[(3S)-3-(Dimethylamino)pyrrol idin-l-yl]carbonyl}-3- fluorophenyl)-5- fluoro-4-(1-isopropyl- 2-methyl-1H-imidazol- 5-yl)pyrimidin-2-amine | 9.96 (d, 1H), 8.63 (d, 1H), 7.73 (d, 1H), 7.46 (d, 1H), 7.41-7.30 (m, 2H), 5.49-5.34 (m, 1H), 3.75-3.57 (m, 1H), 3.47-3.34 (m, 2H, under water), 3.24-3.08 (m, 1H), 2.79- 2.63 (m, 1H), 2.54 (s, 3H), 2.21- 1.96 (m, 7H), 1.81-1.65 (m, 1H), 1.47 (d, 6H) | 470 | Method 2 and Method 61 |
| **77** | N-(4-{[(3R)-3-(Dimethylamino)pyrrol idin-l-yl]carbonyl}-3- methylphenyl)-5- fluoro-4-(1-isopropyl- 2-methyl-1H-imidazol- 5-yl)pyrimidin-2-amine | 9.57 (d, 1H), 8.57 (d, 1H), 7.57 (d, 1H), 7.44 (s, 1H), 7.39-7.35 (m, 1H), 7.14 (t, 1H), 5.48-5.34 (m, 1H), 3.77-3.60 (m, 1H), 3.47-2.93 (m, 3H, under water), 2.77-2.62 (m, 1H), 2.52 (s, 3H), 2.23-1.94 (m, 10H), 1.81-1.64 (m, 1H), 1.44 (d, 6H) | 466 | Method 2 and Method 67 |
| **78** | 4-(1-Isopropyl-2- methyl-1H-imidazol-5-yl)-N-{4-[8-oxa-3- azabicyclo[3.2.1]oct- 3- ylcarbonyl]phenyl}pyri midin-2-amine | 9.21 (s, 1H), 8.40 (d, 1H), 7.74 (d, 2H), 7.36 (s, 1H), 7.32 (d, 2H), 7.03 (d, 1H), 5.60 (septet, 1H), 4.28 (s, 2H), 3.77 (d, 2H), 3.19 (d, 2H), 2.49 (s, 3H), 1.82 (dd, 2H), 1.71 (d, 2H), 1.47 (d, 6H) | 433 | Method 14 and Method 65 |
| **79** | [4-[[4-[3-(Cyclobutylmethyl)-2- methyl-3H-imidazol-4- yl]pyrimidin-2- yl]amino]phenyl]-(4-methylpiperazin-1- yl)methanone | 9.24 (s, 1H), 8.37 (d, 1H), 7.72 (d, 2H), 7.53 (s, 1H), 7.34 (d, 2H), 7.07 (d, 1H), 4.63 (d, 2H), 3.51 (t, 4H), 2.57 - 2.49 (m, 1H obscured by DMSO), 2.40 (s, 3H), 2.34 (t, 4H), 2.22 (s, 3H), 1.83 - 1.48 (m, 6H) | 446 | Example 59 of WO03 / 004472 and Method 78 |
| **80** | [4-[[4-[3-(Cyclobutylmethyl)-2- methyl-3H-imidazol-4- yl]pyrimidin-2- yl]amino]phenyl]-(4-methyl-1,4-diazepan- 1-yl)methanone | 9.20 (s, 1H), 8.37 (d, 1H), 7.71 (d, 2H), 7.53 (s, 1H), 7.32 (d, 2H), 7.07 (d, 1H), 4.63 (d, 2H), 3.58 - 3.53 (m, 4H), 2.64 - 2.50 (m, 4H + 1H obscured by DMSO), 2.40 (s, 3H), 2.30 (s, 3H), 1.83 - 1.48 (m, 8H) | 460 | Method 56 and Method 78 |
| **81** | [4-[[4-[3-(Cyclobutylmethyl)-2- methyl-3H-imidazol-4- yl]pyrimidin-2- yl]amino]phenyl]-[(3S)-3-(dimethylamino)pyrroli din-1-yl]methanone | 9.21 (s, 1H), 8.38 (d, 1H), 7.71 (d, 2H), 7.53 (s, 1H), 7.47 (d, 2H), 7.07 (d, 1H), 4.63 (d, 2H), 3.66 - 3.56 (m, 2H), 3.51 - 3.45 (m, 1H), 3.34 - 3.30 (m, 1H), 2.81 - 2.75 (m, 1H), 2.56 - 2.50 (m, 1H obscured by DMSO), 2.40 (s, 3H), 2.18 (s, 6H), 2.06 - 1.98 (m, 1H), 1.82 - 1.64 (m, 5H), 1.58 - 1.49 (m, 2H) | 460 | Method 47 and Method 78 |
| **82** | [4-[[4-(3-Cyclopentyl- 2-methyl-3H-imidazol- 4-yl)pyrimidin-2- yl]amino]phenyl]-(4-methylpiperazin-1- yl)methanone | 9.22 (s, 1H), 8.41 (d, 1H), 7.73 - 7.71 (m, 2H), 7.34 (s, 1H), 7.34 - 7.31 (m, 2H), 7.02 (d, 1H), 5.59 (quintet, 1H), 3.52 - 3.50 (m, 4H), 2.48 (s, 3H), 2.35 - 2.33 (m, 4H), 2.22 (s, 3H), 2.11 - 2.04 (m, 2H), 2.00 - 1.92 (m, 2H), 1.84 - 1.76 (m, 2H), 1.60 - 1.51 (m, 2H) | 446 | Example 59 of WO03 / 004472 and Method 84 |
| **83** | [4-[[4-(3-Cyclopentyl- 2-methyl-3H-imidazol- 4-yl)pyrimidin-2- yl]amino]phenyl]-(4-methyl-1,4-diazepan- 1-yl)methanone | 9.20 (s, 1H), 8.41 (d, 1H), 7.72 - 7.69 (m, 2H), 7.34 (s, 1H), 7.31 - 7.29 (m, 2H), 7.01 (d, 1H), 5.59 (quintet, 1H), 3.58 - 3.53 (m, 4H), 2.62 - 2.55 (m, 4H), 2.48 (s, 3H, Me obscured by DMSO), 2.30 (s, 3H), 2.11 - 2.04 (m, 2H), 2.00 - 1.92 (m, 2H), 1.84 - 1.76 (m, 4H), 1.60 - 1.52 (m, 2H) | 460 | Method 56 and Method 84 |
| **84** | [4-[[4-(3-Cyclopentyl- 2-methyl-3H-imidazol- 4-yl)pyrimidin-2- yl]amino]phenyl]-[(3S)-3- dimethylaminopyrrolid in-1-yl]methanone | 9.23 (s, 1H), 8.41 (d, 1H), 7.73 - 7.71 (m, 2H), 7.47 - 7.44 (m, 2H), 7.34 (s, 1H), 7.02 (d, 1H), 5.59 (quintet, 1H), 3.65 - 3.57 (m, 2H), 3.51 - 3.45 (m, 1H), 3.33 - 3.29 (m, 1H), 2.78 (quintet, 1H), 2.48 (s, 3H, methyl obscured by DMSO), 2.18 (s, 6H), 2.11-1.92 (m, 5H), 1.84 - 1.73 (m, 3H), 1.60 - 1.52 (m, 2H) | 460 | Method 47 and Method 84 |
| **85** | [4-[[4-(3-Cyclopentyl- 2-methyl-3H-imidazol- 4-yl)pyrimidin-2- yl]amino]phenyl]-(4- propan-2-yl-1,4-diazepan-1- yl)methanone | 9.19 (s, 1H), 8.41 (d, 1H), 7.72 - 7.69 (m, 2H), 7.34 (s, 1H), 7.29 (m, 2H), 7.01 (d, 1H), 5.60 (quintet, 1H), 3.55 - 3.50 (m, 4H), 2.92 - 2.84 (m, 1H obscured by H₂O), 2.69 - 2.61 (m, 4H), 2.48 (s, 3H, Aromatic methyl obscured by DMSO), 2.11 - 2.04 (m, 2H), 2.00 - 1.92 (m, 2H), 1.84 - 1.68 (m, 4H), 1.60 - 1.52 (m, 2H), 0.97 (d, 6H) | 488 | Method 58 and Method 84 |
| **86** | [4-[[4-(2-Methyl-3- propan-2-yl-3H- imidazol-4- yl)pyrimidin-2- yl]amino]phenyl]-[(1S,4S)-2-propyl-2,5- diazabicyclo[2.2.1]hep t-5-yl]methanone | 9.23 (s, 1H), 8.41 (d, 1H), 7.74 (d, 2H), 7.45 (d, 2H), 7.37 (s, 1H), 7.04 (d, 1H), 5.61 (septet, 1H), 4.40 (s, 1H), 3.49 (s, 1H), 3.46 (d, 1H), 3.39 (dd, 1H), 2.85 (dd, 1H), 2.65 (d, 1H), 2.54 - 2.41 (m, 5H), 1.77 (d, 1H), 1.68 (d, 1H), 1.48 (d, 6H), 1.41 (sextet, 2H), 0.88 (t, 3H) | 460 | Method 14 and Method 70 |
| **87** | [4-[[5-Fluoro-4-(2- methyl-3-propan-2-yl- 3H-imidazol-4- yl)pyrimidin-2- yl]amino]phenyl]-(4- propan-2-yl-1,4-diazepan-1- yl)methanone | 9.27 (s, 1H), 8.48 (d, 1H), 7.67 (d, 2H), 7.36 (d, 1H), 7.30 (d, 2H), 5.41 (septet, 1H), 3.54 - 3.49 (m, 4H), 2.93 - 2.84 (m, 1H), 2.66 (t, 2H), 2.63 (t, 2H), 2.52 (s, 3H), 1.74 - 1.69 (m, 2H), 1.45 (d, 6H), 0.97 (d, 6H) | 480 | Method 2 and Method 58 |
| **88** | [4-[[5-Fluoro-4-(2- methyl-3-propan-2-yl- 3H-imidazol-4- yl)pyrimidin-2- yl]amino]-2-methyl- phenyl]-(4-methyl-1,4-diazepan-1- yl)methanone | 9.10 (s, 1H), 8.46 (d, 1H), 7.53 (dd, 1H), 7.43 (s, 1H), 7.35 (d, 1H), 7.05 (d, 1H), 5.38 (septet, 1H), 3.77 - 3.15 (m, 4H), 2.59 - 2.53 (m, 4H), 2.51 (s, 3H), 2.30 (s, 3H), 2.21 (s, 3H), 1.85 - 1.67 (m, 2H), 1.44 (d, 6H) | 466 | Method 2 and Method 59 |
| **89** | [2-Fluoro-4-[[5- fluoro-4-(2-methyl-3- propan-2-yl-3H- imidazol-4- yl)pyrimidin-2- yl]amino]phenyl]-(4-methyl-1,4-diazepan- 1-yl)methanone | 9.50 (s, 1H), 8.52 (d, 1H), 7.64 (dd, 1H), 7.47 (dd, 1H), 7.37 (d, 1H), 7.23 (t, 1H), 5.38 (septet, 1H), 3.76 - 3.27 (m, 4H), 2.65 - 2.54 (m, 4H), 2.52 (s, 3H), 2.30 (s, 3H), 1.86 - 1.72 (m, 2H), 1.47 (d, 6H) | 470 | Method 2 and Method 60 |
| **90** | [4-[[5-Fluoro-4-(2- methyl-3-propan-2-yl- 3H-imidazol-4- yl)pyrimidin-2- yl]amino]-2-methyl- phenyl]-(1,4-oxazepan-4- yl)methanone | (500.133 MHz, CDC1₃) 9.12 (s, 1H), 8.47 (d, 1H), 7.54 (dd, 1H), 7.45 (s, 1H), 7.35 (d, 1H), 7.08 (d, 1H), 5.37 (septet, 1H), 3.74 - 3.29 (m, 8H), 2.51 (s, 3H), 2.22 (s, 3H), 1.85 - 1.72 (m, 2H), 1.44 (d, 6H) | 453 | Method 2 and Method 63 |
| **91** | [2-Fluoro-4-[[5- fluoro-4-(2-methyl-3- propan-2-yl-3H- imidazol-4- yl)pyrimidin-2- yl]amino]phenyl]-(1,4-oxazepan-4- yl)methanone | (500.133 MHz, CDC1₃) 9.52 (s, 1H), 8.53 (d, 1H), 7.65 (dd, 1H), 7.48 (dd, 1H), 7.36 (d, 1H), 7.26 (t, 1H), 5.38 (septet, 1H), 3 .75 - 3.42 (m, 8H), 2.52 (s, 3H), 1.89 - 1.74 (m, 2H), 1.47 (d, 6H) | 457 | Method 2 and Method 64 |
| **92** | [4-[[5-Fluoro-4-(2- methyl-3-propan-2-yl- 3H-imidazol-4- yl)pyrimidin-2- yl]amino]phenyl]-(8- oxa-3- azabicyclo[3.2.1]oct- 3-yl)methanone | 9.33 (s, 1H), 8.49 (d, 1H), 7.69 (d, 2H), 7.35 (d, 1H), 7.32 (d, 2H), 5.40 (septet, 1H), 4.28 (s, 2H), 3.76 (d, 2H), 3.19 (d, 2H), 2.52 (s, 3H), 1.84 - 1.79 (m, 2H), 1.73 - 1.68 (m, 2H), 1.45 (d, 6H) | 451 | Method 2 and Method 65 |
| **93** | [4-[[5-Fluoro-4-(2- methyl-3-propan-2-yl- 3H-imidazol-4- yl)pyrimidin-2- yl]amino]phenyl]-[(1S,4S)-2-propyl-2,5- diazabicyclo[2.2.1]hep t-5-yl]methanone | 9.35 (s, 1H), 8.49 (d, 1H), 7.69 (d, 2H), 7.44 (d, 2H), 7.36 (d, 1H), 5.40 (septet, 1H), 4.46 - 4.33 (m, 1H), 3.48 (s, 1H), 3.45 (d, 1H), 3.38 (dd, 1H), 2.85 (dd, 1H), 2.64 (d, 1H), 2.54 - 2.40 (m,obscured by DMSO, 5H), 1.77 (d, 1H), 1.68 (d, 1H), 1.46 (d, 6H), 1.40 (sextet, 2H), 0.88 (t, 3H) | 478 | Method 2 and Method 70 |
| **94** | [2-Chloro-4-[[5- fluoro-4-(2-methyl-3- propan-2-yl-3H- imidazol-4- yl)pyrimidin-2- yl]amino]phenyl]-(4-methyl-1,4-diazepan- 1-yl)methanone | 9.44 (s, 1H), 8.52 (d, 1H), 7.82 (d, 1H), 7.65 (dd, 1H), 7.36 (d, 1H), 7.22 (d, 1H), 5.35 (septet, 1H), 3.75 - 3.60 (m, 2H), 3.40 - 3.20 (m, 2H), 2.71 - 2.53 (m, 3H), 2.52 (s, 3H), 2.35 - 2.24 (m, 4H), 1.92 - 1.65 (m, 2H), 1.47 (d, 6H) | 487 | Method 2 and Method 68 |
| **95** | [2-Chloro-4-[[5- fluoro-4-(2-methyl-3- propan-2-yl-3H- imidazol-4- yl)pyrimidin-2- yl]amino]phenyl]-(1,4-oxazepan-4- yl)methanone | 9.46 (s, 1H), 8.53 (d, 1H), 7.83 (d, 1H), 7.66 (dd, 1H), 7.36 (d, 1H), 7.25 (d, 1H), 5.34 (septet, 1H), 3.83 - 3.54 (m, 6H), 3.44 - 3.24 (m, 2H), 2.52 (s, 3H), 1.99 - 1.67 (m, 2H), 1.47 (d, 6H) | 474 | Method 2 and Method 69 |
| **96** | [4-[[5-Fluoro-4-(2- methyl-3-propan-2-yl- 3H-imidazol-4- yl)pyrimidin-2- yl]amino]phenyl]-[4-(2-hydroxyethyl)-1,4-diazepan-1- yl]methanone | 9.30 (s, 1H), 8.48 (d, 1H), 7.67 (d, 2H), 7.36 (d, 1H), 7.31 (d, 2H), 5.41 (septet, 1H), 3.98 - 3.81 (m, 1H), 3.57 - 3.46 (m, 6H), 2.75 (t, 2H), 2.70 (t, 2H), 2.60 (t, 2H), 2.51 (s, 3H), 1.76 (quintet, 2H), 1.45 (d, 6H) | 482 | Method 2 and Method 71 |
| **97** | [4-[[5-Fluoro-4-(2- methyl-3-propan-2-yl- 3H-imidazol-4- yl)pyrimidin-2- yl]amino]phenyl]-(1,4-oxazepan-4- yl)methanone | 9.32 (s, 1H), 8.49 (d, 1H), 7.68 (d, 2H), 7.36 (d, 1H), 7.32 (d, 2H), 5.41 (septet, 1H), 3.72 - 3.67 (m, 4H), 3.63 - 3.58 (m, 4H), 2.52 (s, 3H), 1.83 (quintet, 2H), 1.46 (d, 6H) | 439 | Method 2 and Method 72 |
| **98** | [2-Chloro-4-[[4-(2- methyl-3-propan-2-yl- 3H-imidazol-4- yl)pyrimidin-2- yl]amino]phenyl]-(4-methyl-1,4-diazepan- 1-yl)methanone | 9.33 (s, 1H), 8.42 (d, 1H), 7.87 (d, 1H), 7.69 (dd, 1H), 7.37 (s, 1H), 7.22 (d, 1H), 7.07 (d, 1H), 5.53 (septet, 1H), 3 .74 - 3.60 (m, 2H), 3.36 - 3.20 (m, 2H), 2.72 - 2.52 (m, 4H), 2.49 (s, 3H), 2.35 - 2.24 (m, 3H), 1.92 - 1.65 (m, 2H), 1.48 (d, 6H) | 469 | Method 14 and Method 68 |
| **99** | [2-Chloro-4-[[4-(2- methyl-3-propan-2-yl- 3H-imidazol-4- yl)pyrimidin-2- yl]amino]phenyl]-(1,4-oxazepan-4- yl)methanone | 9.35 (s, 1H), 8.43 (d, 1H), 7.88 (d, 1H), 7.70 (dd, 1H), 7.38 (s, 1H), 7.25 (d, 1H), 7.07 (d, 1H), 5.54 (septet, 1H), 3.83 - 3.55 (m, 6H), 3.46 - 3.25 (m, 2H), 2.49 (s, 3H), 1.98 - 1.68 (m, 2H), 1.48 (d, 6H) | 456 | Method 14 and Method 69 |
| **100** | [4-(2-Hydroxyethyl)- 1,4-diazepan-1-yl]-[4-[[4-(2-methyl-3- propan-2-yl-3H- imidazol-4- yl)pyrimidin-2- yl]amino]phenyl]metha none | 9.19 (s, 1H), 8.39 (d, 1H), 7.72 (d, 2H), 7.37 (s, 1H), 7.31 (d, 2H), 7.03 (d, 1H), 5.61 (septet, 1H), 4.02 - 3.80 (m, 1H), 3.54 (m, 4H), 3.49 (t, 2H), 2.77 - 2.74 (m, 2H), 2.72 - 2.70 (m, 2H), 2.60 (t, 2H), 2.49 (s, 3H), 1.79 - 1.74 (m, 2H), 1.47 (d, 6H) | 464 | Method 14 and Method 71 |
| **101** | [4-[[4-(2-Methyl-3- propan-2-yl-3H- imidazol-4- yl)pyrimidin-2- yl]amino]phenyl]-(1,4-oxazepan-4- yl)methanone | 9.21 (s, 1H), 8.40 (d, 1H), 7.73 (d, 2H), 7.37 (s, 1H), 7.33 (d, 2H), 7.03 (d, 1H), 5.60 (septet, 1H), 3.72 - 3.68 (m, 4H), 3.62 - 3.59 (m, 4H), 2.93 (s, 3H), 1.83 (quintet, 2H), 1.47 (d, 6H) | 421 | Method 14 and Method 72 |
| **102** | [4-[[4-(2-Methyl-3- propan-2-yl-3H- imidazol-4- yl)pyrimidin-2- yl]amino]phenyl]-[(3S)-3-(pyrrolidin-1- yl)pyrrolidin-1- yl]methanone | 9.69 (s, 1H), 8.44 (d, 1H), 7.76 (d, 2H), 7.50 (d, 2H), 7.44 (s, 1H), 7.10 (d, 1H), 5.70 (septet, 1H), 3.71 - 3.38 (m, 4H), 2.81 - 2.60 (m, 1H), 2.58 - 2.30 (m, 7H, obscured by DMSO), 2.10 - 1.93 (m, 1H), 1.88 - 1.59 (m, 5H), 1.47 (d, 6H) | 460 | Method 14 and Method 86 |
| **103** | [4-[[4-(2-Methyl-3- propan-2-yl-3H- imidazol-4- yl)pyrimidin-2- yl]amino]phenyl]-[(3S)-3-(1- piperidinyl)pyrrolidin- 1-yl]methanone | 9.20 (s, 1H), 8.40 (d, 1H), 7.72 (d, 2H), 7.46 (d, 2H), 7.37 (s, 1H), 7.04 (d, 1H), 5.60 (septet, 1H), 3.67 - 3.64 (m, 2H), 3.61 - 3.57 (m, 1H), 3.49 - 3.43 (m, 1H), 3.33 - 3.30 (m, 1H), 2.49 (s, 3H), 2.47 - 2.42 (m, 2H), 2.38 - 2.33 (m, 2H), 2.08 - 2.02 (m, 1H), 1.80 - 1.72 (m, 1H), 1.53 - 1.46 (m, 10H),1.43- 1.37 (m, 2H) | 474 | Method 14 and Method 99 |
| **104** | [(3S)-3-(Cyclopropylamino)py rrolidin-1-yl]-[4-[[4-(2-methyl-3-propan-2- yl-3H-imidazol-4- yl)pyrimidin-2- yl]amino]phenyl]metha none | 9.68 (s, 1H), 8.44 (d, 1H), 7.76 (d, 2H), 7.49 (d, 2H), 7.44 (s, 1H), 7.10 (d, 1H), 5.71 (septet, 1H), 3.62 - 3.53 (m, 2H), 3.51 - 3.34 (m, 2H), 2.55 - 2.35 (m, 5H), 2.12 - 1.90 (m, 2H), 1.84 - 1.71 (m, 1H), 1.47 (d, 6H), 0.40 - 0.09 (m, 4H) | 446 | Method 14 and Method 89 |
| **105** | (4-Cyclopropyl-1,4-diazepan-1-yl)-[4-[[4-(2-methyl-3-propan-2- yl-3H-imidazol-4- yl)pyrimidin-2- yl]amino]phenyl]metha none | 9.64 (s, 1H), 8.43 (d, 1H), 7.74 (d, 2H), 7.44 (s, 1H), 7.33 (d, 2H), 7.09 (d, 1H), 5.70 (septet, 1H), 3.65 - 3.38 (m, 4H), 2.90 - 2.68 (m, 4H), 2.51 (s, 3H), 1.97 - 1.63 (m, 3H), 1.46 (d, 6H), 0.49 - 0.37 (m, 2H), 0.32 - 0.24 (m, 2H) | 460 | Method 14 and Method 101 |
| **106** | 1,4-Diazepan-1-yl-[4-[[4-(2-methyl-3- propan-2-yl-3H- imidazol-4- yl)pyrimidin-2- yl]amino]phenyl]metha none | (400.132 MHz, CDC1₃) 8.37 (d, 1H), 7.66 (d, 2H), 7.42 - 7.35 (m, 4H), 6.95 (d, 1H), 5.64 (septet, 1H), 4.04 - 3.45 (m, 4H), 3.20 - 2.64 (m, 5H), 2.59 (s, 3H), 2.07 - 1.75 (m, 2H), 1.53 (d, 6H) | 420 | Method 14 and Method 100 |
| **107** | (4-Cyclobutyl-1,4-diazepan-1-yl)-[4-[[4-(2-methyl-3-propan-2- yl-3H-imidazol-4- yl)pyrimidin-2- yl]amino]phenyl]metha none | (400.132 MHz, CDC1₃) 8.37 (d, 1H), 7.64 (d, 2H), 7.40 (d, 1H), 7.38 (s, 1H), 7.15 (s, 1H), 6.95 (d, 1H), 5.64 (septet, 1H), 3.90 - 3.68 (m, 2H), 3.66 - 3.44 (m, 2H), 3.03 - 2.33 (m, 8H), 2.18 - 1.57 (m, 9H), 1.53 (d, 6H) | 474 | Method 14 and Method 102 |
| **108** | [(3S)-3- Methylaminopyrrolidin -1-yl]-[4-[[4-(2- methyl-3-propan-2-yl- 3H-imidazol-4- yl)pyrimidin-2- yl]amino]phenyl]metha none | (400.132 MHz, CDC1₃) 8.37 (d, 1H), 7.65 (d, 2H), 7.54 (d, 2H), 7.38 (s, 1H), 7.28 (s, 1H), 6.95 (d, 1H), 5.66 (septet, 1H), 3.92 - 3.17 (m, SH), 2.59 (s, 3H), 2.52 - 2.36 (m, 3H), 2.23 - 1.98 (m, 1H), 1.81 (sextet, 1H), 1.72 - 1.61 (m, 1H), 1.53 (d, 6H) | 420 | Method 14 and Method 90 |
| **109** | [(1S,4S)-2,5- Diazabicyclo[2.2.1]he pt-5-yl]-[4-[[4-(2- methyl-3-propan-2-yl- 3H-imidazol-4- yl)pyrimidin-2- yl]amino]phenyl]metha none | 9.22 (s, 1H), 8.40 (d, 1H), 7.74 (d, 2H), 7.44 (d, 2H), 7.36 (s, 1H), 7.04 (d, 1H), 5.60 (septet, 1H), 4.46 (brs, 1H), 3.62 (s, 1H), 3.51 (dd, 1H), 3.24 (d, 1H), 2.97 (d, 1H), 1.71 (d, 1H), 1.60 (d, 1H), 1.48 (d, 6H) | 418 8 | Method 14 and Method 105 |
| **110** | [4-[[5-Chloro-4-(2- methyl-3-propan-2-yl- 3H-imidazol-4- yl)pyrimidin-2- yl]amino]phenyl]-(4- propan-2-yl-1,4-diazepan-1- yl)methanone | (400.132 MHz, CDC1₃) 8.44 (s, 1H), 7.60 (d, 2H), 7.52 (s, 1H), 7.39 (d, 2H), 7.25 (s, 1H), 4.96 (septet, 1H), 3.82 - 3.70 (m, 2H), 3.54 - 3.45 (m, 2H), 3.02 - 2.75 (m, 2H), 2.75 - 2.59 (m, 5H), 1.96 - 1.70 (m, 3H), 1.48 (d, 6H), 1.01 (d, 6H) | 497 | Method 5 in WO 05/075461 and Method 58 |
| **111** | (4-Cyclobutyl-1,4-diazepan-1-yl)-[4-[[5- fluoro-4-(2-methyl-3- propan-2-yl-3H- imidazol-4- yl)pyrimidin-2- yl]amino]phenyl]metha none | (400.132 MHz, CDC1₃) 8.30 (d, 1H), 7.60 - 7.58 (m, 3H), 7.40 (d, 2H), 7.08 (s, 1H), 5.57 (septet, 1H), 3.81 - 3.72 (m, 2H), 3.59 - 3.49 (m, 2H), 2.95 - 2.77 (m, 1H), 2.68 - 2.58 (m, 4H), 2.55 - 2.41 (m, 3H), 2.11 - 1.59 (m, 8H), 1.53 (d, 6H) | 492 | Method 2 and Method 102 |
| **112** | [4-[[5-Fluoro-4-(2- methyl-3-propan-2-yl- 3H-imidazol-4- yl)pyrimidin-2- yl]amino]phenyl]-[(3S)-3-(methylamino)pyrrolidi n-1-yl]methanone | (400.132 MHz, CDC1₃) 8.30 (d, 1H), 7.61 - 7.58 (m, 3H), 7.54 (d, 2H), 7.22 (s, 1H), 5.58 (septet, 1H), 3.89 - 3.63 (m, 2H), 3.58 - 3.20 (m, 3H), 2.62 (s, 3H), 2.48 - 2.39 (m, 3H), 2.23 - 1.96 (m, 1H), 1.84 - 1.74 (m, 1H), 1.53 (d, 6H) | 438 | Method 2 and Method 90 |
| **113** | [(3R)-3- Dimethylaminopyrroli din-1-yl]-[2-methyl-4-[[4-(2-methyl-3- propan-2-yl-3H- imidazol-4- yl)pyrimidin-2- yl]amino]phenyl]metha none | 9.47 (d, 1H), 8.41 (d, 1H), 7.63 (d, 1H), 7.47 (s, 1H), 7.43 (d, 1H), 7.14 (t, 1H), 7.07 (d, 1H), 5.72- 5.60 (m, 1H), 3.77-3.60 (m, 1H), 3.47-3.29 (m, 1H under water), 3.27-2.94 (m, 2H under water), 2.75-2.62 (m, 1H), 2.50 (s, 3H under DMSO). 2.19 (d, 6H), 2.15- 1.92 (m, 4H), 1.83-1.63 (m, 1H), 1.45 (d, 6H) | 448 | Method 14 and Method 67 |
| **114** | [(3S)-3- Dimethylaminopyrroli din-1-yl]-[2-fluoro-4-[[4-(2-methyl-3- propan-2-yl-3H- imidazol-4- yl)pyrimidin-2- yl]amino]phenyl]metha none | 9.86 (d, 1H), 8.47 (d, 1H), 7.79 (d, 1H), 7.49 (d, 1H), 7.46 (s, 1H), 7.39-7.30 (m, 1H), 7.15 (d, 1H), 5.73-5.59 (m, 1H), 3.75-3.58 (m, 1H), 3.47-3.33 (m, 2H under water), 3.23-3.08 (m, 1H), 2.77- 2.64 (m, 1H), 2.50 (s, 3H under DMSO), 2.21-1.96 (m, 7H), 1.81- 1.66 (m. 1H), 1.48 (d, 6H) | 452 | Method 14 and Method 61 |
| **115** | [(3S)-3- Dimethylaminopyrroli din-1-yl]-[2-methyl-4-[[4-(2-methyl-3- propan-2-yl-3H- imidazol-4- yl)pyrimidin-2- yl]amino]phenyl]metha none | 9.47 (d, 1H), 8.41 (d, 1H), 7.63 (d, 1H), 7.47 (s, 1H), 7.43 (d, 1H), 7.14 (t, 1H), 7.07 (d, 1H), 5.72- 5.60 (m, 1H), 3.77-3.60 (m, 1H), 3.47-3.32 (m, 1H under water), 3.27-2.95 (m, 2H under water), 2.75-2.63 (m, 1H), 2.50 (s, 3H under DMSO), 2.19 (d, 6H), 2.15- 1.92 (m, 4H), 1.83-1.63 (m, 1H), 1.45 (d, 6H) | 448 | Method 14 and Method 66 |
| **116** | [4-[[4-[2-(Methoxymethyl)-3- propan-2-yl-3H- imidazol-4- yl]pyrimidin-2- yl]amino]phenyl]-(4- propan-2-yl-1,4-diazepan-1- yl)methanone | 9.66 (s, 1H), 8.50 (d, 1H), 7.74 (d, 2H), 7.50 (s, 1H), 7.31 (d, 2H), 7.11 (d, 1H), 5.53 (septet, 1H), 4.57 (s, 2H), 3.59 (m, 2H), 3.41 (m, 2H), 3.31 (s, 3H), 2.86 (m, 1H), 2.68 (m, 1H), 2.65-2.54 (m, 3H), 1.80-1.58 (m, 2H), 1.47 (d, 6H), 0.97 (m, 6H) | 492 | Method 3 and Method 58 |
| **117** | (3-(3S)- Dimethylaminopyrroli din-1-yl)-[4-[[4-[2-(methoxymethyl)-3- propan-2-yl-3H- imidazol-4- yl]pyrimidin-2- yl]amino]phenyl]metha none | 9.71 (s, 1H), 8.51 (d, H), 7.77 (m, 2H), 7.54 - 7.46 (m, 3H), 7.14 (d, 2H), 5.53 (septet, 1H), 4.57 (s, 2H), 3.74 - 3.43 (m, 3H), 3.34 (m, 1H), 2.77 - 2.58 (m, 2H), 2.22 - 2.04 (m, 6H), 1.72 (m, 1H), 1.48 (d, 6H) | 464 | Method 3 and Method 47 |
| **118** | [4-[[4-[2-(Methoxymethyl)-3- propan-2-yl-3H- imidazol-4- yl]pyrimidin-2- yl]amino]phenyl]-(4-methyl-1,4-diazepan- 1-yl)methanone | (+ D₄ AcOH) 8.49 (d, 1H), 7.76 (d, 2H), 7.51 (s, 1H), 7.34 (d, 2H), 7.12 (d, 1H), 5.52 (septet, 1H), 4.58 (s, 2H), 3.68 - 3.40 (m, 4H), 3.29 (s, 3H), 2.68 - 2.55 (m, 2H), 2.35 - 2.21 (m, 3H), 1.90 - 1.72 (m, 2H), 1.48 (d, 6H) | 464 | Method 3 and Method 56 |
| **119** | [3-(3S)-(Cyclobutylamino)pyrr olidin-1-yl]-[4-[[4-(2- methyl-3-propan-2-yl- 3H-imidazol-4- yl)pyrimidin-2- yl]amino]phenyl]metha none | 9.21 (s, 1H), 8.40 (d, 1H), 7.72 (d, 2H), 7.45 (d, 2H), 7.37 (s, 1H), 7.03 (d, 1H), 5.61 (septet, 1H), 3.60 - 3.55 (m, 2H), 3.47 - 3.42 (m, 1H), 3.26 (quintet, 1H), 3 .22 - 3.18 (m, 2H), 2.49 (s, 3H), 2.16 - 2.05 (m, 2H), 2.00 - 1.94 (m, 1H), 1.72 - 1.52 (m, 5H), 1.47 (d, 6H) | 460 | Method 14 and Method 91 |
| **120** | [4-[[4-(2-Methyl-3- propan-2-yl-3H- imidazol-4- yl)pyrimidin-2- yl]amino]phenyl]-[(3S)-3-(methyl- propyl- amino)pyrrolidin-1- yl]methanone | 9.24 (s, 1H), 8.40 (d, 1H), 7.73 (d, 2H), 7.46 (d, 2H), 7.37 (s, 1H), 7.04 (d, 1H), 5.61 (septet, 1H), 3.65 - 3.57 (m, 2H), 3.49 - 3.44 (m, 1H), 3.31 (dd, 1H), 3.03 (quintet, 1H), 2.49 (s, 3H), 2.38 - 2.28 (m, 2H), 2.18 (s, 3H), 2.06 - 1.99 (m, 1H), 1.81 - 1.74 (m, 1H), 1.47 (d, 6H), 1.45 - 1.39 (m, 2H), 0.85 (t, 3H) | 462 | Method 14 and Method 92 |
| **121** | (3-(3S)- Diethylaminopyrrolidi n-1-yl)-[4-[[4-(2- methyl-3-propan-2-yl- 3H-imidazol-4- yl)pyrimidin-2- yl]amino]phenyl]metha none | 9.24 (s, 1H), 8.40 (d, 1H), 7.73 (d, 2H), 7.46 (d, 2H), 7.37 (s, 1H), 7.04 (d, 1H), 5.61 (septet, 1H), 3.66 - 3.56 (m, 2H), 3.49 - 3.43 (m, 1H), 3.30 - 3.23 (m, 2H), 2.60 - 2.52 (m, 4H), 2.49 (s, 3H), 2.06 - 1.99 (m, 1H), 1.80 - 1.73 (m, 1H), 1.47 (d, 6H), 0.96 (t, 6H) | 462 | Method 14 and Method 94 |
| **122** | [(3S)-3-(Azepan-l- yl)pyrrolidin-1-yl]-[4-[[4-(2-methyl-3- propan-2-yl-3H- imidazol-4- yl)pyrimidin-2- yl]amino]phenyl]metha none | 9.21 (s, 1H), 8.40 (d, 1H), 7.72 (d, 2H), 7.45 (d, 2H), 7.37 (s, 1H), 7.04 (d, 1H), 5.61 (septet, 1H), 3.66 - 3.57 (m, 2H), 3.48 - 3.43 (m, 1H), 3.32 - 3.23 (m, 2H), 2.70 - 2.60 (m, 5H), 2.08 - 2.01 (m, 1H), 1.82 - 1.73 (m, 1H), 1.63 - 1.52 (m, 6H), 1.47 (d, 6H) (Methyl under DMSO) | 488 | Method 14 and Method 95 |
| **123** | [(3S)-3-(2- Methoxyethyl-methyl- amino)pyrrolidin-1- yl]-[4-[[4-(2-methyl-3- propan-2-yl-3H- imidazol-4- yl)pyrimidin-2- yl]amino]phenyl]metha none | 9.20 (s, 1H), 8.40 (d, 1H), 7.72 (d, 2H), 7.46 (d, 2H), 7.36 (s, 1H), 7.04 (d, 1H), 5.61 (septet, 1H), 3.66 - 3.57 (m, 2H), 3.49 - 3.45 (m, 2H), 3.42 (t, 3H), 3.32 (dd, 1H), 3.23 (s, 3H), 3.11 (quintet, 1H), 2.63 - 2.54 (m, 3H), 2.49 (s, 3H), 2.06 - 2.00 (m, 1H), 1.82 - 1.74 (m, 1H), 1.47 (d, 6H) | 478 | Method 14 and Method 96 |
| **124** | [(3S)-3-(Methyl-(2- methylpropyl)amino)p yrrolidin-1-yl]-[4-[[4-(2-methyl-3-propan-2- yl-3H-imidazol-4- yl)pyrimidin-2- yl]amino]phenyl]metha none | (400.132 MHz, CDC1₃) 8.37 (d, 1H), 7.65 (d, 2H), 7.55 - 7.49 (m, 3H), 7.42 - 7.38 (m, 1H), 6.95 (d, 1H), 5.67 (septet, 1H), 3.90 - 3.77 (m, 1H), 3.64 - 3.30 (m, 3H), 3.05 - 2.82 (m, 1H), 2.59 (s, 3H), 2.23 - 1.96 (m, 6H), 1.90 - 1.63 (m, 2H), 1.53 (d, 6H), 0.93 - 0.82 (m, 6H) | 476 | Method 14 and Method 97 |
| **125** | [4-[[4-(2-Methyl-3- propan-2-yl-3H- imidazol-4- yl)pyrimidin-2- yl]amino]phenyl]-[(3S)-3-(propan-2- ylamino)pyrrolidin-1- yl]methanone | (400.132 MHz, CDC1₃) 8.37 (d, 1H), 7.65 (d, 2H), 7.54 (d, 2H), 7.38 (s, 1H), 7.28 (s, 1H), 6.95 (d, 1H), 5.65 (septet, 1H), 3.99 - 3.21 (m, 4H), 3.00 - 2.75 (m, 1H), 2.59 (s, 3H), 2.27 - 1.68 (m, 4H), 1.53 (d, 6H), 1.15 - 0.99 (m, 6H) | 448 | Method 14 and Method 98 |
| **126** | [4-(2-Methoxyethyl)- 1,4-diazepan-1-yl]-[4-[[4-(2-methyl-3- propan-2-yl-3H- imidazol-4- yl)pyrimidin-2- yl]amino]phenyl]metha none | (400.132 MHz, CDC1₃) 8.37 (d, 1H), 7.64 (d, 2H), 7.39 (d, 2H), 7.37 (d, 2H), 6.95 (d, 1H), 5.65 (septet, 1H), 3.83 - 3.71 (m, 2H), 3.62 - 3.42 (m, 4H), 3.36 - 3.30 (m, 3H), 2.95 - 2.86 (m, 1H), 2.83 - 2.66 (m, 5H), 2.59 (s, 3H), 2.05 - 1.92 (m, 1H), 1.90 - 1.77 (m, 1H), 1.53 (d, 6H) | 478 | Method 14 and Method 103 |
| **127** | [4-[[5-Fluoro-4-(2- methyl-3-propan-2-yl- 3H-imidazol-4- yl)pyrimidin-2- yl]amino]phenyl]-[4-(2-methoxyethyl)-1,4-diazepan-1- yl]methanone | (400.132 MHz, CDC1₃) 8.30 (d, 1H), 7.60 (d, 2H), 7.58 (s, 1H), 7.39 (d, 2H), 7.31 (s, 1H), 5.57 (septet, 1H), 3.83 - 3.43 (m, 6H), 3.38 - 3.29 (m, 3H), 2.93 - 2.66 (m, 6H), 2.61 (s, 3H), 2.06 - 1.78 (m, 2H), 1.52 (d, 6H) | 496 | Method 2 and Method 103 |
| **128** | (4-Ethyl-1,4-diazepan- 1-yl)-[4-[[4-(2-methyl- 3-propan-2-yl-3H- imidazol-4- yl)pyrimidin-2- yl]amino]phenyl]metha none | (400.132 MHz, CDC1₃) 8.37 (d, 1H), 7.66 - 7.62 (m, 2H), 7.42 - 7.37 (m, 3H), 7.22 (s, 1H), 6.95 (d, 1H), 5.65 (septet, 1H), 3.82-3.74 (m, 2H), 3.59 - 3.49 (m, 2H), 2.83 -2.79 (m, 1H), 2.72-2.51 (m, 7H), 2.02-1.93 (m, 1H), 1.87-1.77 (m, 2H), 1.53 (d, 6H),1.12-1.02 (m, 3H) | 448 | Method 14 and Method 104 |
| **129** | (4-Ethyl-1,4-diazepan- 1-yl)-[4-[[5-fluoro-4-(2-methyl-3-propan-2- yl-3H-imidazol-4- yl)pyrimidin-2- yl]amino]phenyl]metha none | (400.132 MHz, CDC1₃) 8.30 (d, 1H), 7.61-7.57 (m, 3H), 7.41-7.38 (m, 2H), 7.19 (s, 1H), 5.57 (septet, 1H), 3.8-3.73 (m, 2H), 3.59 -3.49 (m, 2H), 2.83-2.78 (m, 1H), 2.72- 2.49 (m, 7H), 2.01-1.94 (m, 1H), 1.87-1.79 (m, 2H), 1.53 (d, 6H), 1.12-1.01 (m, 3H) | 466 | Method 2 and Method 104 |
| **130** | [4-[[5-Chloro-4-(2- methyl-3-propan-2-yl- 3H-imidazol-4- yl)pyrimidin-2- yl]amino]phenyl]-((3S)-3- methylaminopyrrolidin -1-yl)methanone | (400.132 MHz, CDCI₃) 8.45 (s, 1H), 7.61 (d, 2H), 7.54 - 7.52 (m, 3H), 7.38 (s, 1H), 4.96 (septet, 1H), 3.89 - 3.62 (m, 2H), 3.56 - 3.19 (m, 2H), 2.59 (s, 3H), 2.48 - 2.38 (m, 3H), 2.24 - 2.00 (m, 1H), 1.85 - 1.76 (m, 1H), 1.48 (d, 6H) | 454 | Method 5 in WO05/07546 1 and Method 90 |
| **131** | [(1S,4S)-2,5- Diazabicyclo[2.2.1]he pt-5-yl]-[4-[[5-fluoro- 4-(2-methyl-3-propan- 2-yl-3H-imidazol-4- yl)pyrimidin-2- yl]amino]phenyl]metha none | (400.132 MHz, CDC1₃) 8.31 (d, 1H), 7.64-7.49 (m, 5H), 7.17 (s, 1H), 5.56 (septet, 1H), 4.94-4.40 (m, 1H), 3.87-3.57 (m, 2H), 3.43- 3.03 (m, 3H), 2.62 (s, 3H), 1.91- 1.68 (m, 2H), 1.54 (d, 6H) | 436 | Method 2 and Method 105 |
| **132** | [4-[[5-Fluoro-4-(2- methyl-3-propan-2-yl- 3H-imidazol-4- yl)pyrimidin-2- yl]amino]phenyl]-[(3R)-3- methylaminopyrrolidin -1-yl]methanone | (400.132 MHz, CDC1₃) 8.30 (d, 1H), 7.61-7.59 (m, 3H), 7.54 (d, 2H), 7.29 (s, 1H), 5.58 (septet, 1H), 3.91 - 3.62 (m, 2H), 3.58 - 3.42 (m, 1H), 3.39 - 3.20 (m, 2H), 2.61 (s, 3H), 2.48 - 2.39 (m, 3H), 2.22 - 2.00 (m, 1H), 1.86 - 1.74 (m, 2H), 1.53 (d, 6H) | 438 | Method 2 and Method 108 |
| **133** | [(3R)-3- Methylaminopyrrolidin -1-yl]-[4-[[4-(2- methyl-3-propan-2-yl- 3H-imidazol-4- yl)pyrimidin-2- yl]amino]phenyl]metha none | (400.132 MHz, CDC1₃) 8.37 (d, 1H), 7.65 (d, 2H), 7.54 (d, 2H), 7.44 (s, 1H), 7.38 (s, 1H), 6.95 (d, 1H), 5.66 (septet, 1H), 3.90 - 3.63 (m, 2H), 3.58 - 3.45 (m, 1H), 3.39 - 3.19 (m, 1H), 2.59 (s, 3H), 2.48 - 2.39 (m, 3H), 2.23 - 2.00 (m, 1H), 1.86 - 1.59 (m, 3H), 1.53 (d, 6H) | 420 | Method 14 and Method 108 |
| **134** | [4-[[5-Chloro-4-(2- methyl-3-propan-2-yl- 3H-imidazol-4- yl)pyrimidin-2- yl]amino]phenyl]-[(3R)-3- methylaminopyrrolidin -1-yl]methanone | (400.132 MHz, CDC1₃) 8.45 (s, 1H), 7.61 (d, 2H), 7.54 - 7.52 (m, 3H), 7.38 (s, 1H), 4.96 (septet, 1H), 3.89 - 3.62 (m, 2H), 3.56 - 3.19 (m, 2H), 2.59 (s, 3H), 2.48 - 2.38 (m, 3H), 2.24 - 2.00 (m, 1H), 1.85 - 1.76 (m, 1H), 1.48 (d, 6H) | 454 | Method 5 in WO05/07546 1 and Method 108 |

### Example 135

### 5-Fluoro-4-(1-isopropyl-2-methyl-1H-imidazol-5-yl)-N- {4-[(4-methylpiperazin-1 - yl)carbonyl]phenyl}pyrimidin-2-amine hydrochloride

5-Fluoro-4-(3-isopropyl-2-methyl-3H imidazol-4-yl)-pyrimidin-2-ylamine (Method 2, 64 mg, 0.272 mmol), 1-(4-bromobenzoyl)-4-methylpiperazine (Example 59 of WO 03/004472; 92 mg, 0.325 mmol), BINAP (51 mg, 0.082 mmol) and sodium tert-butoxide (31 mg, 0.323 mmol) were mixed in 1,4-dioxane (2.0 ml). The mixture was flushed with argon for 5 mins, then Pd(OAc)₂ (9.1 mg, 0.045 mmol) was added followed by another purge with argon. The reaction mixture was heated in a sealed tube at 110 °C for 30 mins in a microwave reactor. The solvent was evaporated *in vacuo* and the residue was partitioned between DCM and diluted NaHC0₃ (aq.). The aqueous phase was extracted with DCM and the combined organic phases were dried (Na₂S0₄), filtered and evaporated. The crude of the free base was purified using preparative HPLC, then dissolved in DCM and the HCI-adduct of the product was precipitated from the solution by addition of O.1M HC1 in ether (2 equiv. HCI). The solvent was evaporated and the residue was dissolved in water and freeze dried to give the title compound (43 mg, 36%) as a solid. NMR (D₂0) 8.54 (d, J=2.0 Hz, 1H), 7.79 (d, J=2.0 Hz, 1H), 7.63 (d, J=8.8 Hz, 2H), 7.48 (d, J=8.6 Hz, 2H), 5.39-5.26 (m, 1H), 4.29-3.83 (m, 2H), 3.79-3.33 (m, 4H), 3.32-3.09 (m, 2H), 2.95 (s, 3H), 2.79 (s, 3H), 1.52 (d, J=6.8 Hz, 6H); MS (ESI) m/z 437.

### Example 136

### 5-Fluoro-4-(1-isopropyl-2-methyl-1H-imidazol-5-yl)-N- {3-methoxy-4-[(4-methylpiperazin-1 - yl)carbonyl]phenyl}pyrimidin-2-amine hydrochloride

5-Fluoro-4-(3-isopropyl-2-methyl-3H imidazol-4-yl)-pyrimidin-2-ylamine (Method 2, 49.8 mg, 0.212 mmol), 1-(4-chloro-2-methoxybenzoyl)-4-methylpiperazine (Method 11, 45 mg, 0.167 mmol), caesium carbonate (110 mg, 0.338 mmol) were mixed in anhydrous 1,4-dioxane (2 ml) and the mixture was flushed with argon for 5 mins before Pd₂(dba)₃ (9.3 mg, 0.010 mmol) and Xantphos (11.3 mg, 0.024 mmol) were added. The mixture was flushed with argon, then heated in a sealed tube at 90°C overnight. The solvent was *removed in vacuo* and the residue was taken up in DCM and washed with diluted NaHC0₃ (aq.). The organic layer was dried (Na₂S0₄), filtered and evaporated. The crude of the free base was purified using preparative HPLC, then dissolved in DCM and the HCI-adduct of the product was precipitated from the solution by addition of 0.1 M HCI in ether (2 equiv. HCl). The solvent was evaporated and the residue was dissolved in water and freeze dried to give the title compound (60 mg, 53%) as a solid. NMR: 11.38 (br s, 1H), 10.06 (s, 1H), 8.85 (d, J=2.0 Hz, 1H), 8.12 (d, J--1.8 Hz, 1H), 7.49 (dd, J=8.3, 1.8 Hz, 1H), 7.39 (s, 1H), 7.20 (d, J=8.3 Hz, 1H), 5.30-5.15 (m, 1H), 4.58 (d, J=13.3 Hz, 1H), 3.81 (s, 3H), 3.57-3.13 (m, 6H), 3.13-2.87 (m, 2H), 2.82 (s, 3H), 2.79 (br s, 3H), 1.51 (d, J=7.0 Hz, 6H); m/z (ESI) 468.

### Examples 137-138

The following compounds were prepared by the procedure of Example 136 using the appropriate starting materials.

| **Ex** | **Compound** | **NMR** | **m/z** | **SM** |
|---|---|---|---|---|
| **137** | *N-{3-Chloro-4-[(4-* methylpiperazin-1-yl)carbonyl]phenyl}-5-fluoro-4-(1-isopropyl- 2-methyl-1H-imidazol-5-yl)pyrimidin-2-amine hydrochloride | 11.78 (brs, 1H), 10.31 (s, 1H), 8.88 (d, J=1.8 Hz, 1H), 8.12 (d, J=2.0 Hz, 1H), 7.94 (br s, 1H), 7.71 (dd, J=8.3, 2.0 Hz, 1H), 7.38 (br s, 1H), 5.30-5.17 (m, 1H), 4.57 (d, *J*=13.1 Hz, 1H), 4.20-2.89 (m, 8H), 2.83 (s, 3H), 2.76 (s, 3H), 1.51 (d, *J*=7.1 Hz, 6H) | 472 | Method 2 and 1-(2,4- dichlorobenz oyl)-4-methylpiperaz ine¹ |
| **138** | 5-{[5-Fluoro-4-(1-isopropyl-2-methyl-1H imidazol-5-yl)-pyrimidin-2- yl]amino}-2-[(4-methylpiperazi-1- yl)carbonyl] benzonitrile hydrochloride | 11.79 (br s, 1H), 10.54 (s, 1H), 8.91 (d, J=1.76 Hz, 1H), 8.29 (d, *J*=2.01 Hz, 1H), 8.11 (d, J=1.83 Hz, 1H), 8.02 (dd, J=8.53, 2.26 Hz, 1H), 7.62 (d, *J=8.53* Hz, 1H), 5.26 (s, 1H), 4.57 (br s, 1H), 3.75-2.95 (m, 8H), 2.82 (s, 3H), 2.77 (s, 3H), 1.52 (d, *J=7.03* Hz, 6H) | 463 | Method 2 and Method 13 |

| | | | | |
|---|---|---|---|---|
| Prasad, R.N., et al., J. Med. Chem. 1968, 6, 1144-1150 | | | | |

### Example 139

### 5-Fluoro-4-(1-isopropyl-2-methyl-1H imidazol-5-yl)-N [4-[(4-methylpiperazin-1-yl)carbonyl]-3-(methylsulfonyl)phenyl]pyrimidin-2-amine hydrochloride

Anhydrous 1,4-dioxane (2 ml) was added to 5-fluoro-4-(3-isopropyl-2-methyl-3H-imidazol-4-yl)-pyrimidin-2-ylamine (Method 2, 52 mg, 0.22 mmol), 1-[4-bromo-2-(methylsulfonyl)benzoyl]-4-methylpiperazine (Bruce, R.B., et al. J. Med. Chem. 1968, 5, 1031-1034; 71.0 mg, 0.197 mmol) and sodium tert-butoxide (30.9 mg, 0.32 mmol). The mixture was purged with argon and Pd(OAc)₂ (2 mg, 0.009 mmol) and Pd(t-Bu₃P)₂ (6.1 mg, 0.012 mmol) were added followed by another argon purge. The mixture was heated in a sealed tube at 120°C. After stirring overnight Pd(t-Bu₃P)₂ (12.4 mg, 0.024 mmol) was added and after another 24 hr the following reagents were added; CsCOₛ (107 mg, 0.33 mmol), X-Phos (11.3 mg, 0.024 mmol) and Pd₂(dba)₃ (11.1 mg, 0.012 mmol). The resulting mixture was heated in an oil bath for 90°C for 20 hours. The mixture was filtered through diatomaceous earth and washed with EtOAc. The organic phase was washed with water, dried (Na₂S0₄), filtered and evaporated *in vacuo.* The residue was purified by flash chromatography (MeCN/5% TEA in MeCN gradient; 0 to 5% TEA in MeCN). The product-containing fractions were pooled together and evaporated *in vacuo.* The residue was dissolved in DCM and the organic phase was washed with EDTA (aq.) at pH 1. The EDTA (aq.) phase was neutralized (pH 7) with NaHCO₃ (aq.) and the product was extracted with DCM. The organic phase was dried (Na₂SO₄), filtered and evaporated *in vacuo.* The residue was dissolved in DCM/ether (1:l, 10 ml) and the title compound precipitated by dropwise addition of 1M HCI in ether (2.0 equiv.). The precipitate was collected by filtration, rinsed with DCM, dissolved in water and freeze dried to give the title compound (96 mg, 74 %) as a yellow solid. NMR: 11.47-11.25 (m, 1H), 10.41 (s, 1H), 8.90 (d, *J*=1.5 Hz, 1H), 8.30 (s, 1H), 8.21 (d, *J*=8.5 Hz, 1H), 8.12 (s, 1H), 7.52 (d, *J*=8.0 Hz, 1H), 5.29-5.12 (m, 1H), 4.64-4.48 (m, 1H), 3.62-3.04 (m, 6H), 2.84-2.71 (m, 7H), 2.76 (br s, 4H), 1.52 (d, *J*=7.0 Hz, 6H); MS (ESI) m/z 516.

### Example 140

### 5-{[4-(1-Isopropyl-2-methyl-1H-imidazol-5-yl)pyrimidin-2-yl]amino}-2-[(4-methyl piperazin-1-yl)carbonyl]benzonitrile

4-(3-Isopropyl-2-methyl-3H imidazol-4-yl)-pyrimidin-2-ylamine (Method 14, 0.20g, 0.92 mmol), PdOAc₂ (16 mg, 0.068 mmol), Xantphos (60 mg, 0.10 mmol), caesium carbonate (0.42 g, 1.3 mmol) and 5-chloro-2-(4-methyl-piperazine-1-carbonyl)-benzonitrile (Method 13, 0.32 g, 1.20 mmol) were added to dioxane (7 ml) under a inert atmosphere and heated at 150 °C in the microwave for 1 hour. Purification by flash chromatography on silica using 0-10% MeOH in DCM as eluent gave the desired compound as a yellow foam. Further purification by the RPHPLC gave the desired compound as a colourless foam (204 mg, 50%). NMR (400.132 MHz): 9.95 (s, 1H), 8.49 (d, 1H), 8.26 (s, 1H), 8.01 (d, 1H), 7.48 (d, 1H), 7.46 (s, 1H), 7.17 (d, 1H), 5.60 (septet, 1H), 3.70 - 3.58 (m, 2H), 3.32 - 3.22 (m, 2H), 2.51 (s, 3H), 2.42 - 2.27 (m, 4H), 2.20 (s, 3H), 1.48 (d, 6H); m/z 445.

### Examples 141-144

The following compounds were prepared by the procedure of Example 137 using the appropriate starting materials.

| **Ex** | **Compound** | **NMR** | **m/z** | **SM** |
|---|---|---|---|---|
| **141** | 5-{[4-(1-Isopropyl-2-methyl-1H-imidazol-5-yl)pyrimidin-2-yl]amino}-2-(morpholin-4-ylcarbonyl) benzonitrile | (400.132 MHz): 9.96 (s, 1H), 8.50 (d, 1H), 8.27 (s, 1H), 8.02 (d, 1H), 7.52 (d, 1H), 7.47 (s, 1H), 7.18 (d, 1H), 5.59 (septet, 1H), 3.72 - 3.53 (m, 6H), 3.40 - 3.21 (m, 2H), 2.51 (s, 3H), 1.49 (d, 6H) | 432 | Method 14 and Method 16 |
| **142** | 5-{[5-Fluoro-4-(1-isopropyl-2-methyl-1H-imidazol-5-yl) pyrimidin-2-yl]amino}-2-(morpholin-4- ylcarbonyl)benzonitrile | (400.132 MHz): 10.06 (s, 1H), 8.65 (d, 1H), 8.21 (s, 1H), 7.98 (d, 1H), 7.52 (d, 1H), 7.40 (d, 1H), 5.37 (s, 1H), 3.76 - 3.50 (m, 6H), 3.37 - 3.21 (m, 2H), 2.54 (s, 3H), 1.47 (d, 6H) | 450 | Method 2 and Method 16 |
| **143** | 5-({4-[1-Isopropyl-2-(methoxymethyl)-1*H*-imidazol-5-yl] pyrimidin-2-yl}amino)-2-(morpholin-4-ylcarbonyl)benzonitrile | (400.132 MHz, CDC1₃) 8.46 (d, 1H), 8.23 (s, 1H), 7.82 - 7.80 (m, 2H), 7.45 (s, 1H), 7.41 (d, 1H), 7.05 (d, 1H), 5.42 (septet, 1H), 4.66 (s, 2H), 3.89 - 3.67 (m, 6H), 3.48 - 3.36 (m, 5H), 1.59 (d, 6H) | 462 | Method 3 and Method 16 |
| **144** | 5-({4-[1-Isopropyl-2-(methoxymethyl)-1*H*-imidazol-5-yl]pyrimidin-2-yl}amino)-2-[(4-methylpiperazin-1-yl)carbonyl] benzonitrile | (400.132 MHz, CDCl₃) 8.45 (d, 1H), 8.21 (d, 1H), 7.78 (dd, 1H), 7.71 (s, 1H), 7.45 (s, 1H), 7.39 (d, 1H), 7.04 (d, 1H), 5.43 (septet, 1H), 4.66 (s, 2H), 3.90 - 3.80 (m, 2H), 3.43 - 3.36 (m, 5H), 2.57 - 2.49 (m, 2H), 2.46 - 2.38 (m, 2H), 2.33 (s, 3H), 1.60 (d, 6H) | 475 | Method 3 and Method 13 |

### Example 145

### [(2S)-1-(4-{[4-(1-Isopropyl-2-methyl-1H-imidazol-5-yl)pyrimidin-2-yl]amino}benzoyl)pyrrolidin-2-yl]methanol

To a stirred suspension of 4- {[4-(1-isopropyl-2-methyl-1*H*-imidazol-5-yl)pyrimidin-2-yl]amino}benzoic acid sodium salt (Method 50; 240 mg) in DMF (8 ml) was added HBTU (257 mg). The mixture was stirred at ambient temperature for 20 minutes, then L-prolinol (81 1 mg) was added. The mixture was stirred at room temp for 18 hours, then diluted with EtOAc (80 ml), washed with 2N NaOH (80 ml) then the aqueous layer was extracted with further EtOAc (80 ml). Organics were concentrated in vacuo, then the residue was purified by reverse phase preparative HPLC. Fractions containing product were poured onto a 10g SCX-2 column, washed with MeOH, then eluted with methanolic ammonia. Evaporation of the basic eluent afforded the title compound as a white solid (177 mg, 63%). NMR (300.074 MHz) 0 9.65 (s, 1H), 8.42 (d, 1H), 7.74 (d, 2H), 7.46 - 7.43 (m, 3H), 7.08 (d, 1H), 5.71 - 5.64 (m, 1H), 4.75 (br s, 1H), 4.16 - 4.08 (m, 1H), 3.56 - 3.34 (m, 4H), 2.50 (s, 3H), 1.94 - 1.82 (m, 3H), 1.75 - 1.64 (m, 1H), 1.46 (d, 6H); m/z 421.

### Examples 146-174

The following compounds were prepared by the procedure of Example 145 using the appropriate acid and amine starting materials.

| **Ex** | **Compound** | **NMR** | **m/z** | **SM** |
|---|---|---|---|---|
| **146** | l-(4-{[4-(l-Isopropyl- 2-methyl-1H-imidazol- 5-yl)pyrimidin-2- yl]amino}benzoyl)pipe ridin-4-ol | (399.902 MHz) □9.22 (s, 1H), 8.40 (d, 1H), 7.72 (d, 2H), 7.38 (s, 1H), 7.31 (d, 2H), 7.03 (d, 1H), 5.66 - 5.57 (m, 1H), 4.38 (br s, 1H), 3.81 - 3.75 (m, 3H), 3.25 - 3.17 (m, 2H), 2.48 (s, 3H), 1.79 - 1.73 (m, 2H), 1.46 (d, 6H), 1.43 - 1.35 (m, 2H); m/z 421. | 421 1 | Method 50 |
| **147** | 4-(1-Isopropyl-2- methyl-1H-imidazol-5-yl)-N-(4-{[3-(methylsulfonyl)pyrroli din-1- yl]carbonyl}phenyl)py rimidin-2-amine | (300.074 MHz) 09.70 (s, 1H), 8.43 (d, 1H), 7.77 (d, 2H), 7.49 (d, 2H), 7.45 (s, 1H), 7.10 (d, 1H), 5.72 - 5.63 (m, 1H), 4.02 - 3.96 (m, 1H), 3.89 - 3.82 (m, 2H), 3.71 - 3.54 (m, 2H), 3.03 (s, 3H), 3.03 (s, 3H), 2.32 - 2.24 (m, 2H), 1.46 (d, 6H) | 469 | Method 50 |
| **148** | [(2R)-1-(4-{[4-(1- Isopropyl-2-methyl-1H-imidazol-5-yl)pyrimidin-2- yl]amino}benzoyl)pyrr olidin-2-yl]methanol | (300.074 MHz) 9.65 (s, 1H), 8.42 (d, 1H), 7.74 (d, 2H), 7.46 - 7.43 (m, 3H), 7.08 (d, 1H), 5.71 - 5.64 (m, 1H), 4.75 (br s, 1H), 4.16 - 4.08 (m, 1H), 3.56 - 3.34 (m, 4H), 2.50 (s, 3H), 1.94 - 1.82 (m, 3H), 1.75 - 1.64 (m, 1H), 1.46 (d, 6H) | 421 | Method 50 |
| **149** | (3S)-1-(4-{[4-(1- Isopropyl-2-methyl-1H-imidazol-5-yl)pyrimidin-2- yl]amino}benzoyl)pipe ridin-3-ol | (300.074 MHz) □9.64 (s, 1H), 8.41 (d, 1H), 7.73 (d, 2H), 7.43 (s, 1H), 7.33 (d, 2H), 7.08 (d, 1H), 5.71 - 5.64 (m, 1H), 4.86 (s, 1H), 3.75- 3.60 (m, 1H), 3.52 - 3.45 (m, 1H), 3.18 - 3.07 (m, 2H), 2.96 - 2.83 (m, 1H), 2.49 (s, 3H), 1.89 - 1.80 (m, 1H), 1.73 - 1.66 (m, 1H), 1.45 (d, 6H), 1.41 - 1.35 (m, 2H) | 421 1 | Method 50 |
| **150** | (3R)-1-(4-{[4-(1- Isopropyl-2-methyl-1H-imidazol-5-yl)pyrimidin-2- yl]amino}benzoyl)pipe ridin-3-ol | (300.074 MHz) 9.64 (s, 1H), 8.41 (d, 1H), 7.73 (d, 2H), 7.43 (s, 1H), 7.33 (d, 2H), 7.08 (d, 1H), 5.71 - 5.64 (m, 1H), 4.86 (s, 1H), 3.75- 3.60 (m, 1H), 3.52 - 3.45 (m, 1H), 3.18 - 3.07 (m, 2H), 2.96 - 2.83 (m, 1H), 2.49 (s, 3H), 1.89 - 1.80 (m, 1H), 1.73 - 1.66 (m, 1H), 1.45 (d, 6H), 1.41 - 1.35 (m, 2H) | 421 | Method 50 |
| **151** | [1-(4-{[4-(1- Isopropyl-2-methyl-1H-imidazol-5-yl)pyrimidin-2- yl]amino}benzoyl)pipe - ridin-2-yl]methanol | (300.074 MHz) □9.61 (s,1H), 8.40 (d, 1H), 7.71 (d, 2H), 7.43 (s, 1H), 7.32 (d, 2H), 7.07 (d, 1H), 5.71 - 5.65 (m, 1H), 4.73 (br s, 1H), 4.30 3.90 (m, 2H), 3.64 - 3.48 (m, 2H), 2.98 - 2.84 (m, 1H), 2.49 (s, 3H), 1.74 - 1.50 (m, 6H), 1.44 (d, 6H) | 435 | Method 50 |
| **152** | N-(4-{[(trans)-2,5- Dimethylpiperazin-1- yl]carbonyl}phenyl)-4-(1-isopropyl-2-methyl-1H-imidazol-5-yl)pyrimidin-2-amine | (300.074 MHz) □9.67 (s, 1H), 8.43 (d, 1H), 7.76 (d, 2H), 7.44 (s, 1H), 7.33 (d, 2H), 7.09 (d, 1H), 5.72 - 5.62 (m, 1H), 3.49 - 3.33 (m, 4H), 2.49 (s, 3H), 2.08 - 2.04 (m, 1H), 1.97 - 1.95 (m, 1H), 1.46 (d, 6H), 1.19 - 1.05 (m, 6H) | 476 | Method 50 |
| **153** | 4-(1-Isopropyl-2- methyl-1H-imidazol-5-yl)-N-{4-[(4-methyl- 1,4-diazepan-1- yl)carbonyl]phenyl}py rimidin-2-amine | (300.074 MHz) □9.63 (s, 1H), 8.41 (d, 1H), 7.73 (d, 2H), 7.42 (s, 1H), 7.32 (d, 2H), 7.07 (d, 1H), 5.73 - 5.64 (m, 1H), 3.66 - 3.39 (m, 4H), 2.68 (s, 3H), 2.64 - 2.56 (m, 1H), 2.49 (s, 3H), 2.29 - 2.22 (m, 3H), 1.86 - 1.71 (m, 2H), 1.45 (d, 6H) | 448 | Method 50 |
| **154** | l-(4-{[4-(l-Isopropyl- 2-methyl-1H-imidazol- 5-yl)pyrimidin-2- yl]amino}benzoyl)azet idin-3-ol | (300.074 MHz) 9.74 (s, 1H), 8.43 (d, 1H), 7.77 (d, 2H), 7.57 (d, 2H), 7.43 (s, 1H), 7.10 (d, 1H), 5.75 - 5.62 (m, 1H), 4.55 - 4.39 (m, 2H), 4.33 - 4.17 (m, 1H), 4.10 - 3.95 (m, 1H), 3.85 - 3.72 (m, 1H), 2.69 (s, 3H), 1.46 (d, 6H) | 393 | Method 50 |
| **155** | 4-(1-Isopropyl-2- methyl-1H-imidazol-5-yl)-N-{4-[(4- pyrrolidin-1- ylpiperidin-1- yl)carbonyl]phenyl}py rimidin-2-amine | (399.902 MHz) □9.22 (s, 1H), 8.39 (d, 1H), 7.72 (d, 2H), 7.37 (s, 1H), 7.32 (d, 2H), 7.03 (d, 1H), 5.65 - 5.57 (m, 1H), 3.98 - 3.91 (m, 2H), 3.11 - 3.04 (m, 2H), 2.55 - 2.50 (m, 4H), 2.49 (s, 3H), 2.38 - 2.30 (m, 1H), 1.85 - 1.79 (m, 2H), 1.70 - 1.66 (m, 4H), 1.47 (d, 6H), 1.44 - 1.37 (m, 2H) | 474 | Method 50 |
| **156** | 4-(1-Isopropyl-2- methyl-1H-imidazol-5-yl)-N-(4-{[4-(4-methylpiperazin-1- yl)piperidin-1- yl]carbonyl}phenyl)py rimidin-2-amine | (399.902 MHz) □9.22 (s, 1H), 8.39 (d, 1H), 7.72 (d, 2H), 7.37 (s, 1H), 7.32 (d, 2H), 7.03 (d, 1H), 5.63 - 5.57 (m, 1H), 4.09 - 4.03 (m, 2H), 2.94 - 2.90 (m, 2H), 2.52 - 2.50 (m, 4H), 2.49 (s, 3H), 2.46 - 2.42 (m, 1H), 2.34 - 2.31 (m, 4H), 2.16 (s, 3H), 1.81 - 1.75 (m, 2H), 1.47 (d, 6H), 1.43 - 1.34 (m, 2H) | 503 | Method 50 |
| **157** | N-[4-({4-[2-(Dimethylamino)ethyl] piperazin-1- yl}carbonyl)phenyl]-4-(1-isopropyl-2-methyl-1H-imidazol-5-yl)pyrimidin-2-amine | (399.902 MHz) □9.26 (s, 1H), 8.41 (d, 1H), 7.74 (d, 2H), 7.38 (s, 1H), 7.34 (d, 2H), 7.04 (d, 1H), 5.66 - 5.58 (m, 1H), 3.54 - 3.47 (m, 4H), 3.19 - 3.07 (m, 4H), 2.74 - 2.70 (m, 2H), 2.57 - 2.49 (m, 2H), 2.47 (s, 3H), 2.23 (s, 6H), 1.47 (d, 6H) | 477 | Method 50 |
| **158** | N-{4-[(1,1- Dioxidothiomorpholin -4- yl)carbonyl]phenyl}-4-(1-isopropyl-2-methyl-1H-imidazol-5-yl)pyrimidin-2-amine | (399.902 MHz) □9.31 (s, 1H), 8.40 (d, 1H), 7.76 (d, 2H), 7.43 (d, 2H), 7.37 (s, 1H), 7.04 (d, 1H), 5.63 - 5.56 (m, 1H), 3.95 - 3.89 (m, 4H), 3.21 - 3.16 (m, 4H), 2.93 (s, 3H), 1.47 (d, 6H) | 413 | Method 50 |
| **159** | N-{4-[(4- Cyclopropylpiperazin- 1-yl)carbonyl]phenyl}- 4-(1-isopropyl-2- methyl-1H-imidazol-5-yl)pyrimidin-2-amine | (400.132 MHz) □9.73 (s, 1H), 8.44 (d, 1H), 7.77 (d, 2H), 7.45 (s, 1H), 7.35 (d, 2H), 7.11 (d, 1H), 5.74 - 5.67 (m, 1H), 3.53 - 3.39 (m, 8H), 2.51 (s, 3H), 1.68 - 1.63 (m, 1H), 1.46 (d, 6H), 0.44 - 0.42 (m, 2H), 0.34 - 0.32 (m, 2H) | 446 | Method 50 |
| **160** | 4-(1-Isopropyl-2- methyl-1H-imidazol-5-yl)-N-(4-{[4-(2- methoxyethyl)piperazi n-1- yl]carbonyl}phenyl)py rimidin-2-amine | (400.132 MHz) □9.72 (s, 1H), 8.44 (d, 1H), 7.76 (d, 2H), 7.45 (s, 1H), 7.34 (d, 2H), 7.10 (d, 1H), 5.73 - 5.66 (m, 1H), 3.55 - 3.31 (m, 12H), 3.23 (s, 3H), 2.44 (s, 3H), 1.46 (d, 6H) | 464 | Method 50 |
| **161** | 4-(1-Isopropyl-2- methyl-1H-imidazol-5-yl)-N-(4-{[4-(2- pyrrolidin-1- ylethyl)piperazin-1- yl]carbonyl}phenyl)py rimidin-2-amine | (400.132 MHz) □9.72 (s, 1H), 8.44 (d, 1H), 7.76 (d, 2H), 7.45 (s, 1H), 7.34 (d, 2H), 7.10 (d, 1H), 5.74 - 5.67 (m, 1H), 3.55 - 3.34 (m, 12H), 2.62 - 2.49 (m, 4H), 2.44 (s, 3H), 1.69 - 1.61 (m, 4H), 1.46 (d, 6H) | 503 | Method 50 |
| **162** | N-{4-[(4- Cyclopropylpiperazin- 1-yl)carbonyl]phenyl}- 5-fluoro-4-(1- isopropyl-2-methyl-1H-imidazol-5-yl)pyrimidin-2-amine | (400.132 MHz) 9.82 (s, 1H), 8.60 (d, 1H), 7.72 (d, 2H), 7.38 (d, 1H), 7.35 (d, 2H), 5.48 - 5.40 (m, 1H), 3.52 - 3.38 (m, 8H), 2.50 (s, 3H), 1.69 - 1.63 (m, 1H), 1.45 (d, 6H), 0.45 - 0.41 (m, 2H), 0.35 - 0.31 (m, 2H) | 464 | Method 52 |
| **163** | N-(4-{[trans-2,5- Dimethylpiperazin-1- yl]carbonyl}phenyl)-5- fluoro-4-(1-isopropyl- 2-methyl-1H-imidazol- 5-yl)pyrimidin-2-amine | 9.76 (s, 1H), 8.58 (d, 1H), 7.72 (d, 2H), 7.37 - 7.29 (m, 3H), 5.46 - 5.38 (m, 1H), 4.06 (s, 1H), 3.48 - 3.39 (m, 2H), 3.28 - 3.25 (m, 1H), 2.88-2.95 (m, 1H), 2.51 (s, 3H), 2.05 (s, 1H), 1.96 (s, 1H), 1.44 (d, 6H), 1.18 - 1.14 (m, 3H), 1.10- 1.06 (m, 3H) | 494 (+ MeC N) | Method 52 |
| **164** | 5-Fluoro-4-(1- isopropyl-2-methyl-1H-imidazol-5-yl)-N-(4-{[4-(4-methylpiperazin-1- yl)piperidin-1- yl]carbonyl}phenyl)py rimidin-2-amine | 9.74 (s, 1H), 8.57 (d, 1H), 7.69 (d, 2H), 7.36 (d, 1H), 7.32 (d, 2H), 5.47 - 5.35 (m, 1H), 2.93 - 2.81 (m, 2H), 2.52 (s, 3H), 2.47 - 2.38 (m, 6H), 2.31 - 2.25 (m, 4H), 2.12 (s, 3H), 1.79 - 1.71 (m, 2H), 1.44 (d, 6H), 1.39 - 1.27 (m, 3H) | 521 1 | Method 52 |
| **165** | N-[4-({4-[2-(Dimethylamino)ethyl] piperazin-1- yl}carbonyl)phenyl]-5- fluoro-4-(1-isopropyl- 2-methyl-1H-imidazol- 5-yl)pyrimidin-2-amine | 9.75 (s, 1H), 8.57 (d, 1H), 7.70 (d, 2H), 7.36 (d, 1H), 7.32 (d, 2H), 5.47 - 5.37 (m, 1H), 3.50 - 3.43 (m, 4H), 2.52 (s, 3H), 2.44 - 2.30 (m, 8H), 2.12 (s, 6H), 1.44 (d, 6H) | 495 | Method 52 |
| **166** | N-{4-[(1,1- Dioxidothiomorpholin -4- yl)carbonyl]phenyl}-5- fluoro-4-(1-isopropyl- 2-methyl-1H-imidazol- 5-yl)pyrimidin-2-amine | 9.80 (s, 1H), 8.58 (d, 1H), 7.73 (d, 2H), 7.44 (d, 2H), 7.37 (d, 1H), 5.48 - 5.37 (m, 1H), 3.90 - 3.84 (m, 4H), 3.27 - 3.21 (m, 4H), 2.52 (s, 3H), 1.45 (d, 6H) | 473 | Method 52 |
| **167** | 5-Fluoro-4-(1- isopropyl-2-methyl-1H-imidazol-5-yl)-N- {4-[(4-methyl-l,4- diazepan-1- yl)carbonyl]phenyl}py rimidin-2-amine | 9.73 (s, 1H), 8.57 (s, 1H), 7.68 (d, 2H), 7.38 - 7.29 (m, 3H), 5.48 - 5.38 (m, 1H), 3.61 - 3.36 (m, 4H), 2.72 - 2.61 (m, 2H), 2.50 (s, 3H), 2.42 - 2.34 (m, 2H), 2.25 (s, 3H), 1.85 - 1.69 (m, 2H), 1.43 (d, 6H) | 452 | Method 52 |
| **168** | [(2S)-l-(4-{[5-Fluoro- 4-(1-isopropyl-2- methyl-1H-imidazol-5-yl)pyrimidin-2- yl]amino}benzoyl)pyrr olidin-2-yl]methanol | 9.75 (s, 1H), 8.57 (d, 1H), 7.69 (d, 2H), 7.44 (d, 2H), 7.37 (d, 1H), 5.48 - 5.39 (m, 1H), 4.74 (s, 1H), 4.16 - 4.07 (m, 1H), 3.60 - 3.33 (m, 4H), 2.52 (s, 3H), 1.96 - 1.83 (m, 3H), 1.75 - 1.63 (m, 1H), 1.44 (d, 6H) | 439 | Method 52 |
| **169** | [1-(4-{[5-Fluoro-4-(1-isopropyl-2-methyl-1H-imidazol-5-yl)pyrimidin-2- yl]amino}benzoyl)pipe ridin-2-yl]methanol | 9.71 (s, 1H), 8.56 (s, 1H), 7.67 (d, 2H), 7.37 - 7.30 (m, 3H), 5.48 - 5.39 (m, 1H), 4.73 (s, 1H), 3.63 - 3.46 (m, 2H), 3.41 - 3.32 (m, 2H), 2.98 - 2.85 (m, 1H), 2.52 (s, 3H), 1.75 - 1.45 (m, 6H), 1.43 (d, 6H) | 453 | Method 52 |
| **170** | [(2R)-1-(4-{[5-Fluoro-4-(1-isopropyl- 2-methyl-1H-imidazol- 5-yl)pyrimidin-2- yl]amino}benzoyl)pyrr olidin-2-yl]methanol | 9.75 (s, 1H), 8.57 (d, 1H), 7.69 (d, 2H), 7.44 (d, 2H), 7.37 (d, 1H), 5.48 - 5.39 (m, 1H), 4.74 (s, 1H), 4.16 - 4.07 (m, 1H), 3.60 - 3.33 (m, 4H), 2.52 (s, 3H), 1.96 - 1.83 (m, 3H), 1.75 - 1.63 (m, 1H), 1.44 (d, 6H) | 439 | Method 52 |
| **171** | 4-(1-Isopropyl-2- methyl-1H-imidazol-5-yl)-N-{4-[(4- isopropylpiperazin-1- yl)carbonyl]phenyl}py rimidin-2-amine | □9.67 (s, 1H), 8.43 (d, 1H), 7.76 (d, 2H), 7.44 (s, 1H), 7.35 (d, 2H), 7.10 (d, 1H), 5.74 - 5.64 (m, 1H), 3.54 - 3.43 (m, 4H), 3.41 - 3.36 (m, 1H), 2.51 (s, 3H), 2.47 - 2.43 (m, 4H), 1.47 (d, 6H), 0.97 (d, 6H) | 448 | Method 50 |
| **172** | N-(4-{[4-(Dimethylamino)piperi din-1- yl]carbonyl}phenyl)-4-(1-isopropyl-2-methyl-1H-imidazol-5-yl)pyrimidin-2-amine | 9.66 (s, 1H), 8.43 (d, 1H), 7.75 (d, 2H), 7.44 (s, 1H), 7.34 (d, 2H), 7.10 (d, 1H), 5.72 - 5.65 (m, 1H), 3.40 - 3.32 (m, 2H), 3.00 - 2.83 (m, 2H), 2.50 (s, 3H), 2.39 - 2.33 (m, 1H), 2.19 (s, 6H), 1.79 - 1.73 (m, 2H), 1.46 (d, 6H), 1.38 - 1.28 (m, 2H) | 448 | Method 50 |
| **173** | (3R)-1-(4-{[4-(1- Isopropyl-2-methyl-1H-imidazol-5-yl)pyrimidin-2- yl]amino}benzoyl)pyrr olidin-3-ol | 9.69 (s, 1H), 8.44 (d, 1H), 7.76 (d, 2H), 7.50 (d, 2H), 7.45 (s, 1H), 7.10 (d, 1H), 5 .72 - 5.67 (m, 1H), 4.99 - 4.90 (m, 1H), 4.34 - 4.23 (m, 1H), 3.66 - 3.55 (m, 2H), 3.53 - 3.44 (m, 1H), 2.51 (s, 3H), 1.98 - 1.88 (m, 1H), 1.85 - 1.77 (m, 1H), 1.48 (d, 6H) | 407 | Method 50 |
| **174** | (3S)-1-(4-{[4-(1- Isopropyl-2-methyl-1H-imidazol-5-yl)pyrimidin-2- yl]amino}benzoyl)pyrr olidin-3-ol | 9.69 (s, 1H), 8.44 (d, 1H), 7.76 (d, 2H), 7.50 (d, 2H), 7.45 (s, 1H), 7.10 (d, 1H), 5 .72 - 5.67 (m, 1H), 4.99 - 4.90 (m, 1H), 4.34 - 4.23 (m, 1H), 3.66 - 3.55 (m, 2H), 3.53 - 3.44 (m, 1H), 2.51 (s, 3H), 1.98 - 1.88 (m, 1H), 1.85 - 1.77 (m, 1H), 1.48 (d, 6H) | 407 | Method 50 |

### Example 175

### N-(4-{[4-(Aminomethyl)piperidin-1-yl]carbonyl}phenyl)-4-(1-isopropyl-2-methyl-1H-imidazol-5-yl)pyrimidin-2-amine

To a stirred suspension of 4- {[4-(1-isopropyl-2-methyl-1H-imidazol-5-yl)pyrimidin-2-yl]amino}benzoic acid sodium salt (Method 50; 240 mg) in DMF (8 ml) was added HBTU (257 mg). The mixture was stirred at ambient temperature for 20 minutes, then (tert-butoxycarbonyl-4-aminomethyl)piperidine (81 mg) was added. The mixture was stirred at room temp for 18 hours, then diluted with EtOAc (80 ml), washed with 2N NaOH (80 ml) then the aqueous layer was extracted with further EtOAc (80 ml). Organics were concentrated in vacuo, then the residue was purified by reverse phase preparative HPLC. Fractions containing product were poured onto a 10g SCX-2 column, washed with MeOH, then eluted with methanolic ammonia. Evaporation of the basic eluent afforded a white solid. The solid was dissolved in MeOH (1 ml) then a solution of hydrogen chloride in dioxane (4M, 4 ml) was added. The solution was stirred at ambient temperature for 3 hours, then concentrated in vacuo. The resulting solid was dissolved in MeOH and loaded onto a 10 g SCX-2 column. The column was washed with MeOH then eluted with methanolic ammonia. Evaporation of the basic eluent afforded the title compound as a white solid (85 mg, 30%). NMR (399.902 MHz) □8.46 (s, 1H), 8.41 (d, 1H), 7.79 (d, 2H), 7.74 (d, 2H), 7.37 (s, 1H), 7.05 (d, 1H), 5.65 - 5.58 8 (m, 1H), 3.20 - 3.10 (m, 4H), 3.09 - 2.95 (m, 2H), 2.58 - 2.51 (m, 2H), 2.50 (s, 3H), 1.72 - 1.63 (m, 3H), 1.49 (d, 6H), 1.21 - 1.11 (m, 2H); m/z 434.

### Examples 176-178

The following compounds were prepared by the procedure of Example 175 using the appropriate starting materials.

| **Ex** | **Compound** | **NMR** | **m/z** | **SM** |
|---|---|---|---|---|
| **176** | N-(4-{[(3R)-3- Aminopyrrolidin-1- yl]carbonyl}phenyl)-4-(1-isopropyl-2-methyl-1H-imidazol-5-yl)pyrimidin-2-amine | (399.902 MHz) □9.24 (s, 1H), 8.40 (d, 1H), 7.73 (d, 2H), 7.46 (d, 2H), 7.36 (s, 1H), 7.03 (d, 1H), 5.66 - 5.56 (m, 1H), 3.65 - 3.58 (m, 2H), 3.51 - 3.42 (m, 2H), 3.17 - 3.13 (m, 1H), 2.79 (br s, 2H), 2.49 (s, 3H), 2.03 - 1.95 (m, 1H), 1.66 - 1.59 (m, 1H), 1.47 (d, 6H) | 406 | Method 50 and (3R)-3-(tert- butoxycarbon ylamino) pyrrolidine |
| **177** | N-(4-{[(3S)-3- Aminopyrrolidin-1- yl]carbonyl}phenyl)-4-(1-isopropyl-2-methyl-1H-imidazol-5-yl)pyrimidin-2-amine | (399.902 MHz) □9.24 (s, 1H), 8.40 (d, 1H), 7.73 (d, 2H), 7.46 (d, 2H), 7.36 (s, 1H), 7.03 (d, 1H), 5.66 - 5.56 (m, 1H), 3.65 - 3.58 (m, 2H), 3.51 - 3.42 (m, 2H), 3.17 - 3.13 (m, 1H), 2.79 (br s, 2H), 2.49 (s, 3H), 2.03 - 1.95 (m, 1H), 1.66 - 1.59 (m, 1H), 1.47 (d, 6H) | 406 | Method 50 and (3S)-3-(tert- butoxycarbon ylamino) pyrrolidine |
| **178** | 4-(1-Isopropyl-2- methyl-1H-imidazol-5-yl)-N-(4-{[3-(methylamino)pyrrolidi n-1- yl]carbonyl}phenyl)py rimidin-2-amine | (300.074 MHz) □9.67 (s, 1H), 8.42 (d, 1H), 7.74 (d, 2H), 7.47 (d, 2H), 7.43 (s, 1H), 7.08 (d, 1H), 5.73 - 5.67 (m, 1H), 3.61 - 3.52 (m, 2H), 3.28 - 3.07 (m, 3H), 2.53 (s, 3H), 2.33 - 2.16 (m, 4H), 2.02 - 1.88 (m, 1H), 1.76 - 1.66 (m, 1H), 1.45 (d, 6H) | 420 | Method 50 and 3-(N-tert- butoxycarbon yl-N- methylamino) pyrrolidine |

### Examples 179-199

Examples 179 to 199 were prepared by the following general procedure:
4-{[4-(l-Isopropyl-2-methyl-177-imidazol-5-yl)pyrimidin-2-yl]amino}benzoic acid lithium salt (Method 106; 2.47g) and HBTU (2.73g) were stirred together in anhydrous DMF (97 mL) for 1 hr at ambient temperature. Aliquots of the 4-{[4-(l-isopropyl-2-methyl-17V-imidazol-5-yl)pyrimidin-2-yl]amino}benzoic acid lithium salt and HBTU / DMF solution formed (2 mL) were added to the corresponding amine (0.18 mmol) followed by DIPEA (0.45 mmol). The resulting solutions were vortexed at ambient temperature for 66 hrs. The DMF solutions were concentrated *in vacuo,* dissolved in DCM and vortexed with aqueous sodium bicarbonate solution. The organic layer was separated and concentrated *in vacuo.* Purification by RPHPLC gave the title compounds.

| **Ex** | **Compound** | **NMR / m/z** | **Amine** |
|---|---|---|---|
| **179** | [4-(4-Fluorophenyl)piperazin-1- yl]-[4-[[4-(2-methyl-3-propan-2- yl-3H-imidazol-4-yl)pyrimidin-2- yl]amino]phenyl]methanone | 9.69 (s, 1H), 8.44 (m, 1H), 7.79 (m, 2H), 7.44 (s, 1H), 7.40 (m, 2H), 7.11 - 7.04 (m, 3H), 6.98 (m, 2H), 5.69 (m, 1H), 3.65 (m, 4H), 3.12 (m, 4H), 2.50 (m, 3H), 1.47 (d, 6H); m/z 500 | 1-(4-fluorophenyl)piperazine |
| **180** | [4-[[4-(2-Methyl-3-propan-2-yl- 3H-imidazol-4-yl)pyrimidin-2- yl]amino]phenyl]-(4- phenylpiperazin-1-yl)methanone | m/z 482 | 1- phenylpiperazine |
| **181** | [4-(2-Methoxyphenyl)piperazin-1- yl]-[4-[[4-(2-methyl-3-propan-2- yl-3H-imidazol-4-yl)pyrimidin-2- yl] amino]phenyl]methanone | m/z 512 | 1-(2-methoxyphenyl )piperazine |
| **182** | [(2S)-2-(Anilinomethyl)pyrrolidin- 1-yl]-[4-[[4-(2-methyl-3-propan- 2-yl-3H-imidazol-4-yl)pyrimidin- 2-yl]amino]phenyl]methanone | m/z 496 | (S)-(+)-2-(anilinomethyl) pyrrolidine |
| **183** | [4-(2-Fluorophenyl)piperazin-1- yl]-[4-[[4-(2-methyl-3-propan-2- yl-3H-imidazol-4-yl)pyrimidin-2- yl]amino]phenyl]methanone | (400.132 MHz, CDCl₃) 8.39 (d, 1H), 7.69 (m, 2H), 7.46 (m, 2H), 7.39 (s, 1H), 7.18 (s, 1H), 7.11 - 6.93 (m, 5H), 5.65 (m, 1H), 3.83 (m, 4H), 3.11 (m, 4H), 2.60 (s, 3H), 1.55 (d, 6H); m/z 500 | 1-(2- fluorophenyl)pi perazine |
| **184** | [4-(4-Methoxyphenyl)piperazm-l- yl]-[4-[[4-(2-methyl-3-propan-2- yl-3H-imidazol-4-yl)pyrimidin-2- yl]amino]phenyl]methanone | m/z 512 | 1-(4-methoxyphenyl )piperazine |
| **185** | [4-(3-Methoxyphenyl)piperazin-1- yl]-[4-[[4-(2-methyl-3-propan-2- yl-3H-imidazol-4-yl)pyrimidin-2- yl]amino]phenyl]methanone | m/z 512 | 1-(3- methoxyphenyl )piperazine |
| **186** | [4-(4-Chlorophenyl)piperazin-1- yl]-[4-[[4-(2-methyl-3-propan-2- yl-3H-imidazol-4-yl)pyrimidin-2- yl]amino]phenyl]methanone | m/z 516 | 1-(4- chlorophenyl)p iperazine |
| **187** | [4-(4-Hydroxyphenyl)piperazin-1- yl]-[4-[[4-(2-methyl-3-propan-2- yl-3H-imidazol-4-yl)pyrimidin-2- yl]amino]phenyl]methanone | 9.68 (s, 1H), 8.85 (s, 1H), 8.44 (d, 1H), 7.79 (m, 2H), 7.44 (s, 1H), 7.40 (d, 2H), 7.10 (d, 1H), 6.82 (d, 2H), 6.67 (d, 2H), 5.70 (m, 1H), 3.64 (m, 4H), 2.99 (m, 4H), 2.50 (s, 3H), 1.47 (d, 6H); m/z 498 | 1-(4- hydroxyphenyl) piperazine |
| **188** | [4-(4-Methylphenyl)piperazin-1- yl]-[4-[[4-(2-methyl-3-propan-2- yl-3H-imidazol-4-yl)pyrimidin-2- yl]amino]phenyl]methanone | m/z 496 | 1-(4-methylphenyl)- piperazine |
| **189** | [4-(2-Hydroxyphenyl)piperazin-1- yl]-[4-[[4-(2-methyl-3-propan-2- yl-3H-imidazol-4-yl)pyrimidin-2- yl] amino]phenyl]methanone | m/z 498 | N-(2- hydroxyphenyl) piperazine |
| **190** | [4-[[4-(2-Methyl-3-propan-2-yl- 3H-imidazol-4-yl)pyrimidin-2- yl]amino]phenyl]-(4-pyridin-4-yl- 1-piperidinyl)methanone | m/z 482 | 1,2,3,4,5,6- hexahydro-[4,4']- bipyridinyl |
| **191** | [4-(2,4-Difluorophenyl)piperazin- 1-yl]-[4-[[4-(2-methyl-3-propan- 2-yl-3H-imidazol-4-yl)pyrimidin- 2-yl]amino]phenyl]methanone | m/z 518 | 1-(2,4- difluorophenyl) piperazine |
| **192** | [4-(2,6-Dimethylphenyl)piperazin- 1-yl]-[4-[[4-(2-methyl-3-propan- 2-yl-3H-imidazol-4-yl)pyrimidin- 2-yl]amino]phenyl]methanone | 9.68 (s, 1H), 8.44 (d, 1H), 7.78 (d, 2H), 7.44 (s, 1H), 7.41 (d, 2H), 7.10 (d, 1H), 6.96 (m, 3H), 5.70 (m, 1H), 3.62 (m, 4H), 3.04 (m, 4H), 2.50 (s, 3H), 2.30 (s, 6H), 1.47 (d, 6H); m/z 510 | 1-(2,6- dimethylphenyl )piperazine |
| **193** | [4-(2-Chlorophenyl)piperazin-1- yl]-[4-[[4-(2-methyl-3 -propan-2- yl-3H-imidazol-4-yl)pyrimidin-2- yl]amino]phenyl]methanone | m/z 516 | 1-(2- chlorophenyl)p iperazine |
| **194** | [4-[[4-(2-Methyl-3-propan-2-yl-3H-imidazol-4-yl)pyrimidin-2-yl]amino]phenyl]-(2-pyridin-2-ylpyrrolidin-1-yl)methanone | m/z 468 | 2-pyrrolidin-2- ylpyridine |
| **195** | [4-(2-Methylphenyl)piperazin-1- yl] -[4-[[4-(2-methyl-3-propan-2- yl-3H-imidazol-4-yl)pyrimidin-2- yl] amino]phenyl]methanone | m/z 496 | 1-(0- tolyl)piperazine |
| **196** | [4-(3-Methylphenyl)piperazin-1- yl] -[4-[[4-(2-methyl-3 -propan-2- yl-3H-imidazol-4-yl)pyrimidin-2- yl] amino]phenyl]methanone | m/z 496 | 1-(3- methylphenyl)- piperazine |
| **197** | [4-(5-Chloropyridin-2- yl)piperazin-1-yl]-[4-[ [4-(2- methyl-3-propan-2-yl-3H- imidazol-4-yl)pyrimidin-2- yl] amino]phenyl]methanone | m/z 517 | 1-(5- chloropyridin- 2-yl)piperazine |
| **198** | [4-(2,3-Dimethylphenyl)piperazin- 1 -yl]-[4-[[4-(2-methyl-3-propan- 2-yl-3H-imidazol-4-yl)pyrimidin- 2-yl]amino]phenyl]methanone | m/z 510 | 1-(2,3- dimethylphenyl )-piperazine |
| **199** | [4-(3,4-Difluorophenyl)piperazin- 1-yl]-[[4-[[4-(2-methyl-3-propan- 2-yl-3H-imidazol-4-yl)pyrimidin- 2-yl]amino]phenyl]methanone | m/z 518 | 1-(3,4- difluorophenyl) piperazine |

### Example 200

### [4-[[4-(2-Methyl-3-propan-2-yl-3H-imidazol-4-yl)pyrimidin-2-yl]amino]phenyl]-[(3R)-3-methylsulfonyloxypyrrolidin-1-yl]methanone

(3R)-1-(4-{[4-(1-Isopropyl-2-methyl-1H-imidazol-5-yl)pyrimidin-2-yl]amino}benzoyl)pyrrolidin-3-ol (Example 173; 3.8 g) and TEA (1.92 ml) were added to DCM, the reaction mixture was cooled to 0°C (ice-bath) before the slow addition of methanesulfonyl chloride (0.781 ml). The reaction mixture was stirred for 1 hr before adding water (50 ml), then extracted with DCM (3 x 100 ml). The combined organics were dried and the solvent *removed in vacuo.* The solid obtained was dissolved in the minimum amount of hot acetonitrile, cooled, filtered and dried to give the title compound as a white solid (3.6 g). NMR 9.26 (s, 1H), 8.42 (d, 1H), 7.76 (d, 2H), 7.49 (d, 2H), 7.39 (s, 1H), 7.05 (d, 1H), 5.60 (septet, 1H), 5.31 - 5.27 (m, 1H), 3.87 (dd, 1H), 3.72 (d, 1H), 3.67 - 3.60 (m, 2H), 3.16 (s, 3H), 2.87 (s, 3H), 2.29 - 2.17 (m, 2H), 1.48 (d, 6H); m/z 485.

### Example 201

### [4-[[4₋(2-Methyl-3-propan-2-yl-3H-imidazol-4-yl)pyrimidin-2-yl]amino]phenyl]-[(3S)-3-(morpholin-4-yl)pyrrolidin-1-yl]methanone

A solution of [4-[[4-(2-methyl-3-propan-2-yl-3H-imidazol-4-yl)pyrimidin-2-yl]amino]phenyl]-[(3R)-3-methylsulfonyloxypyrrolidin-1-yl]methanone (Example 200; 0.1 g) and morpholine (0.09 g) in dioxane (10 ml) was heated at 100°C for 48 hrs. The reaction mixture was concentrated *in vacuo,* the residue loaded onto a SCX-2 column in MeOH, washed with MeOH then eluted with 7N NH₃ in MeOH. The crude product was purified via column chromatography on silica gel eluting with 0-10% MeOH in DCM to give the title compound as a colourless solid (0.04 g). NMR (500.133 MHz) 09.22 (s, 1H), 8.40 (d, 1H), 7.73 (d, 2H), 7.46 (d, 2H), 7.37 (s, 1H), 7.04 (d, 1H), 5.60 (septet, 1H), 3.69 - 3.64 (m, 2H), 3.62 - 3.57 (m, 5H), 3.51 - 3.45 (m, 1H), 3.35 (dd, 1H), 2.47 - 2.45 (m, 1H), 2.40 - 2.34 (m, 2H), 2.09 - 2.02 (m, 1H), 1.83 - 1.75 (m, 1H), 1.47 (d, 6H); m/z 476.

### Examples 202-204

The following compounds were prepared by the procedure of Example 201 using the appropriate starting materials.

| **Ex** | **Compound** | **NMR** | **m/z** | **SM** |
|---|---|---|---|---|
| **202** | {(3S)-3-[*N*-(Cyclobutyl)-*N*-(methyl)amino]pyrroli din-1-yl}-[4-[[4-(2- methyl-3-propan-2-yl- 3H-imidazol-4- yl)pyrimidin-2- yl]amino]phenyl]metha none | 9.22 (s, 1H), 8.40 (d, 1H), 7.73 (d, 2H), 7.45 (d, 2H), 7.37 (s, 1H), 7.04 (d, 1H), 5.61 (septet, 1H), 3.61 - 3.54 (m, 2H), 3.47 - 3.41 (m, 1H), 3.32 (dd, 1H), 3.11 - 2.98 (m, 2H), 2.49 (s, 3H), 2.09 (s, 3H), 1.99 - 1.75 (m, 6H), 1.62 - 1.51 (m, 2H), 1.47 (d, 6H) | 474 | Example 200 and N- methylcyclob utanamine |
| **203** | {(3S)-3-[*N*-(Cyclopropylmethyl)- *N-* (methyl)amino]pyrroli din-1-yl}-[4-[[4-(2- methyl-3-propan-2-yl- 3H-imidazol-4- yl)pyrimidin-2- yl]amino]phenyl]metha none | 9.22 (s, 1H), 8.40 (d, 1H), 7.73 (d, 2H), 7.46 (d, 2H), 7.37 (s, 1H), 7.04 (d, 1H), 5.61 (septet, 1H), 3.66 - 3.57 (m, 2H), 3.49 - 3.44 (m, 1H), 3.32 (dd, 1H), 3.09 (quintet, 1H), 2.31 - 2.26 (m, 5H), 2.06 - 2.00 (m, 1H), 1.82 - 1.74 (m, 1H), 1.47 (d, 6H), 0.87 - 0.80 (m, 1H), 0.48 - 0.44 (m, 2H), 0.10 - 0.07 (m, 2H) | 474 | Example 200 and N-(cyclopropyl methyl)metha namine |
| **204** | {(3S)-3-[*N-*-(Cyclopropyl)-N (methyl)amino]pyrroli din-1-yl}-[4-[[4-(2- methyl-3-propan-2-yl- 3H-imidazol-4- yl)pyrimidin-2- yl]amino]phenyl]metha none | 9.21 (s, 1H), 8.40 (d, 1H), 7.73 (d, 2H), 7.46 (d, 2H), 7.36 (s, 1H), 7.04 (d, 1H), 5.61 (septet, 1H), 3.68 - 3.64 (m, 1H), 3.60 - 3.55 (m, 1H), 3.50 - 3.39 (m, 2H), 3.15 (quintet, 1H), 2.28 (s, 3H), 2.10 - 2.03 (m, 1H), 1.93 - 1.85 (m, 1H), 1.73 - 1.69 (m, 1H), 1.47 (d, 6H), 0.48 - 0.39 (m, 2H), 0.36 - 0.28 (m, 2H) | 460 | Example 200 and N- methylcyclopr opanamine |

### Example 205

### [4-[[4-(2-Methyl-3-propan-2-yl-3H-imidazol-4-yl)pyrimidin-2-yl]amino]phenyl]-[(1S,4S)-3-propan-2-yl-3,6-diazabicyclo[2.2.1]hept-6-yl]methanone

[(1S,4S)-2,5-Diazabicyclo[2.2.1]hept-5-yl]-[4-[[4-(2-methyl-3-propan-2-yl-3H-imidazol-4-yl)pyrimidin-2-yl]amino]phenyl]methanone (Example 109; 100 mg), 3A molecular sieves (1g) and acetone (28 mg) were added to methanol (10 mL) and stirred for 10 mins. Sodium triacetoxyborohydride (66 mg) was added and the reaction stirred at ambient temperature for 66 hrs. Additional sodium triacetoxyborohydride (33 mg) and acetone (28 mg) were added and the reaction mixture heated at 50°C for 16 hrs before adding additional sodium triacetoxyborohydride (33 mg) and acetone (28 mg) and heating for 5 hrs. The reaction mixture was cooled, filtered and passed through a SCX-2 column, eluting with MeOH then 3.5N NH₃ in MeOH then 7N NH₃ in MeOH then 1% TEA in MeOH and finally 2% TEA in MeOH. Additional purification by RPHPLC gave the title compound as a colourless foam (62 mg); NMR (500.133 MHz) 9.22 (s, 1H), 8.40 (d, 1H), 7.75 - 7.72 (m, 2H), 7.46 - 7.43 (m, 2H), 7.37 (s, 1H), 7.04 (d, 1H), 5.60 (septet, 1H), 4.45 - 4.36 (m, 1H), 3.68 (s, 1H), 3.47 - 3.36 (m, 2H), 2.99 - 2.96 (m, 1H), 2.64 - 2.59 (m, 2H), 2.50 - 2.48 (m, 3H obscured by DMSO), 1.75 - 1.69 (m, 2H), 1.48 - 1.46 (m, 6H), 1.02 - 0.99 (m, 6H); m/z 460.

### Example 206

### [(1S,4S)-3-Methyl-3,6-diazabicyclo[2.2.1]hept-6-yl]-[4-[[4-(2-methyl-3-propan-2-yl-3H-imidazol-4-yl)pyrimidin-2-yl]amino]phenyl]methanone

[(1S,4S)-2,5-Diazabicyclo[2.2.1]hept-5-yl]-[4-[[4-(2-methyl-3-propan-2-yl-3H-imidazol-4-yl)pyrimidin-2-yl]amino]phenyl]methanone (Example 109; 100 mg) was dissolved in MeOH (5 mL) then aqueous formaldehyde (37% wt; 0.22 mL) added and the resulting solution stirred at ambient temperature for 10 mins. Sodium cyanoborohydride (23 mg) was added in one portion and the mixture stirred for 2 hrs. After which 2M NaOH solution (3 mL) was added, the reaction mixture stirred for 30 min then extracted with DCM (3 x 20 mL). The combined organics were dried (MgSO₄), filtered and the solvent *removed in vacuo.* Purification by RPHPLC then trituration with ether gave the title compound as a colourless solid; NMR (500.133 MHz, DMSO) 9.22 (s, 1H), 8.40 (d, 1H), 7.75 - 7.72 (m, 2H), 7.45 - 7.42 (m, 2H), 7.36 (s, 1H), 7.04 (d, 1H), 5.60 (septet, 1H), 4.44 - 4.35 (m, 1H), 3.49 - 3.46 (m, 1H), 3.39 - 3.35 (m, 2H), 2.79 (d, 1H), 2.67 - 2.64 (m, 1H), 2.50 - 2.48 (m, 3H, obscured by DMSO), 2.33 (s, 3H), 1.80 (d, 1H), 1.68 (d, 1H), 1.48 - 1.46 (m, 6H); m/z 432.

### Example 207

### [4-[[5-Fluoro-4-(2-methyl-3-propan-2-yl-3H-imidazol-4-yl)pyrimidin-2-yl]amino]phenyl]-[(lS,4S)-3-methyl-3,6-diazabicyclo[2.2.1]hept-6-yl]methanone

The title compound was prepared by the procedure of Example 206 using [(1S,4S)-2,5-diazabicyclo[2.2.1]hept-5-yl]-[4-[[5-fluoro-4-(2-methyl-3-propan-2-yl-3H-imidazol-4-yl)pyrimidin-2-yl]amino]phenyl]methanone (Example 131) in place of Example 109. NMR (500.133 MHz, DMSO) 9.33 (s, 1H), 8.49 (d, 1H), 7.69 (d, 2H), 7.44 (d, 2H), 7.36 (d, 1H), 5.40 (septet, 1H), 4.42 - 4.36 (m, 1H), 3.49-3.45 (m, 1H), 3.39 - 3.35 (m, 2H), 2.79 (dd, 1H), 2.65 (d, 1H), 2.52 (s, 3H), 2.34 (s, 3H), 1.80 (d, 1H), 1.68 (d, 1H), 1.46 (d, 6H); m/z 450.

### Example 208

### r (1 SAS)-3-(2- Methoxyethyl)-3,6-diazabicyclor2.2.l1hept-6-yll-r 4-rr 4-(2-methyl-3-propan-2-yl-3H-imidazol-4-yl)pyrimidin-2-yl]amino]phenyl]methanone

[(1S,4S)-2,5-Diazabicyclo[2.2.1]hept-5-yl]-[4-[[4-(2-methyl-3-propan-2-yl-3H-imidazol-4-yl)pyrimidin-2-yl]amino]phenyl]methanone (Example 109; 100 mg), 2-bromoethyl methyl ether (0.034 mL) and TEA (0.067 mL) were added to DMA (5 mL) and heated at 70°C for 18 hrs. The reaction mixture was then cooled, concentrated *in vacuo,* passed through a Flash SCX column eluting with MeOH then 7N NH₃ in MeOH. Additional purification by RPHPLC gave the title compound as a pale yellow solid (23 mg); NMR (500.133 MHz, DMSO) 9.22 (s, 1H), 8.40 (d, 1H), 7.75 - 7.72 (m, 2H), 7.46 - 7.43 (m, 2H), 7.37 (s, 1H), 7.04 (d, 1H), 5.60 (septet, 1H), 4.44 - 4.36 (m, 1H), 3.59 - 3.53 (m, 2H), 3.48 - 3.36 (m, 4H), 3.26 (s, 3H), 2.75 - 2.63 (m, 3H), 2.50 - 2.48 (m, 3H, obscured by DMSO), 1.79 - 1.67 (m, 2H), 1.47 (d, 6H); m/z 476.

### Preparation of Starting materials

### Method 1

### (2Z)-3-(Dimethylamino)-2-fluoro-1 -(1 -isopropyl-2-methyl- 17V-imidazol-5-yi)prop-2-en-1 -one

To a stirred solution of (2*E*)-3-(dimethylamino)-1-(1-isopropyl-2-methyl-1*H*-imidazol-5-yl)prop-2-en-1-one, (Method 24 of WO 03/076436; 5.53g, 25mmol) in MeOH (100ml) at ambient temperature was added in portions over∼5mins (1-chloromethyl-4-fluoro-1,4-diazoniabicyclo[2.2.2]octane bis(tetrafluoroborate) (14.16g, 40mmol). The temperature was maintained at 25-30°C by slight cooling. After stirring for 90 mins the reaction mixture was cooled in ice/acetone and filtered. The filtrate was evaporated under reduced pressure and the residue was taken into DCM. It was washed with aq. ammonia, brine, dried (Na₂S0₄) and evaporated under reduced pressure. The title compound was isolated by MPLC on silica gel using two separate columns (10% EtOH / EtOAc, then 3.5% EtOH / DCM) as a golden viscose oil, which crystallized on standing over several weeks. Yield = 2.50g (42%). NMR: 1.40 (d, 6H), 2.38 (s, 3H), 3.05 (s, 6H), 4.70 (septet, 1H), 6.96 (d, 1H), 7.08 (s, 1H); fluorine NMR (376MHz): -166.7 (d); m/z 240.

### Method 2

### 5-Fluoro-4-(3-isopropyl-2-methyl-3H-imidazol-4-yl)-pyrimidin-2-ylamine

(2Z)-3-(Dimethylamino)-2-fluoro-1 -(1 -isopropyl-2-methyl- 17Y-imidazol-5-yl)prop-2-en-1-one (Method 1; 4.0g, 16.7 mmol) and guanidine carbonate (6.6g, 37 mmol) were pre-mixed in butanol (80 ml) and heated at reflux for 30 hours. The reaction was allowed to cool before being quenched with water (200 ml) the reaction was then extracted with DCM (2 x 200 ml), dried and solvent was *removed in vacuo* to yield a yellow solid. The solid was dissolved in minimum amount of warm DCM, this was then allowed to cool before the addition of ether. An off white solid precipitated this was filtered and dried. The process was repeated to obtain second crop ofproduct (3.18g, 81%). NMR (299.954 MHz, CDCl₃): 8.15 (d, 1H), 7.54 (d, 1H), 7.26 (s, 1H), 5.40 (septet, 1H), 4.88 (s, 2H), 2.59 (s, 3H), 1.56 (d, 6H); m/z 236.

### Method 3

### 4-[1-Isopropyl-2-(methoxymethyl)-1H-imidazol-4-yl]-pyrimidin-2-ylamine

The title compound was prepared by the procedure of Method 2 and on the same scale, using guanidine carbonate and 3-(dimethylamino)-1-[1-isopropyl-2-(methoxymethyl)-1H imidazol-5-yl]prop-2-en-1-one (Method 50 of WO 03/076434) NMR (400.132 MHz, CDCl₃): 8.26 (d, 1H), 7.38 (s, 1H), 6.82 (d, 1H), 5.30 (septet, 1H), 5.14 (s, 2H), 4.64 (s, 3H), 3.39 (s, 3H), 1.59 (d, 6H); m/z 248.

### Method 4

### (4-Bromo-2-methyl-phenyl)-(4-methyl-piperazin-1-yl)-methanone

Bromo-methylbenzoic acid (10 g, 46.5 mmol), and HBTU (23 g, 60.5 mmol) were dissolved in DMF (150 ml), then *N*-methyl piperazine (6.0 g, 60.5 mmol) and DIPEA (21 ml, 121 mmol) were added. The reaction was stirred overnight before the removal of the *DMF in vacuo,* the gum was quenched with 2.0N NaOH (100 ml), extracted with ether (3 x 200 ml), dried and solvent removed in vacuo to yield a viscous gum. Purification on silica using 0-10% MeOH in DCM as eluent, gave the title compound as viscous oil. The oil was dissolved in the minimum amount of ether, iso-hexane was added to give a colourless solid which was filtered and dried (11.8 g, 86%). NMR (CDCl₃): 7.40 (s, 1H), 7.36 (d, 1H), 7.04 (d, 1H), 3.86 - 3.79 (m, 2H), 3.27 - 3.21 (m, 2H), 2.51 - 2.45 (m, 2H), 2.32 - 2.29 (m, 8H); m/z 298.

### Methods 5-9

Using the procedure described for Method 4 the following compounds were prepared in a similar way.

| **Meth** | **Compound** | **NMR** | **m/z** | **SM** |
|---|---|---|---|---|
| **5** | (4-Bromo-2-fluoro-phenyl)-(4-methyl- piperazin-1-yl)- methanone | (CDC1₃) 7.35 (d, 1H), 7.33 - 7.22 (m, 2H), 3.86 - 3.79 (m, 2H), 3.27 - 3.21 (m, 2H), 2.51 - 2.45 (m, 2H), 2.32 - 2.29 (m, 8H) | 301 | 4-bromo-2- fluorobenzoic acid, N- methylpiperazine |
| **6** | (4-Bromo-2-fluoro-phenyl)-morpholin- 4-yl-methanone | (CDCl₃) 7.40 (d, 1H), 7.33 - 7.31 (m, 1H), 7.30 - 7.26 (m, 1H), 3.87 - 3.74 (m, 4H), 3.67 - 3.58 (m, 2H), 3.40 - 3.29 (m, 2H) | 289 | 4-bromo-2- fluorobenzoic acid, morpholine |
| **7** | (4-Bromo-2-chloro-phenyl)-(4-methyl-piperazin-1-yl)-methanone | (400.132 MHz, CDCl₃) 7.59 (s, 1H), 7.46 (d, 1H), 7.17 (d, 1H), 3.89 - 3.75 (m, 2H), 3.32 - 3.18 8 (m, 2H), 2.54 - 2.46 (m, 2H), 2.44 - 2.28 (m, 5H) | 319 | 4-bromo-2- chlorobenzoic acid, N- methylpiperazine |
| **8** | (4-Bromo-2- chloro-phenyl)- morpholin-4-yl- methanone | (400.132 MHz, CDC1₃) 7.60 (s, 1H), 7.48 (d, 1H), 7.18 (d, 1H), 3.91 - 3.83 (m, 1H), 3.79 - 3.74 (m, 3H), 3.72 - 3.66 (m, 1H), 3.62 - 3.57 (m, 1H), 3.31 - 3.26 (m, 1H), 3.23 - 3.18 (m, 1H) | 306 | 4-bromo-2- chlorobenzoic acid, morpholine |
| **9** | (4-Bromo-2- methyl-phenyl)- morpholin-4-yl- methanone | (CDC1₃) 7.40 (s, 1H), 7.36 (d, 1H), 7.04 (d, 1H), 3.83 - 3.73 (m, 4H), 3.61 - 3.56 (m, 2H), 3.26 - 3.20 (m, 2H), 2.30 (s, 3H) | 285 | 4-bromo-2- methylbenzoic acid, morpholine |

### Method 10

### 1-(4-Iodobenzoyl)-N,N-dimethylpyrrolidin-3-amine

*N,N* Dimethylpyrrolidin-3-amine (5.0 g, 43.8 mmol) and TEA (7.3 ml, 52.5 mmol) were stirred in THF (200 ml) under an inert atmosphere. 4-Iodobenzoyl chloride (11.7 g, 43.8 mmol) was added in portions over 5 mins. Stirring was continued for a further 16 hours, then the solvent was evaporated *in vacuo* and the residue partitioned between EtOAc (200 ml) and 1M NaOH (100 ml). The organics were washed with water (100 ml) and brine (100 ml), dried and evaporated to give the title compound as a colourless solid (12.3 g, 74%). NMR (400.13 3 MHz): 7.83 (d, 2H), 7.32 (ap. t, 2H), 3.75-3.58 (m, 1H), 3.52-3.38 (m, 2H), 3.31-3.16 (m, 1H), 2.78-2.59 (m, 1H), 2.18 (s, 3H), 2.11 (s, 3H), 2.10-1.98 (m, 1H), 1.81-1.63 (m, 1H); m/z 345.

### Method 11

### 1-(4-Chloro-2-methoxybenzoyl)-4-methylpiperazine

Thionylchloride (5 ml) was added to 4-chloro-2-methoxybenzoic acid (0.501 g, 2.68 mmol). After addition of one drop of dry DMF, the reaction mixture was stirred under reflux for 30 mins. The solvent was *removed in vacuo* and the residue was co-evaporated with toluene. The residue was redissolved in dry DCM (5 ml) and *N-*methylpiperazine (0.277 mg, 2.77 mmol) was added dropwise followed by TEA (0.28 g, 2.77 mmol), and the resulting mixture was stirred at ambient temperature for 15 mins. The reaction mixture was diluted (DCM), washed with saturated NaHC0₃ (aq.), water, dried (Na₂S0₄), filtered and concentrated *in vacuo* to give the title compound in a quantitative yield. This crude product was used in the next step without further purification. NMR: 7.18 (d, *J=*8.0 Hz, 1H), 7.17 (d, *J=*2.0 Hz, 1H), 7.05 (dd, *J*=8.0, 1.8 Hz, 1H), 3.81 (s, 3H), 3.67-3.51 (m, 2H), 3.14-3.04 (m, 2H), 2.38-2.27 (m, 2H), 2.27-2.19 (m, 2H), 2.18 (s, 3H).

### Method 12

### 1-(4-Chloro-2-iodobenzoyl)-4-methylpiperazine

4-Chloro-2-iodobenzoic acid (0.523 g, 1.85 mmol) was dissolved in thionyl chloride (2.5 ml) and one drop of dry DMF. The reaction mixture was stirred under reflux for 1 hr followed by evaporation of excess thionyl chloride. The residue was dissolved in dry DCM (5 ml) and *N*-methylpiperazine (0.194 g, 1.94 mmol) was added in portions, followed by addition of TEA (0.196 g, 1.94 mmol). The mixture was stirred at ambient temperature overnight. The mixture was then diluted (DCM) and washed with saturated NaHC0₃ (aq.), dried (Na₂SO₄), filtered and evaporated *in vacuo.* The product was purified by silica flash chromatography (CHC1₃/MeOH gradient; 0 to 5% MeOH), giving the title compound (0.415 g, 61%) as a solid. NMR: 7.97 (d, *J*=2.0 Hz, 1H), 7.54 (dd, *J*=8.3, 2.0 Hz, 1H), 7.26 (d, *J*=8.3 Hz, 1H), 3.69-3.53 (m, 2H), 3.08 (t, *J*=5.0 Hz, 2H), 2.38 (t, *J*=5.1 Hz, 2H), 2.36-2.21 (m, 2H), 2.19 (s, 3H); MS (ESI) m/z 365.

### Method 13

### 5 -Chloro-2-[(4-methylpiperazin- I -yi)carbonyl]benzonitrile

1-(4-Chloro-2-iodobenzoyl)-4-methylpiperazine (Method 12, 400 mg, 1.70 mmol), zinc acetate (17.2 mg, 0.079 mmol), zinc cyanide (109 mg, 0.928 mmol), zinc dust (8.0 mg, 0.122 mmol), Pd₂(dba)₃ (44 mg, 0.048 mmol) and 1,1'-bis(diphenylphosphino)ferrocene (117 mg, 0.211 mmol) were mixed in anhydrous 1,4-dioxane (1.5 ml) and flushed with argon. The mixture was heated in a sealed tube at 90-95 °C for 45 mins. The reaction mixture was filtered through diatomaceous earth and rinsed with EtOAc. The organic phase was washed with saturated NaHCO₃ (aq.), dried (Na₂S0₄), filtered and evaporated *in vacuo.* The crude product was purified by flash chromatography (DCM/MeOH-gradient; 0 to 5% MeOH) to give the title compound (280 mg, 62%). NMR: 8.16 (d, *J*=2.0 Hz, 1H), 7.87 (dd, *J*=8.3, 2.0 Hz, 1H), 7.59 (d, *J*=8.3 Hz, 1H), 3.71-3.59 (m, 2H), 3.24-3.14 (m, 2H), 2.43-2.32 (m, 2H), 2.32-2.22 (m, 2H), 2.19 (s, 3H); MS (ESI) m/z 264.

### Method 14

### 4-(3-Isopropyl-2-methyl-3H-imidazol-4-yl)-pyrimidin-2-ylamine

(2*E*)-3-(Dimethylamino)-1-(1-isopropyl-2-methyl-1*H*-imidazol-5-yl)prop-2-en-1-one, (Method 24 of WO 03/076436 4.0g, 18 mmol) and guanidine carbonate (7.2g, 40 mmol) were pre-mixed in 2-methoxyethanol (80 ml) and heated at reflux for 30 hours. The reaction was allowed to cool before being quenched with water (50 ml). The reaction was then extracted with DCM (2 x 200 ml), dried and solvent was *removed in vacuo* to yield a yellow solid. The solid was dissolved in minimum amount of warm DCM, this was then allowed to cool before the addition of ether. An off white solid precipitated this was filtered and dried. The process was repeated to obtain second crop of product (3.18g, 81 %). NMR (299.954 MHz, CDCl₃): 8.22 (d, 1H), 7.33 (s, 1H), 6.80 (d, 1H), 5.45 (septet, 1H), 5.10 (s, 2H), 2.56 (s, 3H), 1.54 (d, 6H); m/z 218.

### Method 15

### (4-Chloro-2-iodo-phenyl)-morpholin-4-yl-methanone

4-Chloro-2-iodobenzoic acid (5.0 g, 17.7 mmol), and HBTU (8.7 g, 23 mmol) were dissolved in DMF (150 ml), to this was added morpholine (2.0 g, 23 mmol) followed by DIPEA (8.2 ml, 46 mmol). The reactions was stirred overnight before the removal of the DMF *in vacuo,* the gum was quenched with 2.ON NaOH (100 ml), extracted with DCM (3 x 200 ml), dried and solvent *removed in vacuo* to yield a brown solid. Purification on silica using 0-3.5% MeOH in DCM as eluent gave the title compound as an off white solid (5.8 g, 94%). NMR (CDC1₃) 7.84 (s, 1H), 7.39 (d, 1H), 7.13 (d, 1H), 3.91 - 3.73 (m, 5H), 3.62 - 3.51 (m, 1H), 3.33 - 3.24 (m, 1H), 3.21 - 3.12 (m, 1H); m/z 352.

### Method 16

### 5-Chloro-2-(morpholine-4-carbonyl)-benzonitrile

(4-Chloro-2-iodo-phenyl)-morpholin-4-yl-methanone (Method 15; 5.8 g, 16.5 mmol), copper cyanide (5.2 g, 58 mmol), Pd₂(dba)₃ (0.45 g, 0.50 mmol), 1,1'-bis(diphenylphosphino) ferrocene (0.82 g, 1.48 mmol) and Et₄NCN (2.6 g, 15.5 mmol) were added to dioxane (75 ml) and heated at reflux under an inert atmosphere for 3 hours. The reaction was filtered through diatomaceous earth and the solvent *removed in vacuo* to yield a viscous brown solid. Purification on silica using 0-2.5% MeOH in DCM as eluent gave the title compound as a brown solid. The solid was added to MeOH (50 ml), heated and then sonicated, the solid obtained was filtered and dried (3.1 g, 76%). NMR (CDC1₃) 7.71 (s, 1H), 7.65 (d, 1H), 7.42 (d, 1H), 3.92 - 3.64 (m, 6H), 3.39 - 3.25 (m, 2H); m/z 251.

### Method 17

### (4-Benzyloxy-2-methoxy-phenyl)-(4-methyl-piperazin-1-yl)-methanone

4-Benzyloxy-2-methoxy-benzoic acid (7.0 g, 27 mmol) and HBTU (13.3 g, 35 mmol) were added to DMF (100 ml) then *N*-methylpiperazine (3.5 g, 35 mmol) and DIPEA (12.5 ml, 70 mmol) added. The reaction was stirred for 1 hour before adding 2.0 NaOH (100 ml), extracted with ether (3 x 200 ml), dried and solvent *removed in vacuo* to yield a yellow oil. (8.5 g, 92%). NMR (CDCl₃) 7.44 - 7.33 (m, 5H), 7.17 (d, 1H), 6.58 (d, 1H), 6.54 (s, 1H), 5.07 (s, 2H), 3.87 - 3.72 (m, 5H), 3.32 - 3.24 (m, 2H), 2.53 - 2.40 (m, 2H), 2.37 - 2.23 (m, 5H).

### Method 18

### (4-Hydroxy-2-methoxy-phenyl)-(4-methyl-piperazin-1-yl)-methanone

(4-Benzyloxy-2-methoxy-phenyl)-(4-methyl-piperazin-1-yl)-methanone (Method 17; 7.0 g, 20.5 mmol), 10% palladium on carbon (0.3 g) and ammonium formate (6.6 g, 103 mmol) were added to MeOH and heated at reflux for 1 hour. The solvent was removed *in vacuo* to yield a white solid. DCM (100 ml) was added and this was sonicated for 20 minutes, the reaction was filtered, the filtrate dried and solvent *removed in vacuo* to yield a white solid (5.0 g), which was used without further purification; m/z 251.

### Method 19

### Trifluoro-methanesulfonic acid 3-methoU-4-(4-methyl-piperazine-l-carbonyl)-phepLI ester

(4-Hydroxy-2-methoxy-phenyl)-(4-methyl-piperazin-1-yl)-methanone (Method 18; 5.0 g, 20 mmol) and TEA was added to DCM (100 ml) and cooled to 0 °C, then triflic anhydride (4.4 ml, 26 mmol) was slowly added and the reaction was stirred for 1 hour. Additional triflic anhydride (0.3 eq,) was added and stirring continued for 1 hour. Water (100 ml) was added and the DCM was removed *in vacuo,* the remaining aqueous was extracted with ether (2 x 100 ml), dried and solvent removed *in vacuo* to yield a black oil. Purification on silica using 0-2.5% MeOH in DCM as eluent gave the title compound as a black gum (4.2 g, 55%). NMR (CDC1₃): 7.34 (d, 1H), 6.94 (d, 1H), 6.82 (s, 1H), 3.97 - 3.89 (m, 2H), 3.87 (s, 3H), 3.44 - 3.31 (m, 2H), 2.77 - 2.69 (m, 2H), 2.62 - 2.54 (m, 2H), 2.51 (s, 3H); m/z 383.

### Method 20

### (4-Benzyloxy-2-methoxy-phenyl)-morpholin-4-yl-methanone

4-Benzyloxy-2-methoxy-benzoic acid (7.0 g, 27 mmol) and HBTU (13.3 g, 35 mmol) were added to DMF (100 ml), to this was added morpholine (3.0 g, 35 mmol) and DIPEA (12.5 ml, 70 mmol). The reaction was stirred for 1 hour before being quenched with 2.0 NaOH (100 ml), extracted with ether (3 x 200 ml), dried and solvent removed *in vacuo* to yield a yellow oil. (8.4 g, 94%). NMR (CDC1₃) 7.44 - 7.32 (m, 5H), 7.19 (d, 1H), 6.59 (d, 1H), 6.53 (s, 1H), 5.07 (s, 2H), 3.80 - 3.69 (m, 7H), 3.64 - 3.53 (m, 2H), 3.32 - 3.20 (m, 2H); m/z 328.

### Method 21

### (4-Hydroxy-2-methoxy-phenyl)-morpholin-4-yl-methanone

(4-Benzyloxy-2-methoxy-phenyl)-morpholin-4-yl-methanone (Method 20; 8.8 g, 27 mmol), ammonium formate (4.3 g, 67.2 mmol) and 10% palladium on carbon (0.3 g) were added to MeOH (150 ml) and heated at reflux for 2 hours. The reaction was filtered and the solvent *removed in vacuo* to yield a white solid. Purification of the solid on silica with 0%-5% MeOH in DCM as eluent gave the title compound as a white solid (5.1 g, 80%). NMR (400.132 MHz) 9.73 (s, 1H), 6.99 (d, 1H), 6.43 (s, 1H), 6.39 (d, 1H), 3.74 (s, 3H), 3.64 - 3.54 (m, 4H), 3.54 - 3.45 (m, 2H), 3.20 - 3.09 (m, 2H); m/z 238.

### Method 22

### Trifluoro-methanesulfonic acid 3-methoxy-4-(morpholine-4-carbonyl)-phenyl ester

(4-Hydroxy-2-methoxy-phenyl)-morpholin-4-yl-methanone (Method 21; 1.0 g, 4.22 mmol) and 2-[N,N-bis(trifluoromethylsulfonyl)amino]-5-chloropyridine (1.80 g, 4.6 mmol) were added to THF (30 ml) and heated at 55 °C overnight. The reaction was quenched with water (50 ml), extracted with ether (3 x 50 ml), dried and solvent *removed in vacuo* to yield a yellow oil. Purification on silica gel with 0%-1% MeOH in DCM as eluent gave the title compound as a yellow gum (1.4 g, 90%). NMR (CDC1₃) 7.34 (d, 1H), 6.82 (d, 1H), 3.88 (s, 3H), 3.83 - 3.72 (m, 4H), 3.66 - 3.57 (m, 2H), 3.29 - 3.18 (m, 2H); m/z 370.

### Method 23

### /V-Ethyl-/V-(5-methyl-isoxazol-4-yl)-isobutyramide

Ethyl-(5-methyl-isoxazol-4-yl)-amine hydrochloride (15 g, 0.092 mol) was added to DCM (200 ml), TEA (32 ml, 0.23 mol) was added, followed by the slow addition of iso-butryl chloride (10.7 g, 0.10 mol). The reaction was stirred for 30 minutes before the removal of the solvent *in vacuo.* The residue was treated with water (150 ml), extracted with ether (3 x 150 ml), dried and solvent *removed in vacuo* to yield a yellow oil (12.9 g, 72%). NMR (300.072 MHz, CDC1₃) 8.14 (s, 1H), 3.61 (q, 2H), 2.46 - 2.37 (m, 4H), 1.09 (t, 3H), 1.03 (d, 6H); m/z 197.

### Method 24

### N-{1-[1-Amino-meth-(Z)-ylidene]-2-oxo-propyl}-N-ethyl-isobutyramide

*N* Ethyl-*N*-(5-methyl-isoxazol-4-yl)-isobutyramide (Method 23; 15.6 g, 0.08 mol) and 10% Pd on carbon (3.9 g) were added to EtOH and stirred at 4 atm over night. The reaction was filtered and solvent *removed in vacuo* to yield an off white solid. Ether (150 ml) was added and the reaction was sonicated for 10 minutes before being filtered and dried. A white solid was obtained (11 g, 69%). NMR (400.132 MHz) 7.57 (t, 1H), 6.99 (brs, 1H), 6.79 (brs, 1H), 3.39 - 3.31 (m, 3H), 2.43 - 2.33 (m, 1H), 2.09 (s, 3H), 0.92 - 0.81 (m, 9H); m/z 199.

### Method 25

### 1-(3-Ethyl-2-isopropyl-3H-imidazol-4-yl)-ethanone

*N*-{1-[1-Amino-meth-(Z)-ylidene]-2-oxo-propyl}-*N*-ethyl-isobutyramide (Method 24; 11 g, 0.056 mol) and NaOH (2.7 g, 0.067 mol) were added to EtOH (150 ml) and heated at reflux for 4 hours. To the reaction was added solid NH₄C1 (4.4 g, 0.084 mol), this was stirred overnight. The resulting slurry was concentrated *in vacuo,* ether (200 ml) was added, stirred for 10 minutes then filtered. The filtrate was concentrated *in vacuo* to yield orange oil. This was distilled using bulb-to-bulb distillation (0.76 mmbar/120°C) to give a clear oil (8.2 g, 81%). NMR (400.132 MHz, CDC1₃) 7.74 (s, 1H), 4.34 (q, 2H), 3.04 (septet, 1H), 2.44 (s, 3H), 1.35 (d, 6H), 1.32 (t, 3H); m/z 181.

### Method 26

### (E)-3-Dimethylamino-1-(3-ethyl-2-isopropyl-3H-imidazol-4-yl)-propenone

1-(3-Ethyl-2-isopropyl-3H-imidazol-4-yl)-ethanone (Method 25; 7.0 g, 0.039 mol) and DMFDMA (13.3 ml, 0.078 mol) were added to DMF and heated at 130°C for 6 hours. The solvent was removed *in vacuo* to yield a dark gum. Ether (50 ml) was added to the gum to afford a golden solid which was filtered and dried to give the title compound (7.7 g, 84%). NMR (400.132 MHz, CDCl₃) 7.66 (d, 1H), 7.54 (s, 1H), 5.52 (d, 1H), 4.42 (q, 2H), 3.09 - 2.89 (m, 9H), 1.36 - 1.33 (m, 9H); m/z 236.

### Method 27

### 4-(3-Ethyl-2-isopropyl-3H-imidazol-4-yl)-pyrimidin-2-ylamine

(E)-3-Dimethylamino-1-(3-ethyl-2-isopropyl-3H imidazol-4-yl)-propenone (Method 26; 6.5 g, 0.028 mol) and guanidine carbonate (12.5 g, 0.069 mol) were added to butanol (100 ml) and heated at reflux for 5 days. The solvent was *removed in vacuo* to yield a yellow gum. Purification by column chromatography on silica using 0-5% MeOH in DCM gave the title compound as a yellow solid. DCM (5 ml) and ether (50 ml) were added then the suspension was filtered and dried to give the title compound as a white solid (5.0 g, 77%). NMR (400.132 MHz) 8.14 (d, 1H), 7.53 (s, 1H), 6.84 (d, 1H), 6.56 (brs, 2H), 4.54 (q, 2H), 3.13 (septet, 1H), 1.25 - 1.20 (m, 9H); m/z 232.

### Method 28

### Cyclopropanecarboxylic acid ethyl-(5-methyl-isoxazol-4-yl)-amide

Ethyl-(5-methyl-isoxazol-4-yl)-amine hydrochloride (15 g, 0.092 mol) was added to DCM (200 ml), to this was added TEA (32 ml, 0.23 mol) followed by the slow addition of cyclopropylcarbonylchloride (10.2g, 0.10 mol). The reaction was stirred for 30 minutes before the removal of the solvent *in vacuo.* The residue was then treated with water (150 ml), extracted with ether (3 x 150 ml), dried solvent removed *in vacuo* to yield a yellow oil (12.2 g, 69%). Used in Method 29 without further purification.

### Method 29

### N-{1-[1-Amino-meth-(Z)-ylidene]-2-oxo-propyl}-N-ethyl-cyclopropylamide

Cyclopropanecarboxylic acid ethyl-(5-methyl-isoxazol-4-yl)-amide (Method 28; 12.2 g, 0.08 mol) and 10% Pd on carbon (3.0 g) were added to EtOH (300 ml) and stirred at 4 atm over night, the reaction was filtered and solvent *removed in vacuo* to yield a off white solid. Ether (150 ml) was added, this was sonicated for 10 minutes before being filtered and dried to give a white solid. (9.2 g, 59%); m/z 197.

### Method 30

### 1-(3-Ethyl-2-cyclopropyl-3H-imidazol-4-yl)-ethanone

*N*-{1-[1-Amino-meth-(Z)-ylidene]-2-oxo-propyl}-*N*-ethyl-cyclopropylamide (Method 29; 9.2 g, 0.047 mol) and NaOH (2.3 g, 0.056 mol) were added to EtOH (150 ml) and heated at reflux for 4 hours. To the reaction was added solid NH₄C1 (4.4 g, 0.084 mol) and the reaction was stirred overnight. The resulting slurry was concentrated *in vacuo,* ether (200 ml) added, stirred for 10 minutes then filtered. The filtrate was *removed in vacuo* to yield an orange oil. This was distilled using bulb-to-bulb distillation (0.50 mbar/110°C) to give a clear oil (5.0 g, 60%). NMR (400.132 MHz, CDC1₃) 7.64 (s, 1H), 4.48 (q, 2H), 2.42 (s, 3H), 1.87 - 1.80 (m, 1H), 1.37 (t, 3H), 1.13 - 1.08 (m, 2H), 1.08 - 1.02 (m, 2H); m/z 179.

### Method 31

### (E)-1-(2-Cyclopropyl-3-ethyl-3H-imidazol-4-yl)-3-dimethylamino-propenone

1-(3-Ethyl-2-cyclopropyl-3H imidazol-4-yl)-ethanone (Method 30; 3.5 g, 0.020 mol) and DMFDMA (6.7 ml, 0.039 mol) were added to DMF (50 ml) and heated at 130°C for 6 hours. The solvent was *removed in vacuo* to yield a yellow solid. DCM (3.0 ml) was added followed by ether (50 ml) the reaction was sonicated for 10 minutes and then filtered. A yellow solid was obtained (3.4g; 72%). NMR (400.132 MHz, CDC1₃) 7.65 (d, 1H), 7.45 (s, 1H), 5.50 (d, 1H), 4.56 (q, 2H), 3.13-2.88 (m, 6H), 1.87-1.81 (m, 1H), 1.39 (t, 3H), 1.09 - 1.06 (m, 2H), 1.02 - 0.98 (m, 2H); m/z 234.

### Method 32

### 4-(3-Ethyl-2-cyclopropyl-3H-imidazol-4-yl)-pyrimidin-2-ylamine

(E)-1-(2-Cyclopropyl-3-ethyl-3H imidazol-4-yl)-3-dimethylamino-propenone (Method 31; 3.4 g, 0.015 mol) and guanidine carbonate (6.6 g, 0.036 mol) were added to butanol (60 ml) and heated at reflux for 4 days. The solvent was *removed in vacuo,* water (50 ml) was added and the residue was extracted with DCM (3 x 75 ml), dried and the solvent was removed *in vacuo* to yield an off white solid. DCM was added, followed by ether, the resulting solid was filtered and dried to give a white solid (2.75 g, 83%). NMR (400.132 MHz, CDC1₃) 8.19 (d, 1H), 7.95 (s, 1H), 6.83 (d, 1H), 4.94 (brs, 2H), 4.64 (q, 2H), 1.90 - 1.84 (m, 1H), 1.41 (t, 3H), 1.11 - 1.07 (m, 2H), 1.05 - 0.99 (m, 2H); m/z 230.

### Method 33

### N-Ethyl-2,2,2-trifluoro-N-(5-methyl-isoxazol-4-yl)-acetamide

Ethyl-(5-methyl-isoxazol-4-yl)-amine hydrochloride (15 g, 0.092 mol) was dissolved in pyridine (100 ml). To this was added trifluoroacetic anhydride (16.9 ml, 0.12 mol) and the reaction was stirred overnight before removal of the *solvent in vacuo.* The residue obtained was quenched with saturated NH₄C1 (200 ml), extracted with ether (3 x 200 ml), dried and solvent *removed in vacuo* to yield a yellow oil (18 g, 88%). NMR (400.132 MHz, CDCl₃) 8.03 (s, 1H), 3.55 (q, 2H), 2.26 (s, 3H), 1.05 (t,3H); m/z223.

### Method 34

### N-{1-[1-Amino-meth-(Z)-ylidene]-2-oxo-propyl}-N-ethyl-2,2,2-trifluoro-acetamide

N Ethyl-2,2,2-trifluoro-N (5-methyl-isoxazol-4-yl)-acetamide (Method 33; 18.0g, 0.081 mol) and 10%Pd on carbon (4.0 g) were reacted under a atmosphere of hydrogen at 4 atm for 3 days. The reaction was filtered and solvent *removed in vacuo* to yield an off white solid, DCM (30 ml) and ether (100 ml) were added. The reaction was stirred for 10 minutes, filtered and dried to give a white solid (11.6 g, 64%); m/z 225.

### Method 35

### 1-(3-Ethyl-2-trifluoromethyl-3H-imidazol-4-yl)-ethanone

*N*-{1-[1-Amino-meth-(Z)-ylidene]-2-oxo-propyl}-*N*-ethyl-2,2,2-trifluoro-acetamide (Method 34; 11.6 g, 0.051 mol) and potassium carbonate (14.4 g, 0.103 mol) were added to dioxane (180 ml) and heated at reflux for 2 hours. The reaction was cooled, filtered and solvent *removed in vacuo* to yield yellow oil. Purification by column chromatography on silica using 0-40% ether in iso-hexane gave the title compound as a clear oil (8.9 g, 85%). NMR (400.132 MHz, CDC1₃) 7.79 (s, 1H), 4.50 (q, 2H), 2.54 (s, 3H), 1.40 (t, 3H); m/z 207.

### Method 36

### (E)-3-Dimethylamino-l-(3-ethyl-2-trifluoromethyl-3H-imidazol-4-yl)-propenone

1-(3-Ethyl-2-trifluoromethyl-3H-imidazol-4-yl)-ethanone (Method 35; 7.0 g, 0.034 mol) and DMFDMA (11.6 ml, 0.068 mol) were added to DMF (90 ml) and heated at 130°C for 1 hour. The solvent was *removed in vacuo* to yield a yellow solid. Purification by column chromatography on silica using 0-5% MeOH in DCM gave the title product as a yellow solid. Ether was added followed by iso-hexane, the solid obtained filtered and dried to give the title compound. (7.6 g, 85%). NMR (400.132 MHz, CDC1₃) 7.74 (d, 1H), 7.55 (s, 1H), 5.53 (d, 1H), 4.57 (q, 2H), 3.17 (brs, 3H), 2.93 (brs, 3H), 1.42 (t, 3H); m/z 262.

### Method 37

### 4-(3-Ethyl-2-trifluoromethyl-3H-imidazol-4-yl)-pyrimidin-2-ylamine

(E)-3-Dimethylamino-1-(3-ethyl-2-trifluoromethyl-3H imidazol-4-yl)-propenone (Method 36; 6.0 g, 0.023 mol) and guanidine carbonate (8.3 g, 0.046 mol) were added to 2-methoxyethoxy ether (80 ml) and heated at 140°C for 2 days. The reaction was cooled and the solvent was removed in vacuo to yield a yellow solid. Water (100 ml) was added and the system was extracted with DCM (3 x 100 ml), dried and the solvent *removed in vacuo* to yield a yellow solid. Purification by column chromatography on silica using 0-5% MeOH in DCM gave the title compound as a yellow solid. Ether (20 ml) followed by iso-hexane (50 ml) were added to yield an off white solid which was filtered and dried (5.9 g, 100%); m/z 258.

### Method 38

### N-Ethyl-2,2-difluoro-N-(5-methyl-isoxazol-4-yl)-acetamide

Ethyl-(5-methyl-isoxazol-4-yl)-amine hydrochloride (15 g, 0.092 mol) and TEA were added to DCM (300 ml), this was cooled to 0°C before the slow addition of difluoroacetyl chloride (11.5 g, 0.10 mol). The reaction was stirred for 1 hour before the removal of the *solvent in vacuo.* The obtained residue was quenched with saturated NH₄C1 (200 ml), extracted with ether (3 x 200 ml), dried and solvent *removed in vacuo* to yield a yellow oil (9.0 g, 48%); m/z 203 (M-H)⁻.

### Method 39

### N-{1-[1-Amino-meth-(Z)-ylidene]-2-oxo-propyl}-N-ethyl-2,2-difluoro-acetamide

*N*-Ethyl-2,2-difluoro-*N*-(5-methyl-isoxazol-4-yl)-acetamide (Method 38; 9.0 g, 0.044 mol) was treated with 10% palladium on carbon (3.0 g) under 4 atm of pressure. The reaction was filtered and solvent *removed in vacuo,* DCM was added and the reaction was filtered to yield an off white solid (3.0 g, 33%); m/z 207.

### Method 40

### 1-(2-Difluoromethyl-3-ethyl-3H-imidazol-4-yl)-ethanone

*N*-{1-[1-Amino-meth-(Z)-ylidene]-2-oxo-propyl}-*N*-ethyl-2,2-difluoro-acetamide (Method 39; 3.0 g, 0.014 mol) and potassium carbonate (3.9 g, 0.028 mol) were added to dioxane (50 ml) and heated at reflux overnight. The reaction was filtered and the solvent removed *in vacuo* to yield a yellow oil. Purification by column chromatography on silica using ether as eluent gave the title compound as a yellow solid (2.4 g, 92%). NMR (400.132 MHz, CDC1₃) 7.74 (s, 1H), 6.78 (t, 1H), 4.54 (q, 2H), 2.51 (s, 3H), 1.40 (t, 3H); m/z 189.

### Method 41

### (E)-1-(2-Difluoromethyl-3-ethyl-3H-imidazol-4-yl)-3-dimethylamino-propenone

1-(2-Difluoromethyl-3-ethyl-3H-imidazol-4-yl)-ethanone (Method 40; 2.4 g, 0.013 3 mol) and DMFDMA (4.4 ml, 0.026 mol) were added to DMF (50 ml) and heated at 130°C for 20 minutes. The solvent was *removed in vacuo* to yield a yellow solid. DCM (3.0 ml) was added followed by ether (50 ml), sonicated for 10 minutes and then filtered. A yellow solid was obtained (2.7 g, 85%). NMR (400.132 MHz, CDC1₃) 7.71 (d, 1H), 7.52 (s, 1H), 6.75 (t, 1H), 5.52 (d, 1H), 4.61 (q, 2H), 3.19 - 2.88 (m, 6H), 1.42 (t, 3H); m/z 244.

### Method 42

### 4-(2-Difluoromethyl-3-ethyl-3H-imidazol-4-yl)-pyrimidin-2-ylamine

(E)-1-(2-Difluoromethyl-3-ethyl-3H imidazol-4-yl)-3-dimethylamino-propenone (Method 41; 2.7 g, 0.011 mol) and guanidine carbonate (4.0 g, 0.022 mol) were added to ethylene glycol diethyl ether (30 ml) and heated at 137°C for 2 days. The solvent was removed *in vacuo* to yield a yellow solid. DCM (5.0 ml) was added followed by ether (50 ml), the obtained solid was filtered and dried. A white solid was obtained (2.5 g, 96%). NMR (400.132 MHz) 8.27 (d, 1H), 7.72 (s, 1H), 7.23 (t, 1H), 6.97 (d, 1H), 6.71 (s, 2H), 4.70 (q, 2H), 1.30 (t, 3H); m/z 240.

### Method 43

### Cyclopropanecarboxylic acid {1-[1-amino-meth-(Z)-ylidene]-2-oxo-propyl}-isopropyl-amide

Cyclopropanecarboxylic acid isopropyl-(5-methyl-isoxazol-4-yl)-amide (Method 36 in WO03/076434; 18 g, 0.086 mol) and 10% palladium on carbon (3.0 g) in EtOH were reacted with hydrogen at 4 atm of pressure. The reaction was filtered and solvent *removed in vacuo* to yield a solid, ether was added and the solid was filtered (7.9 g, 44%); m/z 211.

### Method 44

### 1-(2-Cyclopropyl-3-isopropyl-3H-imidazol-4-yl)-ethanone

Cyclopropanecarboxylic {1-[1-amino-meth-(Z)-ylidene]-2-oxo-propyl}-isopropyl-amide (Method 43; 7.9 g, 0.038 mol) and sodium hydroxide (2.28 g, 0.057 mol) were added to EtOH (150 ml) and heated at reflux overnight. The solvent was removed in vacuo and the resulting solid was treated with saturated NH₄C1 (100 ml), extracted with ether (3 x 100 ml), dried and solvent removed *in vacuo* to yield a black oil. Purification by column chromatography on silica using 100% ether gave the title compound as a yellow oil (3.9 g, 53%). NMR (400.132 MHz, CDC1₃) 7.65 (s, 1H), 5.63 - 5.48 (m, 1H), 2.44 (s, 3H), 1.98 - 1.91 (m, 1H), 1.57 (d, 6H), 1.17 - 1.11 (m, 2H), 1.07 - 1.03 (m, 2H); m/z 193.

### Method 45

### (E)-1-(2-Cyclopropyl-3-isopropyl-3H-imidazol-4-yl)-3-dimethylamino-propenone

1-(2-Cyclopropyl-3-isopropyl-3H imidazol-4-yl)-ethanone (Method 44; 3.74 g, 0.019 mol) and DMFDMA (6.66 ml, 0.039 mol) were added to DMF and heated at 130°C for 4 hours. The solvent was *removed in vacuo* to yield an orange gum, DCM was added followed by ether to give the title compound as a yellow solid which was filtered and dried (4.5 g, 96%). NMR (400.132 MHz, CDC1₃) 7.63 (d, 1H), 7.40 (s, 1H), 5.61 (septet, 1H), 5.50 (d, 1H), 3.12 - 2.88 (m, 6H), 1.98 - 1.92 (m, 1H), 1.60 (d, 6H), 1.13 - 1.09 (m, 2H), 1.03 - 0.98 (m, 2H); m/z 248.

### Method 46

### 4-(2-Cyclopropyl-3-isopropyl-3H-imidazol-4-yl)-pyrimidin-2-ylamine /001 1

(E)-1-(2-Cyclopropyl-3-isopropyl-3*H* imidazol-4-yl)-3-dimethylamino-propenone (Method 45; 4.5 g, 0.019 mol) and guanidine carbonate (6.55 g, 0.036 mol) were added to ethylene glycol diethyl ether (75 ml) and heated at 142°C for 2 days. The solvent was removed *in vacuo,* water (100 ml) was added then extracted with DCM (3 x 150 ml), dried and the solvent removed *in vacuo* to yield a yellow solid. DCM was added followed by ether, the mixture was stirred for 30 minutes before being filtered and dried (3.6 g, 78%). NMR (400.132 MHz, CDCl₃) 8.22 (d, 1H), 7.28 (s, 1H), 6.79 (d, 1H), 5.57 (septet, 1H), 5.01 (brs, 2H), 2.03 - 1.96 (m, 1H), 1.64 (d, 6H), 1.17 - 1.13 (m, 2H), 1.05 - 1.00 (m, 2H); m/z 244.

### Method 47

### ((S)-3-Dimethylamino-pyrrolidin-1-yl)-(4-iodo-phenyl)-methanone

4-Iodobenzoyl chloride (5 g, 0.019 mol) and TEA (6.6 ml, 0.048 mol) were added to DCM (100 ml) and cooled to 0°C. To this was slowly added (S)-dimethylamino pyrrolidine (2.2 g, 0.019 mol), the reaction was stirred for 1 hour before the solvent was removed *in vacuo* to 90% volume. The slurry obtained was quenched with 2.0 M NaOH (50 ml), extracted with ether (3 x 200 ml), dried and solvent removed *in vacuo* to yield a yellow solid. Ether was added and the system was sonicated for 10 minutes and filtered. An off white solid was obtained (3.9 g, 60%). NMR (300.072 MHz, CDC1₃) 7.75 (d, 2H), 7.25 (d, 2H), 3.94 - 3.78 (m, 1H), 3.66 - 3.25 (m, 3H), 2.81 - 2.62 (m, 1H), 2.30 (s, 3H), 2.21 (s, 3H), 2.16 - 2.02 (m, 1H), 1.97 - 1.76 (m, 1H); m/z 345.

### Method 48

### ((R)-3-Dimethylamino-pyrrolidin-1-yl)₋(4-iodo-phenyl)-methanone

The title compound was prepared in a similar manner to Method 47 from 4-iodobenzoyl chloride and (R)-dimethylamino pyrrolidine (5.1 g, 78%). NMR (300.072 MHz, CDC1₃) 7.75 (d, 2H), 7.25 (d, 2H), 3.94 - 3.78 (m, 1H), 3.66 - 3.25 (m, 3H), 2.81 - 2.62 (m, 1H), 2.30 (s, 3H), 2.21 (s, 3H), 2.16 - 2.02 (m, 1H), 1.97 - 1.76 (m, 1H); m/z 345.

### Method 49

### Ethyl 4-{[4-(1-isopropyl-2-methyl-1H-imidazol-5-yl)pyrimidin-2-yl]amino}benzoate

To a solution of 4-(3-isopropyl-2-methyl-3H-imidazol-4-yl)-pyrimidin-2-ylamine (Method 14; 7.8 g) in dioxane (200 ml) was added ethyl 4-iodobenzoate (9.445 g), palladium (II) acetate (461 mg), Xantpos (1.785 g), and caesium carbonate (22.29 g). The mixture was degassed, and purged with nitrogen, then heated under reflux for 3 hours. The mixture was cooled to room temperature, the solids were removed by filtration, then the filtrate concentrated in vacuo. Purification on silica using 2-5 % MeOH in DCM as eluent gave the title compound as a yellow solid (3.82 g, 31%). NMR 9.87 (s, 1H), 8.46 (d, 1H), 7.90 - 7.83 (m, 4H), 7.45 (s, 1H), 7.14 (d, 1H), 5.72 - 5.63 (m, 1H), 4.27 (q, 2H), 2.49 (s, 3H), 1.47 (d, 6H), 1.30 (t, 3H); m/z 366.

### Method 50

### 4-{[4-(1-Isopropyl-2-methyl-1H-imidazol-5-yl)pyrimidin-2-yl]amino}benzoic acid sodium salt

Ethyl 4- {[4-(1-isopropyl-2-methyl-1*H*-imidazol-5-yl)pyrimidin-2-yl]amino}benzoate (Method 49; 3.82 g) was dissolved in THF (130 ml) then a solution of NaOH (419 mg) in water (20 ml) was added. The mixture was heated under reflux for 2 days. The mixture was concentrated in vacuo, then dissolved in water (400 ml) and washed with EtOAc (2 x 300 ml). The aqueous layer was concentrated in vacuo to yield the title compound as a white solid (3.53 g, 94%). NMR□9.43 (s, 1H), 8.38 (d, 1H), 7.79 (d, 2H), 7.56 (d, 2H), 7.41 (s, 1H), 7.03 (d, 1H), 5.82 - 5.72 (m, 1H), 2.49 (s, 3H), 1.44 (d, 6H); m/z 338.

### Method 51

### Ethyl 4-{[5-fluoro-4-(1-isopropyl-2-methyl-1H-imidazol-5-yl)pyrimidin-2-yl]amino}benzoate

To a solution of 5-fluoro-4-(3-isopropyl-2-methyl-3H-imidazol-4-yl)-pyrimidin-2-ylamine (Method 2; 5.32 g) in dioxane (100 ml) was added ethyl 4-iodobenzoate (3.59 g), palladium (II) acetate (305 mg), Xantphos (1.18 g), and caesium carbonate (14.74 g). The mixture was degassed, and purged with nitrogen, then heated under reflux for 3 hours. The mixture was cooled to room temperature, the solids were removed by filtration, then the filtrate concentrated in vacuo. Purification on silica using 2-5 % MeOH in DCM as eluent gave the title compound as a yellow solid (2.75 g, 32%). NMR 9.97 (s, 1H), 8.62 (d, 1H), 7.88 (d, 2H), 7.80 (d, 2H), 7.38 (d, 1H), 5.47 - 5.38 (m, 1H), 4.27 (q, 2H), 2.53 (s, 3H), 1.46 (d, 6H), 1.30 (t, 3H); m/z 384.

### Method 52

### 4-{[5-Fluoro-4-(1-isopropyl-2-methyl-1H-imidazol-5-yl)pyrimidin-2-yl]amino}benzoic acid lithium salt

To a stirred solution of ethyl 4-{[5-fluoro-4-(1-isopropyl-2-methyl-1H-imidazol-5-yl)pyrimidin-2-y1]amino}benzoate (Method 51; 2.75 g) in EtOH (70 ml) was added a solution of lithium hydroxide (301 mg) in water (15 ml). The mixture was heated under reflux for 18 hours, then concentrated in vacuo and partitioned between water (300 ml) and EtOAc (300 ml). The aqueous layer was washed with further EtOAc (200 ml) then concentrated in vacuo to yield the title compound as a white solid (2.07 g, 80%). NMR 9.56 (s, 1H), 8.53 (d, 1H), 7.80 (d, 2H), 7.53 (d, 2H), 7.36 (d, 1H), 5.56 - 5.46 (m, 1H), 2.51 (s, 3H), 1.43 (d, 6H); m/z 356.

### Method 53

### 1 ₋(4-Iodobenzoyl)pyrrolidin-3-ol

The title compound was prepared in similar manner to Method 10 from pyrrolinol and 4-iodobenzoyl chloride. NMR 7.78 (d, 2H), 7.28 (d, 2H), 4.96 (dd, 1H), 4.35-4.18 (br d, 1H), 3.60-3.15 (m, 4H overlapping water), 2.00-1.65 (m, 2H); m/z 318.

### Method 54

### (2Z)-3-(Dimethylamino)-2-fluoro-1-(1-cyclobutyl-2-methyl-1H-imidazol-5-yl)prop-2-en-1-one

The title compound was prepared in a similar manner to Method 1 by using (2E)-3-(dimethylamino)-1-(1-cyclobutyl-2-methyl-1*H*-imidazol-5-yl)prop-2-en-1-one (Method 37 in WO 03/076435) in place of (2E)-3-(dimethylamino)-1-(1-isopropyl-2-methyl-1H imidazol-5-yl)prop-2-en-1-one. NMR (300.074 MHz, CDC1₃) 7.27-7.17 (m, 1H), 6.85 (d, 1H), 5.06-4.91 (m, 1H), 3.12-3.05 (m, 6H), 2.54-2.39 (m, 7H), 1.74 (m, 2H) ; m/z 252.

### Method 55

### 5-Fluoro-4-(3-cyclobutyl-2-methyl-3H-imidazol-4-yl)-pyrimidin-2-ylamine

The title compound was prepared in a similar manner to Method 2 by using (2Z)-3-(dimethylamino)-2-fluoro-1-(1-cyclobutyl-2-methyl-1*H*-imidazol-5-yl)prop-2-en-1-one (Method 54) in place of (2*Z*)-3-(dimethylamino)-2-fluoro-1-(1-isopropyl-2-methyl-1*H* imidazol-5-yl)prop-2-en-1-one. NMR (300.074 MHz, CDC1₃) 8.26 (d, 1H), 7.21 (d, 1H), 6.58 (br.s, 1H), 5.17 (quintet, 1H), 3.45 (s, 3H), 2.42-2.29 (m, 4H), 1.80-1.64 (m, 2H); m/z 248.

### Method 56

### 1-(4-Iodobenzoyl)-4-methyl-1,4-diazepane

The title compound was prepared in similar manner to Method 10 from N-methylhomopiperazine and 4-iodobenzoyl chloride. NMR (400.132 MHz, CDC1₃) 7.79 (d, 2H), 7.18 (d, 2H), 3.84 - 3.79 (m, 2H), 3.54 - 3.52 (m, 1H), 3.48 (t, 1H), 2.79 - 2.77 (m, 1H), 2.70 - 2.66 (m, 1H), 2.62 - 2.55 (m, 2H), 2.45 (s, 3H), 2.07 - 2.00 (m, 1H), 1.91 - 1.84 (m, 1H); m/z 345.

### Method 57

### 1 -Isopropyl-1,4-diazepane

tert-Butyl 1,4-diazepane-1-carboxylate (17 g) and acetone (10 g) were added to MeOH (150 ml) and stirred at 0°C for 20 mins. NaCNBH₃ (6.4 g) was slowly added over a 20-minute period keeping the temperature below 0°C. After complete addition the reaction was allowed to warm up to ambient temperature and stirred over the weekend. The reaction was concentrated *in vacuo* to yield a yellow residue. This was quenched with water (100 ml), extracted with ether (3 x 100 ml), dried and solvent *removed in vacuo* to yield a viscous clear oil (20 g). The oil was added to TFA (50 ml) and DCM (50 ml), the reaction was stirred for 16 hours before concentration *in vacuo.* The reaction was quenched with water (30 ml), to this was added potassium carbonate until the aqueous was fully saturated, this was then extracted with EtOAc (3 x 200 ml), dried and solvent carefully *removed in vacuo* to yield the title compound as a yellow oil (5.2 g). NMR (400.132 MHz, CDC1₃) 2.94 - 2.86 (m, 5H), 2.68 - 2.63 (m, 4H), 1.74 - 1.68 (m, 2H), 1.01 (d, 6H).

### Method 58

### 1 -Benzoyl-4-isopropyl-1,4-diazepane

The title compound was prepared in similar manner to Method 10 from 1-isopropyl-1,4-diazepane (Method 57) and 4-iodobenzoyl chloride. NMR (400.132 MHz, CDC1₃) 7.74 (d, 2H), 7.13 (d, 2H), 3.75 - 3.72 (m, 2H), 3.40 (t, 2H), 2.96 - 2.84 (m, 1H), 2.79 - 2.77 (m, 1H), 2.67 (t, 1H), 2.62 - 2.56 (m, 2H), 1.92 - 1.87 (m, 1H), 1.71 - 1.67 (m, 1H), 1.03 - 0.97 (m, 6H); m/z 373.

### Method 59

### 1-(4-Bromo-2-methylbenzoyl)-4-methyl-1,4-diazepane

The title compound was prepared in similar manner to Method 4 from N-methylhomopiperazine and 4-bromo-2-methylbenzoic acid. NMR (400.132 MHz, CDC1₃) 7.38 (s, 1H), 7.34 (d, 1H), 7.04 (t, 1H), 3.86 - 3.76 (m, 2H), 3.34 (t, 1H), 3.27 (t, 1H), 2.75 - 2.73 (m, 1H), 2.69 - 2.50 (m, 2H), 2.50 - 2.43 (m, 1H), 2.37 (s, 3H), 2.30 (s, 3H), 1.99 (quintet, 1H), 1.80 (quintet, 1H) (rotamers); m/z 313.

### Method 60

### 1-(4-Bromo-2-fluorobenzoyl)-4-methyl-1,4-diazepane

The title compound was prepared in similar manner to Method 4 from N-methylhomopiperazine and 4-bromo-2-fluorobenzoic acid. NMR (400.132 MHz, CDC1₃) 7.35 (d, 1H), 7.30 (d, 1H), 7.24 (t, 1H), 3.83 - 3.77 (m, 2H), 3 .45 - 3.43 (m, 1H), 3.38 (t, 1H), 2.75 - 2.73 (m, 1H), 2.65 - 2.62 (m, 1H), 2.58 - 2.56 (m, 1H), 2.54 - 2.52 (m, 1H), 2.38 (d, 3H), 2.02 - 1.96 (m, 1H), 1.88 - 1.82 (m, 1H); m/z 317.

### Method 61

### (3S)-1-(4-Bromo-2-fluorobenzoyl)-N,N-dimethylpyrrolidin-3-anine

The title compound was prepared in similar manner to Method 4 from (3S)-*N,N-*dimethylpyrrolidin-3-amine and 4-bromo-2-fluorobenzoic acid. NMR (300.072 MHz, CDC1₃) 7.38 - 7.27 (m, 3H), 3.98 - 3.81 (m, 1H), 3.63 - 3.17 (m, 3H), 2.85 - 2.68 (m, 1H), 2.30 (s, 3H), 2.21 (s, 3H), 2.19 - 2.04 (m, 1H), 1.92 - 1.77 (m, 1H); m/z 316.

### Method 62

### (3R)-1-(4-Bromo-2-fluorobenzoyl)-N,N-dimethylpyrrolidin-3-amine

The title compound was prepared in similar manner to Method 4 from (3R)-N,N dimethylpyrrolidin-3-amine and 4-bromo-2-fluorobenzoic acid. NMR (300.072 MHz, CDC1₃) 7.38 - 7.27 (m, 3H), 3.97 - 3.81 (m, 1H), 3.63 - 3.17 (m, 3H), 2.84 - 2.68 (m, 1H), 2.30 (s, 3H), 2.21 (s, 3H), 2.17 - 2.02 (m, 1H), 1.90 - 1.77 (m, 1H); m/z 316.

### Method 63

### 4-(4-Bromo-2-methylbenzoyl)-1,4-oxazepane

The title compound was prepared in similar manner to Method 4 from 1,4-oxazepane and 4-bromo-2-methylbenzoic acid. NMR (400.132 MHz, CDC1₃) 7.39 (d, 1H), 7.35 (d, 1H), 7.05 (d, 1H), 3.90 - 3.80 (m, 4H), 3.76 (t, 1H), 3.64 - 3.57 (m, 1H), 3.36 - 3.32 (m, 2H), 2.30 (d, 3H), 2.03 (quintet, 1H), 1.75 (quintet, 1H); m/z 300.

### Method 64

### 4-(4-Bromo-2-fluorobenzoyl)-1,4-oxazepane

The title compound was prepared in similar manner to Method 4 from 1,4-oxazepane and 4-bromo-2-fluorobenzoic acid. NMR (400.132 MHz, CDC1₃) 7.36 (d, 1H), 7.33 - 7.29 (m, 1H), 7.24 (t, 1H), 3.87 - 3.80 (m, 4H), 3.76 (t, 1H), 3.66 (t, 1H), 3.46 - 3.42 (m, 2H), 2.04 (quintet, 1H), 1.82 (quintet, 1H); m/z 304.

### Method 65

### 3-(4-Iodobenzoyl)-8-oxa-3-azabicyclo[3.2.1]octane

The title compound was prepared in similar manner to Method 10 from 8-oxa-3-azabicyclo[3.2.1]octane and 4-iodobenzoyl chloride. NMR (400.132 MHz, CDC1₃) 7.76 (d, 2H), 7.12 (d, 2H), 4.50 - 4.16 (m, 3H), 3.50 - 3.24 (m, 2H), 3.19 - 3.06 (m, 1H), 2.06 - 1.82 (m, 3H), 1.77 - 1.48 (m, 1H) (rotamers); m/z 344.

### Method 66

### (3S)-1-(4-Bromo-2-methylbenzoyl)-N,N-dimethylpyrrolidin-3-amine

The title compound was prepared in similar manner to Method 4 from (3S)-*N,N-*dimethylpyrrolidin-3-amine and 4-bromo-2-methylbenzoic acid. NMR (300.072 MHz, CDC1₃) 7.39 (s, 1H), 7.35 (d, 1H), 7.06 (d, 1H), 4.00 - 3.84 (m, 1H), 3.61 - 3.35 (m, 1H), 3.31 - 3.23 (m, 1H), 3.18 - 2.95 (m, 1H), 2.80 - 2.63 (m, 1H), 2.29 (s, 6H), 2.19 - 2.03 (m, 4H), 1.90 - 1.74 (m, 1H); m/z 312.

### Method 67

### (3R)-1-(4-Bromo-2-methylbenzoyl)-N,N-dimethylpyrrolidin-3-amine

The title compound was prepared in similar manner to Method 4 from (3R)-*N,N* dimethylpyrrolidin-3-amine and 4-bromo-2-methylbenzoic acid. NMR (300.072 MHz, CDC1₃) 7.39 (s, 1H), 7.35 (d, 1H), 7.07 (dd, 1H), 4.00 - 3.84 (m, 1H), 3.61 - 3.35 (m, 1H), 3.31 - 2.95 (m, 2H), 2.81 - 2.64 (m, 1H), 2.30 (s, 6H), 2.19 - 2.02 (m, 4H), 1.90 - 1.76 (m, 1H); m/z 312.

### Method 68

### (4-Bromo-2-chloro-phenyl)-(4-methyl-1,4-diazepan-1-yl)methanone

The title compound was prepared in similar manner to Method 4 from N-methylhomopiperazine and 4-bromo-2-chlorobenzoic acid except purification was by distillation under reduced pressure (180°C @ 0.80 mmHg). NMR 7.73 (d, 1H), 7.59 (dd, 1H), 7.28 (d, 1H), 3.71 - 3.63 (m, 2H), 3.28 - 3.19 (m, 2H), 2.95 - 2.89 (m, 1H), 2.69 - 2.64 (m, 1H), 2.62 - 2.52 (m, 2H), 2.29 (d, 3H), 1.90 - 1.82 (m, 1H), 1.75 - 1.67 (m, 1H); m/z 332.

### Method 69

### (4-Bromo-2-chloro-phenyl)-(1,4-oxazepan-4-yl)methanone

The title compound was prepared in similar manner to Method 4 from 1,4-oxazepane and 4-bromo-2-chlorobenzoic acid except purification was by distillation under reduced pressure (182°C *@* 0.58 mmHg). NMR 7.74 (s, 1H), 7.60 (d, 1H), 7.31 (d, 1H), 3.79 - 3.66 (m, 5H), 3.61 - 3.55 (m, 1H), 3.34 - 3.25 (m, 2H), 1.95 - 1.87 (m, 1H), 1.77 - 1.69 (m, 1H); m/z 319.

### Method 70

### (4-Iodophenyl)-[(1S,4S)-2-propyl-2,5-diazabicyclo[2.2.1]hept-5-yl]methanone

The title compound was prepared in similar manner to Method 10 from (1S,4S)-2-propyl-2,5-diazabicyclo[2.2.1]heptane and 4-iodobenzoyl chloride. NMR 7.80 (d, 2H), 7.27 (d, 2H), 3.48 (s, 1H), 3.45 - 3.38 (m, 1H), 3.32 (dd, 1H), 2.93 (s, 1H), 2.83 (d, 1H), 2.61 (d, 1H), 2.54 - 2.39 (m, 2H), 1.77 (d, 1H), 1.68 (d, 1H), 1.40 (sextet, 2H), 0.87 (t, 3H); m/z 372.

### Method 71

### [4-(2-Hydroxyethyl)-1,4-diazepan-1-yl]-(4-iodophenyl)methanone

The title compound was prepared in similar manner to Method 10 from 2-(1,4-diazepan-1-yl)ethanol and 4-iodobenzoyl chloride. NMR (400.132 MHz, CDC1₃) 7.75 (d, 2H), 7.13 (d, 2H), 3.80 - 3.72 (m, 2H), 3.62 - 3.50 (m, 2H), 3.50 - 3.42 (m, 2H), 2.92 - 2.81 (m, 1H), 2.81 - 2.60 (m, 6H), 2.01 - 1.92 (m, 1H), 1.83 - 1.74 (m, 1H); m/z 375.

### Method 72

### (4-Iodophenyl)-(1,4-oxazepan-4-yl)methanone

The title compound was prepared in similar manner to Method 10 from 1,4-oxazepane and 4-iodobenzoyl chloride. NMR (400.132 MHz, CDC1₃) 7.76 (d, 2H), 7.14 (d, 2H), 3.89 - 3.72 (m, 5H), 3.69 - 3.59 (m, 1H), 3.54 - 3.45 (m, 2H), 2.09 - 1.97 (m, 1H), 1.87 - 1.75 (m, 1H); m/z 332.

### Method 73

### N-(5-Methyl-1,2-oxazol-4-yl)cyclobutanecarboxamide

Cyclobutyl carbonyl chloride (26.7 ml) was added dropwise to a stirred solution of 5-methyl-1,2-oxazol-4-amine hydrochloride (30g) and TEA (80 ml) in DCM (450 ml) at ambient temperature. The reaction mixture was stirred for 30 min then washed with water (150 ml), 10% aq. citric acid (2 x 100 ml), sat. aq. NaHCOₛ (2 x 100 ml). The aqueous layers were reextracted with DCM (2 x 100 ml), the combined organic extracts dried (Na₂SO₄), filtered and concentrated *in vacuo.* The residue was triturated with ether (250 ml), filtered and dried to give the title compound as a beige solid (35.3 g). NMR (300.072 MHz, CDC1₃) 8.52 (s, 1H), 6.60 (br.s, 1H), 3.14 (quintet, 1H), 2.45-2.16 (m, 5H), 2.11-1.84 (m, 2H); m/z 181.

### Method 74

### N-(Cyclobutylmethyl)-5-methyl-1,2-oxazol-4-amine hydrochloride

Borane dimethyl sulphide complex (200 ml of a 2M solution in THF) was added over 30 mins to a stirred solution of N-(5-methyl-1,2-oxazol-4-yl)cyclobutanecarboxamide (Method 73; 32.7 g) in THF (150 ml) under an inert atmosphere at ambient temperature. The reaction mixture was stirred for 30 mins at ambient temperature then heated under reflux (caution: exotherm and effervescence) for 2 hrs. The reaction mixture was cooled to 0°C and MeOH added cautiously before stirring at ambient temperature for 3 hrs. 4M HC1 in dioxane (55 ml) was added dropwise, stirred for 1 hr then the solvent *removed in vacuo.* Ether was added, the resultant solid filtered and dried to give the title compound as a colourless solid (36.9 g). NMR 8.68 (s, 1H), 3.20 (d, 2H), 2.58 (m, 1H), 2.49 (s, 3H), 2.06-1.92 (m, 2H), 1.88-1.64 (m, 4H); m/z 166.

### Method 75

### N-(Cyclobutylmethyl -N-(5-methyl-1,2-oxazol-4-yl)acetamide

Acetic anhydride (35 ml) was added over 30 mins to a stirred suspension ofN-(cyclobutylmethyl)-5-methyl-1,2-oxazol-4-amine hydrochloride (Method 74; 37.3 g) and sodium acetate (15.1 g) in acetic acid (250 ml). The reaction mixture was stirred for 16 hrs at ambient temperature then concentrated *in vacuo.* The residue was stirred with 10% aq. potassium carbonate (400 ml) for 1 hr then extracted with DCM (1 x 300 ml, 2 x 100 ml). The organic extracts were washed with brine (100 ml), dried (Na₂SO₄) and concentrated *in vacuo* to give the title compound as a yellow oil (36.2g). NMR (300.072 MHz, CDC1₃) 8.08 (s, 1H), 3.63 (d, 2H), 2.41 (m, 1H), 2.34 (s, 3H), 2.05-1.80 (m, 7H), 1.74-1.57 (m, 2H); m/z 209.

### Method 76

### N-[(E)-1-Amino-3-oxo-but-1-en-2-yl]-N-(cyclobutylmethyl)acetamide

N-(Cyclobutylmethyl)-N-(5-methyl-1,2-oxazol-4-yl)acetamide (Method 75; 36.0 g) and 10% Pd on carbon (8.0 g) in EtOH (360 ml) was stirred under a hydrogen atmosphere at 4 atmospheres pressure for 16 hrs. The reaction mixture was filtered through diatomaceous earth with 10% MeOH in DCM and the solvent *removed in vacuo.* The residue obtained was triturated with ether and dried under vacuum to give the title compound as a colourless solid (31.4 g). NMR 7.46 (m, 1H), 6.95 (br., 1H), 6.66 (br., 1H), 3.39-3.16 (m, 2H), 2.29 (m, 1H), 2.03 (s, 1H), 1.95-1.79 (m, 2H), 1.75-1.63 (m, 2H), 1.61 (s, 3H), 1.60-1.47 (m, 2H); m/z 211 1

### Method 77

### (E)-1-[3-(Cyclobutylmethyl)-2-methyl-imidazol-4-yl]-3-dimethylamino-prop-2-en-1-one

N-[(E)-1-Amino-3-oxo-but-1 -en-2-yl]-N-(cyclobutylmethyl)acetamide (Method 76; 33.4 g) and NaOH (7.63 g) were added to EtOH (250 ml) and heated at reflux for 3 hrs. The reaction mixture was concentrated *in vacuo,* aqueous ammonium chloride (200 ml) added and the aqueous layer was extracted with ether (3 x 300 ml). The combined organics were dried and the solvent *removed in vacuo* to give an orange oil (30 g) which was distilled (106°C @ 0.69 mbar). The colourless oil (30 g) obtained and DMFDMA (53 ml) were added to DMF (250 ml) and heated at 130°C for 4 hrs. The solvent was *removed in vacuo* to give a yellow solid. DCM (20 ml) was added followed by ether (100 ml), sonicated for 10 mins and then filtered to give the title compound as a yellow solid (35.3 g). NMR (400.132 MHz, CDC1₃) 7.65 (d, 1H), 7.49 (s, 1H), 5.52 (d, 1H), 4.41 (d, 2H), 3.07 - 2.89 (m, 6H), 2.72 (septet, 1H), 2.42 (s, 3H), 2.01 - 1.92 (m, 2H), 1.86 - 1.70 (m, 4H); m/z 248.

### Method 78

### 4-[3-(Cyclobutylmethyl)-2-methyl-imidazol-4-yl]pyrimidin-2-amine

(E)-1-[3-(Cyclobutylmethyl)-2-methyl-imidazol-4-yl]-3-dimethylamino-prop-2-en-1-one (Method 77; 1.5 g) and guanidine (2.6 g) were heated under reflux in n-butanol (60 ml) for 30 hrs. The solvent was *removed in vacuo* to yield a yellow solid that was quenched with water (60 ml) then extracted with DCM (3 x 70 ml). The combined organics were dried and concentrated *in vacuo* to give a yellow solid, which was dissolved in the minimum amount of hot DCM and allowed to cool. The solid obtained was filtered and dried to give the title compound as a colourless solid (1.35 g). NMR 8.17 (d, 1H), 7.23 (s, 1H), 6.74 (d, 1H), 6.57 (s, 2H), 5.38 (quintet, 1H), 2.51 - 2.35 (m, 7H), 1.83 - 1.69 (m, 2H); m/z 230.

### Method 79

### N-Cyclopentyl-5-methyl-1,2-oxazol-4-amine

5-Methyl-1,2-oxazol-4-amine hydrochloride (20 g), cyclopentanone (13.9 ml) and sodium acetate (12.3 g) were added to MeOH (200 ml) and stirred at 0°C for 1 hr. NaCNBH₃ (11.5 g) was slowly added over 20 mins, whilst maintaining the temperature below 0°C. After complete addition the reaction mixture was warmed to ambient temperature and stirred for 16 hrs before the solvent was removed *in vacuo.* The solid obtained was dissolved in saturated aq. NH₄C1 (100 ml) and extracted with ether (2 x 200 ml then 1 x 100 ml). The combined organic extracts were dried, filtered and the solvent *removed in vacuo* to give a yellow oil. The oil was purified by column chromatography on silica using 10-50% ether in isohexane as eluent. The solvent was *removed in vacuo* to give the title compound as a yellow oil (17.2 g). NMR (300.072 MHz, CDC1₃) 8.03 (s, 1H), 3.51 (quintet, 1H), 2.31 (s, 3H), 1.94 - 1.83 (m, 2H), 1.79 - 1.54 (m, 4H), 1.47 - 1.37 (m, 2H); m/z 167.

### Method 80

### N-Cyclopentyl-N-(5-methyl-1,2-oxazol-4-yl)acetamide

Acetic anhydride (18.9 ml) was added portionwise over 20 mins to a stirred solution of N-cyclopentyl-5-methyl-1,2-oxazol-4-amine (Method 79; 16.0 g) in acetic acid (160 ml). After 1 hr the solvent was *removed in vacuo* and the resulting slurry was treated with aqueous K₂CO₃ (50 ml, caution: CO₂ evolved). The aqueous layer was extracted with DCM (3 x 50 ml), the combined organics dried (Na₂SO₄) and the solvent was removed *in vacuo.* The solid obtained was dried under high vacuum to give the title compound as a yellow solid (19.0g). NMR 8.64 (s, 1H), 4.77 (quintet, 1H), 2.33 (s, 3H), 1.80-1.74 (m, 2H), 1.69 (s, 3H), 1.51-1.41 (m, 4H), 1.24-1.08 (m, 2H); m/z 209.

### Method 81

### N-[(E)-1-Amino-3-oxo-but-1-en-2-yl]-N-cyclopentyl-acetamide

The title compound was prepared in similar manner to Method 76 from N-cyclopentyl-N-(5-methyl-1,2-oxazol-4-yl)acetamide (Method 80) to give a colourless solid (16.0 g). NMR 7.59 (t, 1H), 6.84 (d, 2H), 4.44 (quintet, 1H), 2.06 (s, 3H), 1.80-1.59 (m, 5H), 1.49-1.19 (m, 6H); m/z 211.

### Method 82

### 1-(3-Cyclopentyl-2-methyl-imidazol-4-yl)ethanone

N-[(E)-1-Amino-3-oxo-but-1-en-2-yl]-N-cyclopentyl-acetamide (Method 81; 16.0 g) and NaOH (3.66 g) were added to EtOH (200 ml) and heated under reflux for 4 hrs. NH₄C1 (6.11 g) was added and the mixture was stirred for 16 hrs at ambient temperature then concentrated *in vacuo.* Ether (350 ml) was added, the mixture stirred for 10 mins then filtered and concentrated *in vacuo.* The yellow oil obtained was distilled under reduced pressure (0.55 mbar/100°C) to give the title compound as a clear oil (10.08 g). NMR (400.132 MHz, CDC1₃) 7.73 (s, 1H), 5.22 (quintet, 1H), 2.50 (s, 3H), 2.45 (s, 3H), 2.04- 1.97 (m, 6H), 1.71-1.66 (m, 2H); m/z 193.

### Method 83

### (E)-1-(3-Cyclopentyl-2-methyl-imidazol-4-yl)-3-dimethylamino-prop-2-en-1-one

1-(3-Cyclopentyl-2-methyl-imidazol-4-yl)ethanone (Method 82; 10.08 g) and DMFDMA (17.9 ml) were added to DMF (150 ml) and heated at 130°C for 6 hrs. The solvent was *removed in vacuo* and DCM (10 ml) was added followed by ether (100 ml). The mixture was sonicated for 10 mins before filtering and drying to give the title compound as a yellow solid (9.74 g). NMR (400.132 MHz, CDC1₃) 7.62 (d, 1H), 7.48 (s, 1H), 5.52 (d, 1H), 5.35 (quintet, 1H), 2.99 (s, 6H), 2.49 (s, 3H), 2.14-1.91 (m, 6H), 1.72-1.61 (m, 2H); m/z 248.

### Method 84

### 4-(3-Cyclopentyl-2-methyl-imidazol-4-yl)pyrimidin-2-amine

(E)-1-(3-Cyclopentyl-2-methyl-imidazol-4-yl)-3-dimethylamino-prop-2-en-1-one (Method 83; 3.00 g) and guanidine carbonate (4.38 g) were added to 2-methoxy ethanol (50 ml) and heated at 140°C for 36 hrs. The reaction mixture was cooled and the solvent was *removed in vacuo* to give a yellow solid. Water (50 ml) was added and the system was extracted with DCM (3 x 50 ml), the combined organics dried and the solvent removed in *vacuo.* The yellow solid obtained was triturated with DCM followed by ether then filtered and dried under vacuum to yield the title compound as an off white solid (2.46 g). NMR (400.132 MHz, CDC1₃) 8.23 (d, 1H), 7.33 (s, 1H), 6.80 (d, 1H), 5.41 (quintet, 1H), 5.01 (s, 2H), 2.54 (s, 3H), 2.17-2.02 (m, 4H), 1.97-1.87 (m, 2H), 1.74-1.64 (m, 2H); m/z 244.

### Method 85

### (3S)-3-Pyrrolidin-1-ylpyrrolidine

tert-Butyl (3R)-3-methylsulfonyloxypyrrolidine-1-carboxylate (JCS Perkin Transactions 1, 1993, 13, 1421-4; 26 g, 0.098 mol) and pyrrolidine (15.3 g, 0.215 mol) were added to DMF and heated at 80°C for 36 hrs. The solvent was *removed in vacuo* to yield a brown liquid. Distillation under reduced pressure (200°C at 1.0 mmHg) removed volatile material and the resulting gum was quenched with aqueous NaOH (50 ml). The aqueous layer was saturated with solid potassium carbonate then extracted with DCM (3 X 200 ml). The combined organics were dried and the solvent *removed in vacuo* to yield a brown oil. Purification by column chromatography with 0-50% MeOH in DCM as eluent gave a brown oil. This was distilled under reduced pressure (40°C*@* 0.56 mmHg) to give the title compound as a clear oil. NMR (400.132 MHz, CDC1₃) 3.08 - 3.01 (m, 2H), 2.94 - 2.88 (m, 1H), 2.82 - 2.78 (m, 1H), 2.64 (quintet, 1H), 2.55 - 2.48 (m, 4H), 2.25 (s, 1H), 1.97 - 1.89 (m, 1H), 1.82 - 1.75 (m, 4H), 1.73 - 1.64 (m, 1H).

### Method 86

### (4-Iodophenyl)-[(3S)-3-pyrrolidin-1-yipyrrolidin-1-yl]methanone

The title compound was prepared in similar manner to Method 10 from (3S)-3-pyrrolidin-1-ylpyrrolidine (Method 85) and 4-iodobenzoyl chloride. NMR (400.132 MHz, CDC1₃; rotamers) 7.75 (d, 2H), 7.25 (d, 2H), 3.92 - 3.78 (m, 1H), 3.65 - 3.33 (m, 3H), 2.91 - 2.68 (m, 1H), 2.61 - 2.42 (m, 4H), 2.21 - 2.00 (m, 1H), 1.99 - 1.89 (m, 1H), 1.87 - 1.75 (m, 4H); m/z 371.

### Method 87

### [(3R)-3-Hydroxypyrrolidin-1-yl]-(4-iodophenyl)methanone

4-Iodobenzoyl chloride (20 g) in DCM (200 ml) was added dropwise to a solution of (3R)-pyrrolidin-3-ol (6.9 g) and TEA (23 ml) in DCM (300 ml). The reaction was stirred for 1 hr then sat. aq. NH₄C1 (200 ml) was added. The aqueous layer was extracted with DCM (3 x 200 ml), the combined organics dried and the solvent *removed in vacuo* to give a yellow solid. The crude product was dissolved in a minimum amount of hot acetonitrile which was then allowed to cool, filtered and dried to give the title compound as a pale yellow solid (21.2 g). NMR 7.77 (d, 2H), 7.28 (d, 2H), 4.63 (d, 1H), 4.34 - 4.22 (m, 1H), 3.62 - 3.50 (m, 2H), 3.49 - 3.39 (m, 1H), 3.34 - 3.22 (m, 1H), 2.00 - 1.91 (m, 1H), 1.85 - 1.76 (m, 1H); m/z 318.

### Method 88

### (4-Iodophenyl)-[(3R)-3-methylsuhbnyloxypyrrolidin-1-yl]methanone

Methanesulfonyl chloride (5.25 ml) in DCM (20 ml) was added dropwise to a solution of [(3R)-3-hydroxypyrrolidin-1-yl]-(4-iodophenyl)methanone (Method 87; 19.5 g) and TEA (12.8 ml) in DCM (200 ml). The reaction mixture was stirred for 1 hr then sat. aq. NH₄CI (150 ml) was added. The aqueous layer was extracted with DCM (3 x 200 ml), dried and the solvent *removed in vacuo* to yield a yellow solid. This was dissolved in a minimum amount of hot acetonitrile and ether was added to precipitate a colourless solid. Additional ether was added, and then the suspension was filtered and dried to give the title compound as a colourless solid. NMR 7.81 (d, 2H), 7.31 (d, 2H), 5.30 - 5.26 (m, 1H), 3.83 - 3.80 (dd, 1H), 3.70 - 3.63 (m, 1H), 3.60 - 3.57 (m, 2H), 3.16 (s, 3H), 2.31 - 2.15 (m, 2H); m/z 396.

### Method 89

### [(3S)-3-(Cyclopropylamino)pyrrolidin-1-yl]-(4-iodophenyl)methanone

4-Iodophenyl)-[(3R)-3-methylsulfonyloxypyrrolidin-1-yl]methanone (Method 88; 3.0 g) and cyclopropane amine (4.4 g) were added to dioxane (40 ml) and heated at 109°C in a sealed tube for 5 days. Sat. aq. K₂CO₃ (50 ml) was added and the aqueous layer extracted with ether (3 x 100 ml). The combined organics were dried and concentrated *in vacuo* to give a yellow gum. Purification on silica eluting with 0-5% MeOH in DCM gave the title compound as a viscous yellow oil (1.72g). NMR (400.132 MHz, CDC1₃) 7.37 (d, 2H), 6.89 (d, 2H), 3.49 - 3.35 (m, 1H), 3.29 - 3.15 (m, 2H), 3.09 - 2.87 (m, 2H), 1.83 - 1.75 (m, 1H), 1.70 - 1.63 (m, 1H), 1.49 - 1.41 (m, 1H), 1.31 - 1.18 (m, 1H), 0.13 - -0.17 (m, 4H); m/z 357.

### Method 90

### [(3S)-3-(Cyclopropylamino)pyrrolidin-1-yl]-(4-iodophenyl)methanone

(4-Iodophenyl)-[(3R)-3-methylsulfonyloxypyrrolidin-1-yl]methanone (Method 88; 4.0 g) and methylamine (2.0 M in THF; 50 ml) were heated at 150°C under microwave irradiation for 4 hrs. The reaction was concentrated *in vacuo,* aqueous K₂C0₃ (50 ml) added then extracted with DCM (3 x 100 ml). The combined organics were dried and solvent removed *in vacuo* to give an orange gum. Purification on silica eluting with 0-20% MeOH in DCM gave the title compound as a yellow gum. NMR (400.132 MHz, CDC1₃) 7.75 (d, 2H), 7.28 - 7.24 (m, 2H), 3.83 - 3.31 (m, 4H), 3.25 - 3.19 (m, 1H), 2.47 - 2.37 (m, 3H), 2.21 - 1.98 (m, 1H), 1.84 - 1.74 (m, 1H); m/z 331.

### Method 91

### [(3S)-3-(Cyclobutylanmino)pyrrolidin-1-yl]-(4-iodophenyl)methanone

The title compound was prepared in similar manner to Method 89 from (4-iodophenyl)-[(3R)-3-methylsulfonyloxypyrrolidin-1-yl]methanone (Method 88) and cyclobutanamine; NMR (500.133 MHz, DMSO) 7.78 (d, 2H), 7.27 (d, 2H), 3.57 - 3.48 (m, 2H), 3.43 - 3.34 (m, 1H), 3.26 (quintet, 1H), 3.22 - 3.11 (m, 3H), 2.16 - 2.04 (m, 2H), 1.96 (sextet, 1H), 1.72 - 1.52 (m, 5H); m/z 371.

### Method 92

### (4-lodophenyl)-[(3S)-3-(methyl-propyl-amino)pyrrolidin-1-yl]methanone

The title compound was prepared in similar manner to Method 89 from (4-iodophenyl)-[(3R)-3-methylsulfonyloxypyrrolidin-1-yl]methanone (Method 88) and N-methylpropan-1-amine; NMR (500.133 MHz, DMSO) 7.79 (d, 2H), 7.27 (d, 2H), 3.63 - 3.49 (m, 2H), 3.44 - 3.38 (m, 1H), 3.25 (dd, 1H), 3.05 (quintet, 1H), 2.37 - 2.27 (m, 2H), 2.16 (s, 3H), 2.04 - 1.98 (m, 1H), 1.82 - 1.74 (m, 1H), 1.42 (sextet, 2H), 0.84 (t, 3H); m/z 373.

### Method 93

### (4-Bromophenyl)-[(3R)-3-methylsulfonyloxypyrrolidin-1-yl]methanone

4-Bromobenzoyl chloride (15 g) in DCM (200 mL) was added slowly via a dropping funnel to a stirred solution of (3R)-pyrrolidin-3-ol (6.0 g) and TEA (21 mL) in DCM (300 mL). The reaction was stirred for 1 hr before adding satd. NH₄C1 (200 mL) and extraction with DCM (3 x 200 mL). The combined organics were dried and concentrated *in vacuo* to give a yellow solid. The solid was dissolved in a minimum amount of hot acetonitrile, cooled and then filtered to give a pale yellow solid. The isolated solid (13.4g) and TEA (10.3 mL) were dissolved in DCM (200 mL) and then methanesulfonyl chloride (4.54 mL) in DCM (20 mL) added using a dropping funnel. The reaction was stirred for 1 hr before adding satd. NH₄C1 (150 mL) and extracting with DCM (3 x 200 mL). The combined organics were dried and concentrated *in vacuo* to give a yellow gum. Acetonitrile and diethyl ether were then added, the mixture heated and then sonicated to give the title compounds as an off white solid (16 g); NMR (400.132 MHz, CDC1₃, rotamers) 7.56 (d, 2H), 7.44 - 7.37 (m, 2H), 5.38 - 5.25 (m, 1H), 3.94 - 3.59 (m, 4H), 3.13 - 3.02 (m, 3H), 2.39 - 2.35 (m, 1H), 2.31 - 2.11 (m, 1H); m/z 349.

### Method 94

### (4-Bromophenyl)-[(3S)-3 -diethylaminopyrrolidin-1-yl]methanone

(4-Bromophenyl)-[(3R)-3-methylsulfonyloxypyrrolidin-1-yl]methanone (Method 93; 2.5 g) and diethylamine (10 g) were added to dioxane (30 mL) and heated at 100°C until complete consumption of the starting material was observed. The reaction mixture was concentrated *in vacuo* then loaded onto a 50g SCX column in MeOH before eluting with MeOH then 7N NH₃ in MeOH. Purification on silica eluting with 0 to 2.5% MeOH in DCM gave the title compound as an orange gum (1.1 g); NMR (500.133 MHz, DMSO) 7.60 (d, 2H), 7.43 (d, 2H), 3.63 - 3.49 (m, 2H), 3.44 - 3.38 (m, 1H), 3.30 - 3.20 (m, 2H), 2.58 - 2.50 (m, 4H), 2.05 - 1.98 (m, 1H), 1.80 - 1.73 (m, 1H), 0.96 (t, 6H); m/z 326.

### Method 95

### [(3S)-3-(Azepan-1-yl)pyrrolidin-1-yl]-(4-bromophenyl)methanone

The title compound was prepared in similar manner to Method 94 from (4-bromophenyl)-[(3R)-3-methylsulfonyloxypyrrolidin-1-yl]methanone (Method 93) and azepane; NMR (500.133 MHz, DMSO) 7.60 (d, 2H), 7.43 (d, 2H), 3.64 - 3.49 (m, 2H), 3.43 - 3.38 (m, 1H), 3.28 - 3.21 (m, 2H), 2.66 - 2.57 (m, 4H), 2.06 - 2.00 (m, 1H), 1.80 - 1.72 (m, 1H), 1.61 - 1.52 (m, 8H); m/z 353.

### Method 96

### (4-Bromophenyl)-[(3S)-3-(2-methoxyethyl-methyl-anmino)pyrrolidin-1-yl]methanone

The title compound was prepared in similar manner to Method 94 from (4-bromophenyl)-[(3R)-3-methylsulfonyloxypyrrolidin-1-yl]methanone (Method 93) and 2-methoxy-N-methyl-ethanamine; NMR (500.133 MHz, DMSO) 7.60 (d, 2H), 7.43 (d, 2H), 3.64 - 3.49 (m, 2H), 3.41 (m, 3H), 3.28 - 3.24 (m, 4H), 3.12 (quintet, 1H), 2.63 - 2.53 (m, 2H), 2.23 (s, 3H), 2.05 - 1.99 (m, 1H), 1.82 - 1.74 (m, 1H); m/z 343.

### Method 97

### (4-Bromophenyl)-[(3S)-3-(methyl-(2-methylpropyl)amino)pyrrolidin-1-yl]methanone

The title compound was prepared in similar manner to Method 94 from (4-bromophenyl)-[(3R)-3-methylsulfonyloxypyrrolidin-1-yl]methanone (Method 93) and N,2-dimethylpropan-1-amine; NMR (500.133 MHz, DMSO) 7.60 (d, 2H), 7.43 (d, 2H), 3.62 - 3.50 (m, 2H), 3.44 - 3.38 (m, 1H), 3.28 - 3.25 (m, 1H), 3.04 (quintet, 1H), 2.16 (s, 3H), 2.14 - 1.98 (m, 3H), 1.82 - 1.66 (m, 2H), 0.84 (d, 6H); m/z 340.

### Method 98

### (4-Bromophenyl)-[(3S)-3-(propan-2-ylamino)pyrrolidin-1-yl]methanone

(4-Bromophenyl)-[(3R)-3-methylsulfonyloxypyrrolidin-1-yl]methanone (Method 93; 2.5 g) and propan-2-amine (3.12 g) were dissolved in dioxane (50 mL) and heated at 150°C under microwave irradiation for 8 hrs. The reaction mixture was concentrated *in vacuo,* dissolved in MeOH and loaded onto a 50g SCX column eluting with MeOH then 7N NH₃ in

MeOH. Purification on silica, eluting with 0-5% MeOH in DCM gave the title compound as an orange gum (1.6 g); NMR (500.133 MHz, DMSO) 7.60 (d, 2H), 7.43 (d, 2H), 3.63 - 3.49 (m, 2H), 3.45 - 3.33 (m, 2H), 3.17 - 3.10 (m, 1H), 2.81 - 2.71 (m, 1H), 2.05 - 1.98 (m, 1H), 1.69 - 1.63 (m, 1H), 1.00 - 0.97 (m, 6H); m/z 312.

### Method 99

### (4-lodophenyl)-[(3S)-3-(1-piperidyl)pylrolidin-1-yl]methanone

A solution of (4-iodophenyl)-[(3R)-3-methylsulfonyloxypyrrolidin-1-yl]methanone (Method 88; 2.0 g) and piperidine (1.84 g) in dioxane (10 ml) was heated at 101°C for 3 days in a sealed tube. The reaction mixture was concentrated *in vacuo,* dissolved in MeOH and passed through a 50g SCX column, washed with additional MeOH then eluted with 7N NH₃ in MeOH. The solid obtained was suspended in hot acetonitrile then filtered to give the title compound as a colourless solid. NMR 7.79 (d, 2H), 7.27 (d, 2H), 3.65 - 3.49 (m, 2H), 3.44 - 3.38 (m, 1H), 3.25 (dd, 1H), 2.95 - 2.84 (m, 1H), 2.45 - 2.40 (m, 2H), 2.37 - 2.30 (m, 2H), 2.07 - 2.01 (m, 1H), 1.80 - 1.72 (m, 1H), 1.54 - 1.47 (m, 4H), 1.41 - 1.37 (m, 2H); m/z 385.

### Method 100

### 1,4-Diazepan-1-yl-(4-iodophenyl)methanone

tert-Butyl 1,4-diazepane-1-carboxylate (15.7 g) was added slowly to a solution of 4-iodobenzoyl chloride (20 g) and TEA (23 ml) in DCM (300 ml). The reaction was stirred for 30 mins then 2M NaOH (100 ml) was added and the aqueous layer extracted with DCM (3 x 200 ml). The combined organics were dried and solvent *removed in vacuo* to give a yellow gum (33 g) that was dissolved in DCM (200 ml), TFA (150 ml) was added and the reaction mixture stirred for 1 hr before concentrating *in vacuo.* 2M NaOH (100 ml) was added then the aqueous layer was saturated with solid potassium carbonate. The aqueous layer was extracted with DCM (3 x 200 ml), dried and the solvent *removed in vacuo* to yield a colourless solid. This was dissolved in a minimum amount of hot DCM; ether was then added until the solution became cloudy. The solution was stirred until a solid precipitated, which was filtered and dried to give the title compound as a colourless solid (20.8 g). NMR (400.132 MHz, CDC1₃) 7.74 (d, 2H), 7.14 (d, 2H), 3.78 - 3.73 (m, 2H), 3.47 - 3.39 (m, 2H), 3.07 - 3.04 (m, 1H), 2.97 - 2.89 (m, 2H), 2.86 - 2.83 (m, 1H), 1.96 - 1.87 (m, 1H), 1.74 - 1.67 (m, 2H); m/z 331.

### Method 101

### (4-Cyclopropyl- 1,4-diazepan-1-yl)-(4-iodophenyl)methanone

1,4-Diazepan-1-yl-(4-iodophenyl)methanone (Method 100; 4.0 g), acetic acid (3.6 g) molecular sieves (6 g) and (1-ethoxycyclopropyl)oxytrimethylsilane (4.1 g) were added to MeOH and stirred for 10 mins. NaCNBH₃ (1.17 g) was added and the reaction was heated under reflux for 24 hrs. The solvent was *removed in vacuo* to yield a viscous gum, 2M NaOH (50 ml) was added then extracted with DCM (3 x 100 ml). The combined organics were dried and concentrated *in vacuo* to give a clear oil. Distillation under reduced pressure (0.56 mmHg @ 138°C) gave the title compound as a clear oil (2.92 g). NMR (400.132 MHz, CDC1₃) 7.74 (d, 2H), 7.12 (d, 2H), 3.79 - 3.69 (m, 2H), 3.47 - 3.39 (m, 2H), 3.01 - 2.91 (m, 1H), 2.89 - 2.71 (m, 3H), 1.98 - 1.68 (m, 3H), 0.49 - 0.36 (m, 4H); m/z 371.

### Method 102

### (4-Cyclobutyl-1,4-diazepan-1-yl)-(4-iodophenyl)methanone

1,4-Diazepan-1-yl-(4-iodophenyl)methanone (Method 100; 3.5 g) and cyclobutanone (1.48 g) were added to MeOH (100 ml) and stirred at 0°C for 20 mins. NaCNBH₃ (1.02 g) was slowly added over a 20 min period keeping the temperature below 0°C. After complete addition the reaction was allowed to warm to ambient temperature and stirred for 2 days. The reaction mixture was then concentrated *in vacuo,* 2M NaOH (50 ml) added then extracted with ether (3 x 100 ml). The combined organics were dried and solvent *removed in vacuo* to give a viscous clear oil. Purification was achieved via column chromatography on silica eluting with 0-5% MeOH in DCM to give the title compound as a viscous clear oil (3.1 g). NMR (400.132 MHz, CDC1₃) 7.74 (d, 2H), 7.13 (d, 2H), 3.76 - 3.74 (m, 2H), 3.46 - 3.40 (m, 2H), 2.95 - 2.82 (m, 1H), 2.63 - 2.60 (m, 1H), 2.51 - 2.49 (m, 1H), 2.44 - 2.39 (m, 2H), 2.08 - 1.91 (m, 2H), 1.89 - 1.57 (m, 6H); m/z 385.

### Method 103

### (4-Iodophenyl)-[4-(2-methoxyethyl)-1,4-diazepan-1 -yl]methanone

1,4-Diazepan-1-yl-(4-iodophenyl)methanone (Method 100; 1.5 g), TEA (1.2 mL) and 2-methoxybromoethane (0.95 g) were added to DMA (50 mL) and heated at 70°C for 66 hrs. The reaction mixture was concentrated *in vacuo,* dissolved in MeOH and loaded onto a 50g SCX column eluting with MeOH then 7N NH₃ in MeOH. Purification on silica, eluting with 0-5% MeOH in DCM gave the title compound as an orange gum (1.02 g); NMR (400.132 MHz, CDC1₃) 7.74 (d, 2H), 7.13 (d, 2H), 3.78 - 3.73 (m, 2H), 3.51 - 3.39 (m, 4H), 3.36 - 3.32 (m, 3H), 2.88 - 2.86 (m, 1H), 2.77 - 2.67 (m, 5H), 2.00 - 1.93 (m, 1H), 1.83 - 1.75 (m, 1H); m/z 389.

### Method 104

### (4-Ethyl- 1,4-diazepan-1-yl)-(4-iodophenyl)methanone

1,4-Diazepan-1-yl-(4-iodophenyl)methanone (Method 100; 3.0 g) and ethanal (0.56 mL) were dissolved in MeOH (150 mL, stirred at ambient temperature for 10 mins then sodium cyanoborohydride (0.69 g) was added in one portion. After 2 hrs additional ethanal (0.56 mL) was added and the reaction stirred for a further 2 hrs. After which 2M NaOH solution (11 mL) was added and the reaction mixture stirred for 1 hr before concentrating in *vacuo.* The residue was partitioned between DCM and water and the aqueous layer was extracted with DCM twice. The combined organic extracts was filtered through a phase separation membrane (PTFE filter) and the solvent evaporated *in vacuo* to give a pale yellow oil which was further purified by RPHPLC (1.39g); NMR (400.132 MHz, CDCl₃) 7.76 - 7.72 (m, 2H), 7.15 - 7.11 (m, 2H), 3.79 - 3.73 (m, 2H), 3.48 - 3.39 (m, 2H), 2.80 - 2.77 (m, 1H), 2.70 - 2.66 (m, 1H), 2.63 - 2.49 (m, 4H), 2.00 - 1.92 (m, 1H), 1.82 - 1.76 (m, 1H), 1.11-1.01 1 (m, 3H); m/z 359.

### Method 105

### [(1S,4S)-2,5-Diazabicyclo[2.2.l]hept-5-yl]-(4-iodophenyl)methanone hydrochloride

4-Iodobenzoyl chloride (5.37 g) and tert-butyl (lS,4S)-2,5-diazabicyclo[2.2.1]heptane-2-carboxylate (4.0 g) were dissolved in DCM (150 ml), TEA (4.2 ml) was added and the reaction mixture stirred for 1 hr. After which 2.0 M NaOH (50 ml) was added and the reaction mixture was extracted with DCM (3 x 100 ml), the combined organics were dried and solvent *removed in vacuo* to give a colourless solid (8.9 g). This was dissolved in DCM (150 ml) and TFA (100 ml), stirred for 1 hr then concentrated *in vacuo.* Saturated aq. K₂C0₃ (50 ml) was added then the aqueous layer was extracted with DCM (3 x 100 ml), the combined organics dried and solvent *removed in vacuo* to give a viscous yellow gum. This was dissolved in acetonitrile, 4.0 N HC1 in dioxane (5.0 ml) was added then filtered and dried to give the title compound as a light yellow solid (4.3 g). NMR 9.63 (brs, 1H), 7.83 (d, 2H), 7.33 (d, 2H), 4.72 - 4.59 (m, 1H), 4.39 (s, 1H), 3.74 (d, 1H), 3.58 (dd, 1H), 3.38 (dd, 1H), 3.27 (dd, 1H), 2.07 (d, 1H), 1.93 (d, 1H); m/z 329.

### Method 106

### 4-{[4-(1-Isopropyl-2-methyl-1H-imidazol-5-yl)pyrimidin-2-yl]amino}benzoic acid Lithium salt

The title compound was prepared in similar manner to Method 52 from ethyl 4-{[4-(1-isopropyl-2-methyl-1*H*-imidazol-5-yl)pyrimidin-2-yl]amino}benzoate (Method 49) in place of ethyl 4- {[5-fluoro-4-(1-isopropyl-2-methyl-1*H*-imidazol-5-yl)pyrimidin-2-yl]amino}benzoate (Method 51); NMR (400.132 MHz, DMSO) 9.51 (s, 1H), 8.40 (d, 1H), 7.85 (d, 2H), 7.61 (d, 2H), 7.43 (s, 1H), 7.05 (d, 1H), 5.78 (m, 1H), 2.50 (s, 3H), 1.45 (d, 6H); m/z 338.

### Method 107

### (4-Iodophenyl)-[(3S)-3-methylsulfonyloxypyrrolidin-1-yl]methanone

The title compound was prepared in similar manner to Method 93 from (3S)-pyrrolidin-3-ol in place of (3R)-pyrrolidin-3-ol; NMR (400.132 MHz, CDC1₃) (rotamers) 7.78 (d, 2H), 7.34 - 7.20 (m, 2H), 5.37 - 5.25 (m, 1H), 3.93 - 3.47 (m, 4H), 3.16 - 3.02 (m, 4H), 2.42 - 2.32 (m, 1H), 2.31 - 2.11 (m, 1H); m/z 396.

### Method 108

### (4-Iodophenyl)-[(3R)-3-methylaminopyrrolidin-1-yl]methanone

The title compound was prepared in similar manner to Method 90 using (4-iodophenyl)-[(3S)-3-methylsulfonyloxypyrrolidin-1-yl]methanone (Method 107) in place of (4-Iodophenyl)-[(3R)-3-methylsulfonyloxypyrrolidin-1-yl]methanone (Method 88); NMR (400.132 MHz, CDC1₃) (rotamers) 7.78 (d, 2H), 7.34 - 7.20 (m, 2H), 5.37 - 5.25 (m, 1H), 3.93 - 3.47 (m, 4H), 3.16 - 3.02 (m, 4H), 2.42 - 2.32 (m, 1H), 2.31 - 2.11 (m, 1H); m/z 396.

### Example 209

The following illustrate representative pharmaceutical dosage forms containing the compound of formula (**I**), or a pharmaceutically acceptable salt *or in vivo* hydrolysable ester thereof (hereafter compound X), for therapeutic or prophylactic use in humans:

| **(a): Tablet I** | **mg/tablet** |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |

| **(b): Tablet II** | **mg/tablet** |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

| **(c): Tablet III** | **mg/tablet** |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |

| **(d): Capsule** | **mg/capsule** |
|---|---|
| Compound X | 10 |
| Lactose Ph.Eur | 488.5 |
| Magnesium stearate | 1.5 |

| **(e): Injection I** | **(50 mg/ml)** |
|---|---|
| Compound X | 5.0% w/v |
| 1M Sodium hydroxide solution | 15.0% v/v |
| 0.1M Hydrochloric acid | (to adjust pH to 7.6) |
| Polyethylene glycol 400 | 4.5% w/v |
| Water for injection | to 100% |

| **(f): Injection II** | **10 mg/ml** |
|---|---|
| Compound X | 1.0% w/v |
| Sodium phosphate BP | 3.6% w/v |
| 0.1 M Sodium hydroxide solution | 15.0% v/v |
| Water for injection | to 100% |

| **(g): Injection III** | **(lmg/ml,buffered to pH6)** |
|---|---|
| Compound X | 0.1 % w/v |
| Sodium phosphate BP | 2.26% w/v |
| Citric acid | 0.38% w/v |
| Polyethylene glycol 400 | 3.5% w/v |
| Water for injection | to 100% |

| | |
|---|---|
| Note | |

The above formulations may be obtained by conventional procedures well known in the pharmaceutical art. The tablets (a)-(c) may be enteric coated by conventional means, for example to provide a coating of cellulose acetate phthalate.

## Claims

1. A compound of formula (**I**): 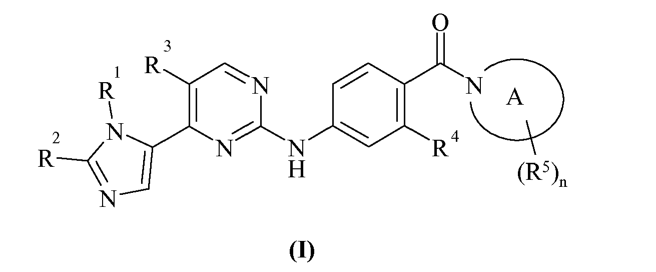 wherein:
**R**¹ is ethyl, propyl, isopropyl, butyl, iso-butyl, sec-butyl, t-butyl, cyclopropyl, cyclopropylmethyl, 1-cyclopropylethyl, cyclobutylmethyl, cyclopentyl or cyclobutyl; wherein R¹ may be optionally substituted on carbon by one or more R⁶;
**R**² is methyl, ethyl, isopropyl, fluoromethyl, difluoromethyl, trifluoromethyl, methoxymethyl, cyclopropylmethyl or cyclopropyl;
**R**³ is hydrogen or halo;
**R**⁴ is hydrogen, ethynyl, halo, nitro, cyano, hydroxy, amino, carboxy, carbamoyl, mercapto, methylthio, mesyl, trifluoromethyl, trifluoromethoxy, methyl, ethyl or methoxy;
**Ring A** is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷;
**R**⁵ is a substituent on carbon and is selected from halo, cyano, hydroxy, trifluoromethoxy, amino, carboxy, carbamoyl, sulphamoyl, C₁₋₆alkyl, C₂-₆alkenyl, C₂₋₆alkynyl, C ₁₋₆alkanoyl, *N* (C₁₋₆alkyl)carbamoyl, *NN*-(C₁₋₆akl)₂carbamoyl,C₁₋₆alkylS(O)ₐ wherein a is 0 to 2, C₁₋₆alkylsulphonyloxy, C₁₋₆alkoxycarbonyl, carbocyclyl, heterocyclyl, N (C₁₋₆alkyl)sulphamoyl or *N,N* (C₁₋₆alkyl)₂sulphamoyl; wherein R⁵ independently may be optionally substituted on carbon by one or more R⁸; wherein if said heterocyclyl contains an -NH- moiety that nitrogen may be optionally substituted by R¹⁵; or R⁵ is -NHR⁹, -NR¹⁰R¹¹ or -O-R¹²; ,
n is 0-2; wherein the values of R⁵ maybe the same or different;
**R**⁶ is selected from halo, methoxy and hydroxy;
**R**⁷, **R**⁹, **R**¹⁰, **R**¹¹, **R**¹² and **R**¹⁵ are independently selected from C₁₋₄alkyl, C₁₋₄alkanoyl, C₁₋₄alkylsulphonyl, C₂₋₄alkenylsulphonyl, C₂₋₄akynylsulphonyl, C₁₋₄alkoxycarbonyl, carbamoyl, *N*-(C₁₋₄alkyl)carbamoyl, *N,N* (C₁₋₄alkyl)carbamoyl, carbocyclyl or heterocyclyl; wherein R⁷, R⁹, R¹⁰, R¹¹, R¹² and R¹⁵ may be independently optionally substituted on carbon by one or more groups selected from R¹³; and wherein if said heterocyclyl contains an -NH- moiety that nitrogen may be optionally substituted by R¹⁴;
R⁸ is selected from halo, cyano, hydroxy, trifluoromethoxy, trifluoromethyl, amino, carboxy, carbamoyl, sulphamoyl, methyl, ethyl, methoxy, ethoxy, acetyl, acetoxy, methylamino, ethylamino, dimethylamino, diethylamino, *N-*methyl-*N*-ethylamino, acetylamino, phenylamino, N methylcarbamoyl, N ethylcarbamoyl, N,N-dimethylcarbamoyl, *N,N* diethylcarbamoyl, *N* methyl-*N*-ethylcarbamoyl, methylthio, ethylthio, methylsulphinyl, ethylsulphinyl, mesyl, ethylsulphonyl, methoxycarbonyl, ethoxycarbonyl, N-methylsulphamoyl, N-ethylsulphamoyl, N,N-dimethylsulphamoyl, *N,N-*diethylsulphamoyl or *N*-methyl-N-ethylsulphamoyl;
**R**¹³ is selected from halo, cyano, hydroxy, amino, trifluoromethyl, trifluoromethoxy, dimethylamino, carbocyclyl, heterocyclyl, C₁₋₃akyl and C₁₋₃akoxy; and
**R**¹⁴ is selected from C₁₋₃akyl, C₁₋₃akanoyl, C₁₋₃alkylsulphonyl, C₁₋₃alkoxycarbonyl, carbamoyl, N-(C₁₋₃alkyl)carbamoyl and *N,N*-(C₁₋₃alkyl)carbamoyl; or a pharmaceutically acceptable salt or *an in vivo* hydrolysable ester thereof.

2. A compound of formula (I), or a pharmaceutically acceptable salt or *an in vivo* hydrolysable ester thereof, as claimed in claim 1 wherein R¹ is ethyl, isopropyl, cyclopropylmethyl, 1-cyclopropylethyl, cyclobutylmethyl, cyclopentyl or cyclobutyl.

3. A compound of formula (I), or a pharmaceutically acceptable salt or *an in vivo* hydrolysable ester thereof, as claimed in either claim 1 or claim 2 wherein R² is methyl, ethyl, isopropyl, difluoromethyl, trifluoromethyl, methoxymethyl or cyclopropyl.

4. A compound of formula (I), or a pharmaceutically acceptable salt or *an in vivo* hydrolysable ester thereof, as claimed in any one of claims 1-3 wherein R³ is hydrogen, fluoro or chloro.

5. A compound of formula **(I)**, or a pharmaceutically acceptable salt or an *in vivo* hydrolysable ester thereof, as claimed in any one of claims 1-4 wherein R⁴ is hydrogen, halo, cyano, mesyl, methyl or methoxy.

6. A compound of formula **(I)**, or a pharmaceutically acceptable salt or *an in vivo* hydrolysable ester thereof, as claimed in any one of claims 1-5 wherein Ring A is a nitrogen linked 4-7 membered saturated ring which optionally contains an additional nitrogen, oxygen or sulphur atom; wherein 2 atoms of Ring A, when Ring A is a nitrogen linked 5-7 membered saturated ring, may optionally be connected by a one or two atom bridge; and wherein if Ring A contains an additional nitrogen atom that nitrogen may be optionally substituted by R⁷; wherein
R⁷ is selected from C₁-₄alkyl, carbocyclyl or heterocyclyl; wherein R⁷ may be optionally substituted on carbon by one or more groups selected from R¹³;
R¹³ is selected from halo, hydroxy, C₁-₃alkyl, C₁₋₃alkoxy, dimethylamino or heterocyclyl.

7. A compound of formula **(I)**, or a pharmaceutically acceptable salt or an *in vivo* hydrolysable ester thereof, as claimed in any one of claims 1-6 wherein R⁵ is a substituent on carbon and is selected from hydroxy, amino, C₁-₆alkyl, C₁-₆alkylsulphonyloxy, C₁-₆alkylS(O)ₐ wherein a is 2 or heterocyclyl; wherein R⁵ independently may be optionally substituted on carbon by one or more R⁸; or R⁵ is -NHR⁹ or-NR¹⁰R11; wherein if said heterocyclyl contains an -NH- moiety that nitrogen may be optionally substituted by R¹⁵; wherein
R⁶ is selected from halo, methoxy and hydroxy;
R⁹, R¹⁰, R¹¹ and R¹⁵ are independently selected from C₁-₄alkyl or carbocyclyl; wherein R⁹, R ¹⁰ R¹¹ and R¹⁵ may be independently optionally substituted on carbon by one or more groups selected from R¹³_{;}
R⁸ is selected from hydroxy, amino and phenylamino; and
R¹³ is selected from carbocyclyl and C₁-₃alkoxy.

8. A compound of formula **(I)**, or a pharmaceutically acceptable salt or an *in vivo* hydrolysable ester thereof, as claimed in any one of claims 1-7 wherein n is 0 or 1.

9. A compound of formula (I): 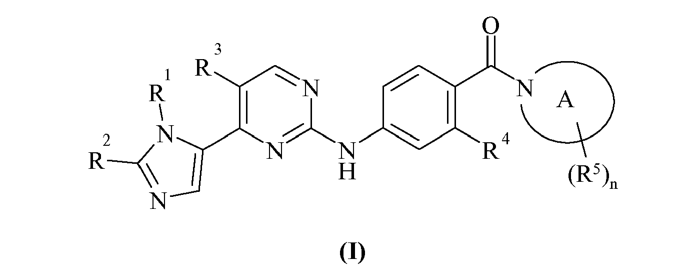 wherein:
R¹ is ethyl, isopropyl, cyclopropylmethyl, 1-cyclopropylethyl, cyclobutylmethyl, cyclopentyl or cyclobutyl;
R² is methyl, ethyl, isopropyl, difluoromethyl, trifluoromethyl, methoxymethyl or cyclopropyl;
R³ is hydrogen, fluoro or chloro;
R⁴ is hydrogen, fluoro, chloro, cyano, mesyl, methyl or methoxy;
Ring A is morpholino, 1,1-dioxothimorpholino, piperidin-1-yl, piperazin-1-yl, azetidin-1-yl, 4-methyl- 1,4-diazepan-1-yl, 4-ethyl- 1,4-diazepan- 1-yl, 4-(2-dimethylaminoethyl)piperazin-1-yl, 4-(2-methoxyethyl)piperazin-1-yl, 4-(2-pyrrolidin-1-ylethyl)piperazin-1-yl, 4-cyclopropylpiperazin-1-yl, 4-methylpiperazin-1-yl, 4-isopropylpiperazin-1-yl, 4-(4-fluorophenyl)piperazin-1-yl, 4-(2-fluorophenyl)piperazin-1-yl, 4-(2,4-difluorophenyl)piperazin-1-yl, 4-(3,4-difluorophenyl)piperazin-1-yl, 4-(2-chlorophenyl)piperazin-1-y1, 4-(4-chlorophenyl)piperazin-1-yl, 4-(4-phenyl)piperazin-1-yl, 4-(2-methoxyphenyl)piperazin-1-y1, 4-(3-methoxyphenyl)piperazin-1-y1, 4-(4-methoxyphenyl)piperazin-1-yl, 4-(3-methylphenyl)piperazin-1-yl, 4-(2-methylphenyl)piperazin-1-yl, 4-(4-methylphenyl)piperazin-1-yl, 4-(2,3-dimethylphenyl)piperazin-1-yl, 4-(2,6-dimethylphenyl)piperazin-1-yl, 4-(4-hydroxyphenyl)piperazin- 1-yl, 4-(2-hydroxyphenyl)piperazin-1-yl, 4-(5-chloropyrid-2-yl)piperazin-1-yl, 4-cyclopropylhomopiperazin-1-yl, 4-cyclobutylhomopiperazin-1-yl, 4-(2-hydroxyethyl)homopiperazin-1-yl, 4-(2-methoxyethyl)homopiperazin-1-yl, 4-isopropylhomopiperazin-1-yl, 1,4-oxazepan-4-yl, 8-oxa-3-azabicyclo[3.2.1]oct-3-yl, pyrrolidin-1-yl, 2,5-diazabicyclo[2.2.1]hept-5-yl, 2-methyl-2,5-diazabicyclo[2.2.1] ]hept-5-yl, 2-(2-methoxyethyl)-2,5-diazabicyclo[2.2.1]hept-5-yl, 2-ethyl-2,5-diazabicyclo[2.2.1]hept-5-yl or 2-isopropyl-2,5-diazabicyclo[2.2.1] hept-5-yl;
R⁵ is a substituent on carbon and is selected from hydroxy, amino, methyl, mesyl, mesyloxy, morpholino, piperidin-1-yl, dimethylamino, diethylamino, isopropyl, pyrid-2-yl, hydroxymethyl, methylamino, aminomethyl, 4-methylpiperazin-1-yl, cyclopropylamino, pyrrolidin-1-yl, homopiperazin-1-yl, cyclobutylamino, phenylaminomethyl, *N*-methyl-*N*-(cyclopropylmethyl)amino, *N*-methyl-*N*-cyclopropylamino, *N*-methyl-*N*-isobutylamino, N methyl-N (2-methoxyethyl)amino, *N*-ethyl-*N*-propylamino or *N*-methyl-*N*-cyclobutylamino;
n is 0 or 1;
or a pharmaceutically acceptable salt or an *in vivo* hydrolysable ester thereof.

10. A compound of formula **(I)**: 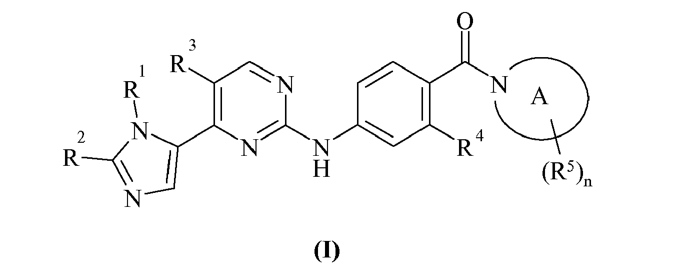 selected from:
4-(1 -Isopropyl-2-methyl-1H-imidazol-5-yl)-N- {4-[(4-methyl-1,4-diazepan-1 - yl)carbonyl]phenyl}pyrimidin-2-amine;
N-(4- {[(3S)-3-(Dimethylamino )pyrrolidin-l-yl]carbonyl}phenyl)-5- fluoro-4-(1-isopropyl-2-methyl-1 1-H-imidazol-5 -yl)pyrimidin-2-amine;
5-Fluoro-4-(1-isopropyl-2-methyl-1H-imidazol-5-yl)-N-{4-[(4-methyl-1,4-diazepan-1-yl)carbonyl]phenyl}pyrimidin-2-amine;
5-Chloro-N-(4- {[(3S)-3-(dimethylamino)pyrrolidin-l-yl]carbonyl }phenyl)-4-(1-isopropyl-2-methyl-1H-imidazol-5-yl)pyrimidin-2-amine;
5-Chloro-4-(1-isopropyl-2-methyl-1H-imidazol-5-yl)-N- {4-[(4-methyl-1,4-diazepan-1 - yl)carbonyl]phenyl}pyrimidin-2-amine;
N- {4-[(4-Isopropyl-1,4-diazepan-1-yl)carbonyl]phenyl}-4-(1-isopropyl-2-methyl-1H-imidazol-5-yl)pyrimidin-2-amine;
N-(4- {[(3S)-3-(Dimethylamino)pyrrolidin-1-yl] carbonyl}phenyl)-4-(1-isopropyl-2-methyl-1H-imidazol-5-yl)pyrimidin-2-amine;
N-(4- {[(3 S)-3 -(Dimethylamino)pyrrolidin-1-yl]carbonyl}-3-fluorophenyl)-5-fluoro-4-(1-isopropyl-2-methyl-1H-imidazol-5-yl)pyrimidin-2-amine;
[4-[[5-Fluoro-4-(2-methyl-3-propan-2-yl-3H-imidazol-4-yl)pyrimidin-2-yl]amino]phenyl]-(4-propan-2-yl-1,4-diazepan-1-yl)methanone;
[4-[[5-Fluoro-4-(2-methyl-3-propan-2-yl-3H-imidazol-4-yl)pyrimidin-2-yl]amino]phenyl]-[(3S)-3-(methylamino)pyrrolidin-1-yl]methanone;
or a pharmaceutically acceptable salt or an *in vivo* hydrolysable ester thereof.

11. A pharmaceutical composition which comprises a compound of the formula (I), or a pharmaceutically acceptable salt *or in vivo* hydrolysable ester thereof, as claimed in any one of claims 1-10, and a pharmaceutically-acceptable diluent or carrier.

12. A compound of the formula (I), or a pharmaceutically acceptable salt *or in vivo* hydrolysable ester thereof, as claimed in any one of claims 1-10, for use as a medicament.
